# EUROPEAN PATENT APPLICATION

(11) **EP 2 527 459 A1**
(43) Date of publication of application: **28.11.2012**
(21) Application number: 11164480.3
(22) Date of filing: 02.05.2011
(51) Int. Cl.: C12Q 1/68

(54) **Blood-based gene detection of non-small cell lung cancer**

(71) Applicant: Rheinische Friedrich-Wilhelms-Universität Bonn, 53113 Bonn (DE); UNIVERSITÄT ZU KÖLN, 50923 Köln (DE)
(72) Inventor: Hofmann, Andrea, 53111 Bonn (DE); Schultze, Joachim L., Prof. Dr., 53639 Königswinter (DE); Staratschek-Jox, Andrea, Dr., 50733 Köln (DE); Wolf, Jürgen, Dr., 50733 Köln (DE); Zander, Thomas, Dr., 53115 Bonn (DE)
(74) Representative: Hoppe, Georg Johannes

(57) **Abstract**

The present invention provides a method for early detection of non-small cell lung cancer based on the abundance of RNAs from blood samples as well as diagnostic tools such as kits and arrays suitable for such method.

## Description

The present invention provides a method for the detection of non-small cell lung cancer (NSCLC) based on the abundance of particular RNAs from blood samples, as well as diagnostic tools such as kits and arrays suitable for such method.

### Background of the Invention

Lung cancer is still the leading cause of cancer related death worldwide. Prognosis has remained poor with a disastrous 2 year survival rate of only about 15% due to diagnosis of the disease in late, i.e. incurable stages in the majority of patients (Jemal, A. et al., CA Cancer J. Clin., 58: 71-96 (2008)) and still disappointing therapeutic regimens in advanced disease (Sandler, A. et al., N. Engl. J. Med., 355:2542-50 (2006)). Thus, there is an urgent need to establish reliable tools for the identification of non-small cell lung cancer (NSCLC) patients at early stages of the disease e.g. prior to the development of clinical symptoms. Today, the only way to detect non-small cell lung cancer is by means of imaging technologies detecting morphological changes in the lung in combination with biopsy specimens taken for histological examination. However, these screening approaches are not easily applied to secondary prevention of non-small cell lung cancer in an asymptomatic population (Henschke, C.I. et al., N. Engl. J. Med., 355: 1763-71 (2006)).

The use of surrogate tissue-based, e.g. blood-based, biomarkers for non-small cell lung cancer might therefore circumvent the known pitfalls of imaging technologies and invasive diagnostics ((Henschke, C.I. et al., N. Engl. J. Med., 355: 1763-71 (2006); Bach, P. B. et al., Chest, 132: 69S-77S (2007)). Such biomarkers might be utilized to direct imaging based and invasive screening approaches to only those individuals identified as potential non-small cell lung cancer patients by biomarker screening.

Array-based assessment of disease-specific gene expression patterns in peripheral blood mononuclear cells (PBMC) have been reported for non-malignant (Staratschek-Jox, A. et al., Expert Review of Molecular Diagnostics, 9: 271-80 (2009)) and malignant diseases including renal cell carcinoma, melanoma, bladder, breast and lung cancer (Burczynski, M.E. et al., Clin. Cancer Res., 11: 1181-9 (2005); Twine, N. C. et al., Cancer Res., 63: 6069-75 (2003); Sharma, P. et al., Breast Cancer Res., 7: R634-44 (2005); Osman, I. et al., Clin. Cancer Res., 12:3374-80 (2006); Critchley-Thorne, R. J. et al., PLoS Med., 4: e176 (2007); Showe, M.K. et al., Cancer Res., 69: 9202-10 (2009)). In some cases, gene expression profiles derived from PBMC were even suggested as promising tools for early detection (Sharma, P. et al., Breast Cancer Res., 7: R634-44 (2005); Showe, M.K. et al., Cancer Res., 69: 9202-10 (2009)) or prediction of prognosis (Burczynski, M.E. et al., Clin. Cancer Res., 11: 1181-9 (2005)), albeit these findings have not yet been validated in independent studies. Furthermore, circumventing known pitfalls of analyzing PBMC in a clinical setting (Debey, S. et al., Pharmacogenomics J., 4: 193-207 (2004); Debey, S. et al., Genomics, 87: 653-64 (2006)) by using stabilized RNA derived from whole blood would further strengthen the validity of blood-based surrogate biomarkers for early diagnosis of lung cancer and other malignant diseases.

### Description of the invention

The inventors have shown for the first time that measuring the abundance of a set of at least 5 different RNAs, possibly stemming from the transcription of genes, from a blood sample allows for a highly accurate prediction of whether a human individual suffers from non-small cell lung cancer or not. Thus, a means for detecting or diagnosing non-small cell lung cancer is provided that is based on a minimally invasive method such as drawing a blood sample from a patient.

In this context, the invention teaches a test system that is trained in detection of a non-small cell lung cancer, comprising at least 5 RNAs, which can be quantitatively measured on an adequate set of training samples, with adequate clinical information on carcinoma status, applying adequate quality control measures, and on an adequate set of test samples, for which the detection yet has to be made. Given the quantitative values for the biomarkers and the clinical data for the training, a classifier can be trained and applied to the test samples to calculate the probability of the presence of the non-small cell lung cancer. The 484 RNAs of the invention are defined in particular in table 2 and are characterized through their nucleotide sequence or further through synonymous probe IDs provided in table 2. It is noted that the abundance of a particular RNA may be determined using different probe IDs, as known in the art.

Therefore, the inventors provide a means for diagnosing or detecting NSCLC in a human individual with a sensitivity and specificity (as shown e.g. by the AUC values in lists 1 to 51), as it has thus far not yet been described for a blood-based method. In a first aspect, the invention provides a method for the detection of non-small cell lung cancer (NSCLC) of any clinical stage in a human individual based on RNA obtained from a blood sample obtained from the individual. Such a method comprises at least the following two steps: Firstly, the abundance of at least 5 RNAs that are chosen from the RNAs listed in Table 2 is determined in the sample. Secondly, based on the measured abundance, it is concluded whether the patient has NSCLC or not.

In preferred embodiments of the invention, the abundance of at least 6 RNAs, of at least 7 RNAs, of at least 11 RNAs, of at least 16 RNAs, of at least 21 RNAs, of at least 25 RNAs, or of at least 34 RNAs that are chosen from the RNAs listed in Table 2 is determined, respectively. Reference is made in this context to lists 1 to 51, which show exemplary embodiments of sets of RNAs whose abundance can be measured in the method of the invention. For each set of RNAs, the area under the curve is provided, which is a quantitative parameter for the clinical utility (specificity and sensitivity) of the invention.

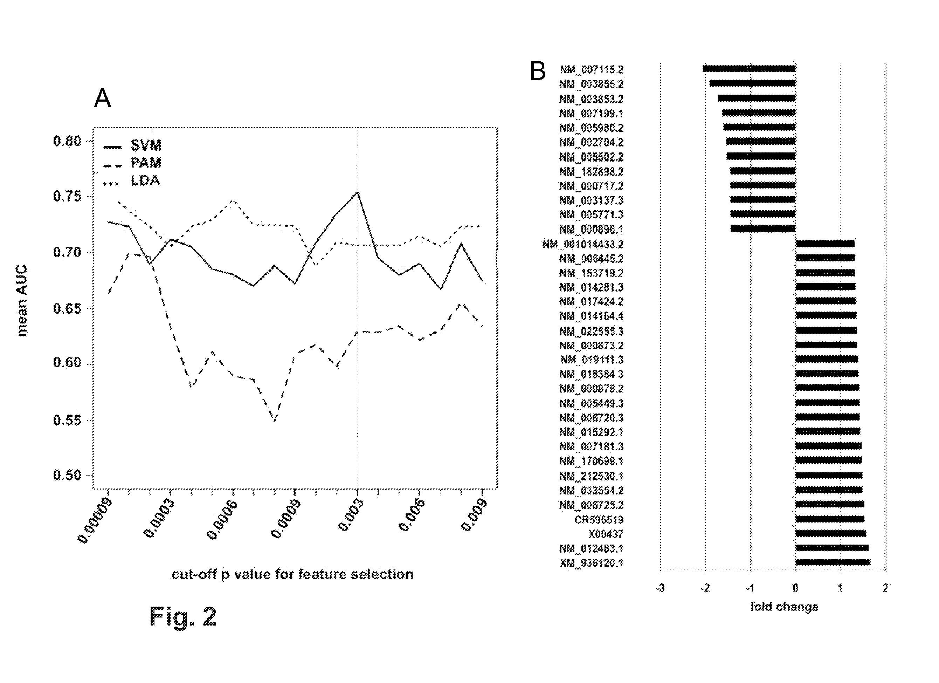

In a preferred embodiment of this method, it comprises determining the abundance of the RNAs specified in figure 2B.

In further preferred embodiments of the invention, the abundance of at least 5 RNAs from the RNAs listed in Table 2 is measured, wherein at least one RNA that is chosen from the group consisting of SEQ ID NOs:72 (PLSCR1), 85 (VPREB3), 99 (NT5C2), 146 (BTN3A2), 183 (ANKMY1) and 283 (BLR1) is excluded from the measurement. In preferred embodiments, at least two RNAs that are chosen from the group consisting of SEQ ID NOs:72, 85, 99, 146, 183 and 283, or all six RNAs from the group consisting of SEQ ID NOs:72, 85, 99, 146, 183 and 283 are not measured. In the latter case the abundance of at least 5 RNAs is measured that are listed in Table 2 and that are chosen from the group consisting of SEQ ID NOs: 1-71, 73-84, 86-98, 100-145, 147-182, 184-282 and 284-484. It is understood that the embodiments of the method of the invention that avoid the determination of the abundance one or more of the RNAs of SEQ ID NOs:72, 85, 99, 146, 183 and 283 are at least equally sensitive as those including the determination of said RNAs. An RNA obtained from an individual's blood sample, i.e. an RNA biomarker, is an RNA molecule with a particular base sequence whose presence within a blood sample from a human individual can be quantitatively measured. The RNA can e.g. be mRNA, cDNA, unspliced RNA, or fragments of any of the before mentioned molecules.

The term "abundance" refers to the amount of RNA in a sample of a given size. In a preferred embodiment, the term "abundance" is equivalent to the term "expression level".

Lung Cancer is subdivided into two major histological and clinical groups: Small Cell Lung Cancer and Non Small Cell Lung Cancer (WHO Lung Cancer classificationTravis et al IARC Press 2004). The UICC based staging system has recently been revised. All data obtained for this study were based on the UICC based staging system version 6 (Travis et al JTO 2008).

The conclusion whether the patient has NSCLC or not may comprise, in a preferred embodiment of the method, classifying the sample as being from a healthy individual or from an individual having NSCLC based on the specific difference of the abundance of the at least 5 RNAs in healthy individuals versus the abundance of the at least 5 RNAs in individuals with NSCLC in a reference set. In the present method, a sample can be classified as being from a patient with NSCLC or from a healthy individual without the necessity to run a reference sample of known origin (i.e. from an NSCLC patient or a healthy individual) at the same time.

In a preferred embodiment the method of the invention is a method for the detection of NSCLC in a human individual based on RNA obtained from a blood sample obtained from the individual, comprising:
- determining the abundance of at least 5 RNAs in the sample that are chosen from the RNAs listed in Table 2, and
- classifying the sample as being from a healthy individual or from an individual having NSCLC based on the specific difference of the abundance of the at least 5 RNAs in healthy individuals versus the abundance of the at least 5 RNAs in individuals with NSCLC. Particularly preferred for this method is that the abundance of at least 7 RNAs, of at least 11 RNAs, of at least 16 RNAs, of at least 21 RNAs, of at least 25 RNAs, or of at least 34 RNAs chosen from the RNAs listed in Table 2 is determined.

The conclusion or test result whether the individual has NSCLC or not is preferably reached on the basis of a classification algorithm, such as a support vector machine, a random forest method, or a K-nearest neighbor method, as known in the art. The conclusion or test result whether the individual has NSCLC or not is preferably reached on the basis of a classification algorithm, such as a support vector machine, as known in the art.

For the development of a model that allows for the classification for a given set of biomarkers, such as RNAs, only those methods are needed that are generally known to a person of skill in the art.

The major steps of such a model are:
1) condensation of the raw measurement data (for example combining probes of a microarray to probeset data, and/or normalizing measurement data against common controls);
2) training and applying a classifier (i.e. a mathematical model that generalizes properties of the different classes (NSCLC vs. healthy individual) from the training data and applies them to the test data resulting in a classification for each test sample.

The key component of these classifier training and classification techniques is the choice of RNA biomarkers that are used as input to the classification algorithm.

The classification is in one embodiment achieved by applying a Prediction Method for Support Vector Machines (hereinafter SVM; David Meyer based on C++-code by Chih-Chung Chang and Chih-Jen Lin), which predicts values based upon a model trained by svm.

### Usage:

## S3 method for class 'svm':
   predict(object, newdata, decision.values = FALSE,
   probability = FALSE, ..., na.action = na.omit)

### Arguments:

| | |
|---|---|
| Object | Object of class "svm", created by svm. |
| Newdata | A matrix containing the new input data. A vector will be transformed to a n x 1 matrix. |
| decision.values | Logical controlling whether the decision values of all binary classifiers computed in multiclass classification shall be computed and returned. |
| Probability | Logical indicating whether class probabilities should be computed and returned. Only possible if the model was fitted with the probability option enabled. |
| na.action ... | A function to specify the action to be taken if 'NA's are found. The default action is na.omit, which leads to rejection of cases with missing values on any required variable. An alternative is na.fail, which causes an error ifNA cases are found. (NOTE: If given, this argument must be named.) Currently not used. |

A vector of predicted values (for classification: a vector of labels, for density estimation: a logical vector). If decision.value is TRUE, the vector gets a "decision.values" attribute containing a n x c matrix (n number of predicted values, c number of classifiers) of all c binary classifiers' decision values. There are k * (k - 1) / 2 classifiers (k number of classes). The colnames of the matrix indicate the labels of the two classes. If probability is TRUE, the vector gets a "probabilities" attribute containing a n x k matrix (n number of predicted values, k number of classes) of the class probabilities.

If the training set was scaled by svm (done by default), the new data is scaled accordingly using scale and center of the training data.

The specific SVM algorithm suitable for the method of the invention is shown in Table 3.

The determination of the expression profiles of the RNAs described herein is performed in a blood-based fashion. In particular, blood samples are used in the method of the invention. Methods for determining the expression profiles therefore comprise e.g. in situ hybridization, PCR-based methods, sequencing, or preferably microarray-based methods.

The conclusion of a sample as stemming from a healthy individual or from an individual with NSCLC is based on increases or decreases in the abundance of the RNAs in the sample compared to reference values. Specifically, an increase of the abundance preferably provides for a change of > 1.1, >1.2, or >1.3. A decrease of the expression preferably provides for a change < 0.9, < 0.8, or < 0.7 relative to the respective expression in healthy individuals.

The abundance can e.g. be determined with an RNA hybridization assay, preferably with a solid phase hybridization array (microarray), or with a real-time polymerase chain reaction, or through sequencing. Preferably, the abundance of the at least 5 RNAs of Table 2 is determined through a hybridization with probes. Such a probe may comprise 12 to 150, preferably 25 to 70 consecutive nucleotides with a sequence that is reverse complementary to at least part of the RNA whose abundance is to be determined, such that a specific hybridization between the probe and the RNA whose abundance is to be determined can occur.

In another aspect, the invention refers to a solid phase hybridization array (microarray) for the detection of NSCLC based on a blood sample from a human individual. Such an array comprising probes for detecting at least 5 of the RNAs that are chosen from the RNAs listed in Table 2. Preferably, the array comprises probes for detecting at least 6 RNAs, of at least 7 RNAs, of at least 11 RNAs, of at least 16 RNAs, of at least 21 RNAs, of at least 25 RNAs, or of at least 34 RNAs that are chosen from the RNAs listed in Table 2, respectively. It is further preferred that said probes for detecting said at least 5 RNAs from the RNAs listed in Table 2 exclude probes for detecting one, or two, or all six of the RNAs that is chosen from the group consisting of SEQ ID NOs:72, 85, 99, 146, 183 and 283. In particularly preferred embodiments, the probes for detecting the at least 5 RNAs as listed in Table 2 are chosen from the group consisting of SEQ ID NOs: 1-71, 73-84, 86-98, 100-145, 147-182, 184-282 and 284-484.

A micoroarray includes a specific set of probes, such as oligonucleotides and/or cDNA's (e.g. ESTs) corresponding in whole or in part, and/or continuously or discontinuously, to regions of RNAs that can be extracted from a blood sample of a human individual; wherein the probes are localized onto a support. The probes can correspond in sequence to the RNAs of the invention such that hybridization between the RNA from the individual and the probe occurs, yielding a detectable signal. This signal can be detected and together with its location on the support can be used to determine which probe hybridized with RNA from the individual's blood sample.

In another aspect, the invention refers to the use of a hybridization array as described above and herein for the detection of NSCLC in a human individual based on RNA from a blood sample obtained from the individual, comprising determining the abundance of at least 5 RNAs, preferably at least 7 RNAs, stemming from the 484 genes listed in Table 2 in the sample.

In another aspect, the invention refers to a kit for the detection of NSCLC, comprising means for determining the abundance of at least 5, preferably at least 7 out of 484 RNAs in the sample that are chosen from the RNAs listed in table 2. Preferably said kit comprises means for determining the abundance of at least 5 RNAs chosen from the RNAs listed in Table 2, wherein the means for determining one, or two, or all six of the RNAs that is chosen from the group consisting of SEQ ID NOs:72, 85, 99, 146, 183 and 283 are excluded. In particularly preferred embodiments, the kit comprises means for determining the abundance of at least 5 RNAs as listed in Table 2 that are chosen from the group consisting of SEQ ID NOs: 1-71, 73-84, 86-98, 100-145, 147-182, 184-282 and 284-484.

Such a kit may comprise probes and/or a microarray as described above and herein. Specifically, the kit preferably comprises probes, which in turn comprise 15 to 150, preferably 30 to 70 consecutive nucleotides with a reverse complementary sequence to the at least 5 RNAs (or more, as disclosed above and herein) whose abundance is to be determined. Alternatively, the kit preferably comprises a micorarray comprising probes with a reverse complementary sequence to the at least 5 RNAs whose abundance is to be determined. Optionally, the kit may further comprise a mixture of at least 5 of the RNAs of table 2 in a given amount or concentration for use as a standard and/or other components, such as solvents, buffers, labels, primers and/or reagent.

The expression profile of the herein disclosed at least 5 RNAs (or more, as disclosed above and herein) is determined, preferably through the measurement of the quantity of the mRNA of the marker gene. This quantity of the mRNA of the marker gene can be determined for example through chip technology (microarray), (RT-) PCR (for example also on fixated material), Northern hybridization, dot-blotting, sequencing, or *in situ* hybridization.

The microarray technology, which is most preferred, allows for the simultaneous measurement of RNA abundance of up to many thousand gene products and is therefore an important tool for determining differential expression in this context, in particular between two biological samples or groups of biological samples. As will be understood by a person of skill in the art, the analysis can also be performed through single reverse transcriptase-PCR, competitive PCR, real time PCR, differential display RT-PCR, Northern blot analysis, sequencing, and other related methods. In general, the larger the number of markers is that are to be measured, the more preferred is the use of the microarray technology.

Measurements can be performed using the complementary DNA (cDNA) or complementary RNA (cRNA), which is produced on the basis of the RNA to be analyzed, e.g. using microarrays. A great number of different arrays as well as their manufacture are known to a person of skill in the art and are described for example in the U.S. Pat. Nos. 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,331; 5,554,501; 5,561,071; 5,571,639; 5,593,839; 5,599,695; 5,624,711; 5,658,734; and 5,700,637.

Using three independent data sets of patients and controls, the validity of whole blood-based gene expression profiling for the detection of NSCLC patients among smokers was investigated. It is shown that RNA-stabilized blood samples allow for the identification of NSCLC patients among hospital based controls as well as healthy individuals.

Using RNA-stabilized whole blood from smokers in three independent sets of NSCLC patients and controls, the inventors present a gene expression based classifier that can be used as a biomarker to discriminate between NSCLC cases and controls. The optimal parameters of this classifier were first determined by applying a classical 10-fold cross-validation approach to a training set consisting of NSCLC patients (stage I-IV) and hospital based controls (TS). Subsequently this optimized classifier was successfully applied to two independent validation sets, namely VS1 comprising NSCLC patients of stage I-IV and hospital based controls (VS1) and VS2 containing patients with stage I NSCLC and healthy blood donors. This successful application of the classifier in both validation sets underlines the validity and robustness of the classifier. Extensive permutation analysis using random feature lists and the possibility of building specific classifiers independently of the composition of the initial training set further support the specificity of the classifier. The inventors found no associations between stage of disease and the probability score assigned to each sample. In addition, the inventors observed no association between other cancers and the probability score of the controls (data not shown). But controls without documented morbidity (controls in VS2) tend to have lower probability scores to be a case as compared to controls with documented morbidity, although this was not statistically significant.

The gene set used to build the classifier was enriched in genes related to immune functions. The inventors therefore postulate, without wanting to be bound by theory, that the classifier is based on the transcriptome of blood-based immune effector cells rather than influenced by the occurrence of rare tumor cells occasionally detected in blood of cancer patients although this possibility cannot be ruled out (Nagrath, S. et al., Nature, 450: 1235-9 (2007)). Moreover, the lack of NSCLC tumor cell specific transcripts e.g. TTF1, cytokeratins or hTERT in the inventors' classifier points into the same direction. Gene set enrichment analysis (Osman, I. et al., Clin. Cancer Res., 12:3374-80 (2006)) of the gene set used in the inventors' diagnostic approach in comparison with published expression datasets from a variety of cancer entities (Segal, E. et al., Nat. Genet., 36: 1090-8 (2004)) revealed an significant overlap with 26 % of the lung cancer tissue specific gene expression profiles. Since NSCLC tissue consists of tumor cells, immune and stromal cells (Critchley-Thorne, R. J. et al., PLoS Med., 4: e176 (2007)) the inventors presume that the similarities of both gene sets is due to a similar regulation of genes present in immune cells in NSCLC tissue and peripheral blood of NSCLC patients. These findings are in line with data demonstrating tumor-induced alteration of the immune system in mice (Burczynski, M.E. et al., Clin. Cancer Res., 11: 1181-9 (2005); Twine, N. C. et al., Cancer Res., 63: 6069-75 (2003)) and in men (Showe, M.K. et al., Cancer Res., 69: 9202-10 (2009); Keller, A. et al., BMC Cancer, 9: 353 (2009)).

Recently, Showe, M.K. et al., Cancer Res., 69: 9202-10 (2009) reported a NSCLC associated gene expression signature derived from PBMC of predominantly early stage NSCLC patients. Also in this study an enrichment of immune-associated pathways in the signature was observed further indicating that the alteration of the immune system might be a common feature already during the initial phase of NSCLC development. Since the inventors used RNA-stabilized whole blood and not PBMC for analysis, the inventors were not surprised that the signature identified by Showe et al. could not be used in the inventors' dataset to distinguish between cases and controls. The same holds true when applying the inventors' classifier to the published data set. Findings derived from several of the inventors' own studies further underline that signatures derived from PBMC and RNA-stabilized whole blood samples cannot be directly compared (Debey, S. et al., Pharmacogenomics J., 4: 193-207 (2004); Debey, S. et al., Genomics, 87: 653-64 (2006)). However, for clinical applicability and robustness, RNA-stabilized approaches reveal more reliable results in a multi-center setting (Debey, S. et al., Genomics, 87: 653-64 (2006)).

### Description of the Figures

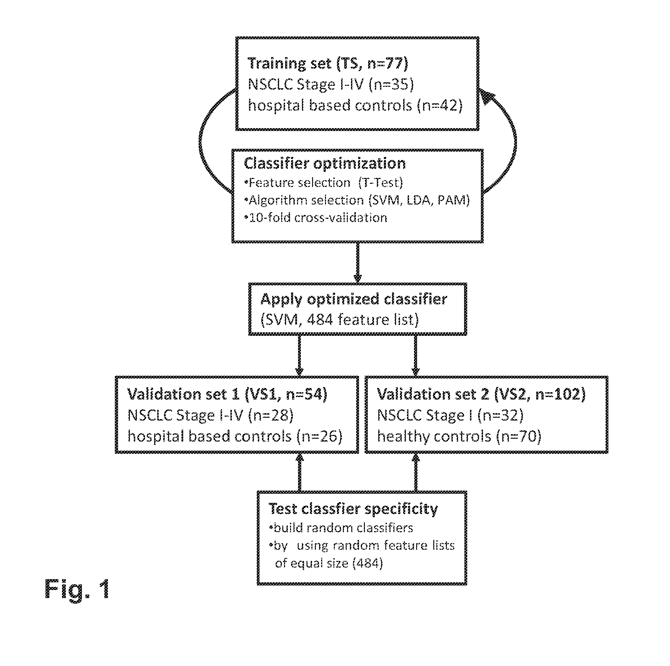

Figure 1: Experimental design. In the training data set (TS) the optimal classifier was established, and then applied to two validation data sets (VS1 and VS2). To test the specificity of this optimized classifier additional 1000 classifiers using random feature lists of equal size were permuted and applied to validation set 1 and 2. SVM = support vector machine, LDA = linear discrimination analysis, PAM = prediction analysis of microarrays, NSCLC = non-small cell lung cancer.
Figure 2: (A) Identification of the optimal algorithm for a classification based on the training set. The mean area under the receiver operator curve (AUC) is plotted against the cut-off p value of the T-statistics for feature selection for all three algorithms (SVM, LDA, PAM) in the 10-fold cross-validation of the training set. SVM leads to the highest mean AUC (mean AUC = 0.754) at a cut-off p-value of 0.003 which is highlighted by a dotted line. (B) The fold change of the genes with the most significant changes (p<0.003, fold change > 1.3 or <0.7, absolute difference >80) are shown. All transcripts used in the optimized classifier are given in Table 2.

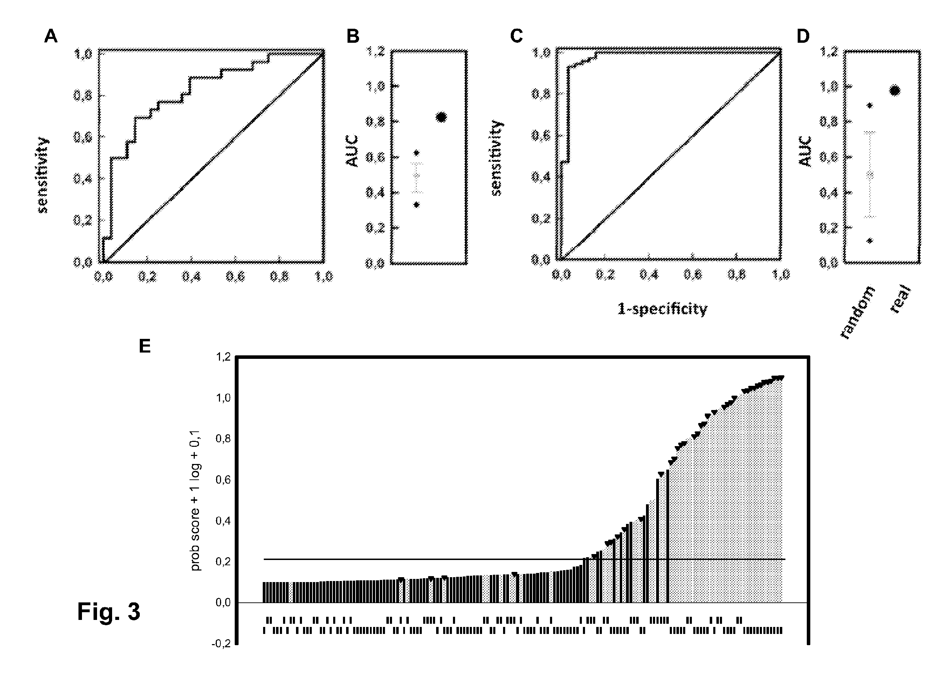

Figure 3: Performance of optimized classifier in validation set 1 and 2, respectively. The classifier established in the training set was applied to validation data set 1 and 2 respectively using SVM. (A) Receiver operating characteristic (ROC) curve for the optimized classifier applied to validation set 1 (VS1: all stage NSCLC patients and hospital based controls) AUC = 0.824, p<0.001. (B) The box plot comprises 1000 AUCs obtained by using 1000 random list of 484 features to build the classifier in TS and then apply it to VS1. The real AUC using the specific classifier (see Figure, 3A) is depicted. (C) ROC curve for the optimized classifier applied to validation set 2 (VS2: stage I NSCLC patients, healthy controls) AUC = 0.977 (p<0.001). (D) The box plot comprises AUCs obtained by applying 1000 randomly permutated classifiers of same feature size to validation set 2. The real AUC using the specific classifier (see Figure. 3C) is depicted. (E) Test scores to be a case of all samples from VS1 and VS2 were ranked. NSCLC cases are marked in grey and controls in black. Cases with stage I NSCLC are indicated by ▲. Membership in a specific cohort is indicated by a vertical line underneath the graph (VS1 upper row, VS2 lower row). A horizontal line is drawn at the threshold defined in TS.

### Description of the Lists

Lists 1 to 51 show exemplary sets of RNAs whose abundance in a blood sample from a human individual can be determined according to the invention to detect NSCLC in the individual. Each list shows a set of RNAs (defined by accession number and probe ID) with an area under the curve (AUC) of at least 0.8. The AUC is a quantitative parameter for the clinical utility (specificity and sensitivity) of the detection method described herein. An AUC of 1.0 refers to a sensitivity and specificity of 100 %.

### Examples

The invention is further described in the following non-limiting examples.

### Material and Methods

Cases and controls: NSCLC cases and hospital based controls were recruited at the University Hospital Cologne and the Lung Clinic Merheim, Cologne, Germany. Healthy blood donors were recruited at the Institute for Transfusion Medicine, University of Cologne. From all individuals, PAXgene stabilized blood samples were taken for blood-based gene expression profiling. For all NSCLC cases, blood was taken prior to chemotherapy. To establish and validate a NSCLC specific classifier, 3 independent sets of cases and controls were assembled. The training set (TS) comprised 77 individuals. 35 of those represent NSCLC cases of stage I-IV admitted to the hospital with symptoms of non-small cell lung cancer (coughing, dyspnea, weight loss or reduction in general health state) and 42 were hospital based controls with a comparable comorbidity but no prior history of lung cancer. The validation set 1 (VS1, n=54) likewise contained 28 NSCLC cases of stage I-IV and 26 hospital based controls. Overall, the hospital based controls in TS and VS1 enclosed individuals suffering from advanced chronic obstructive pulmonary disease (COPD) as typically seen in a population of heavily smoking adults (TS: n=7 VS1: n=5). Other diseases such as hypertension (TS: n=17, VS: n=11) or other malignancies (TS: n=10; VS1: n=6) were also observed in the group of hospital based controls. The validation set 2 (VS2, n=102) contained 32 NSCLC cases that had documented stage I NSCLC and were diagnosed mostly during routine chest-rays or due to clinical workup of unspecific symptoms such as reduced general health status. All individuals had an ECOG performance status of 0. In addition VS2 contains 70 healthy blood donors without prior history of lung cancer. Detailed information on cases and controls are summarized in Table 1. The analyses were approved by the local ethics committee and all probands gave informed consent.

Blood collection, cRNA synthesis and array hybridization: Blood (2.5 ml) was drawn into PAXgene vials. After RNA isolation biotin labeled cRNA preparation was performed using the Ambion® Illumina RNA amplification kit (Ambion, UK) or Epicentre TargetAmp™ Kit (Epicentre Biotechnologies, USA) and Biotin-16-UTP (10 mM; Roche Molecular Biochemicals) or Illumina® TotalPrep RNA Amplification Kit (Ambion, UK). Biotin labeled cRNA (1.5 µg) was hybridized to Sentrix® whole genome bead chips WG6 version 2, (Illumina, USA) and scanned on the Illumina® BeadStation 500x. For data collection, the inventors used Illumina® BeadStudio 3.1.1.0 software. Data are available at http://www.ncbi.nlm.nih.gov/geo/GSE12771).

Quality control: For RNA quality control the ratio of the OD at wavelengths of 260 nm and 280 nm was calculated for all samples and was between 1.85 and 2.1. To determine the quality of cRNA, a semi-quantitative RT-PCR amplifying a 5'prime and a 3'prime product of the β-actin gene was used as previously described (Zander T. et al., J. Med. Biol. Res., 39: 589-93 (2006)) and demonstrated no sign of degradation with the 5' prime and a 3'prime product being present. All expression data presented in this manuscript were of high quality. Quality of RNA expression data was controlled by different separate tools. First, the inventors performed quality control by visual inspection of the distribution of raw expression values. Therefore, the inventors constructed pairwise scatterplots of expression values from all arrays (R-project Vs 2.8.0) (Team RDC. R: A language and environment for statistical computing. R Foundation for Statistical Computing (2006)). For data derived from an array of good quality a high correlation of expression values is expected to lead to a cloud of dots along the diagonal. In all comparisons the r² was above 0.95. Second the present call rate was high in all samples. Finally, the inventors performed quantitative quality control. Here, the absolute deviation of the mean expression values of each array from the overall mean was determined (R-project Vs 2.8.0) (Team RDC. R: A language and environment for statistical computing. R Foundation for Statistical Computing (2006)). In short, the mean expression value for each array was calculated. Next the mean of these mean expression values (overall mean) was taken and the deviation of each array mean from the overall mean was determined (analogous to probe outlier detection used by Affymetrix before expression value calculation) (Affymetrix. Statistical algorithms description document; http:.//www.affymetrix.com/support/technical/whitepapers/sadd_whitepaper.pdf (2002)). The deviation was below 28 for all samples.

Classification algorithm: An overview of the experimental design is depicted in figure 1. Expression values were independently quantile normalized. The classifier for NSCLC was built and optimized based on the training set (TS, n=77; 35 NSCLC cases stage I-IV and 42 hospital based controls) using a 10 fold cross-validation design. Briefly TS was divided 10 times into an internal training and an internal validation set in a ratio 9:1 (distribution to internal validation group see Supplementary Table 1). In the internal training set the differentially expressed genes between NSCLC cases and controls were calculated using a T-test. Next, 36 different feature lists were extracted from this list of differential expressed genes by 36 times sequentially increasing the cut-off of the p-value (p = 0.00001, p = 0.00002, p = 0.00003 ...-...p = 0.08, p = 0.09, p = 0.1). Subsequently, for each of the resulting 36 feature lists 3 different learning algorithms (support vector machine (SVM), linear discrimination analysis (LDA), and prediction analysis for microarrays (PAM)) were trained on the internal training set and used to calculate the probability score for each case of the respective internal validation set. This approach was repeated 10 times according to the 10 dataset splittings of this 10-fold cross-validation. For each of the 10 cross-validation steps the area under the receiver operator curve (AUC) was calculated for the internal validation set. For each of the 36 cut-offs the mean of the 10 AUCs was calculated. Each of the 10 split data sets was used once as internal validation set. The optimal cut-off p value of the T-statistics and the optimal classification algorithm were selected according to the maximum mean AUC ever reached in all of the three algorithms (Figure 2). The inventors subsequently built a classifier using the respective cut-off p-value of the T-statistics and the selected algorithm in the TS. To further control for overfitting (Lee, S., Stat. Methods Med. Res., 17: 635-42 (2008)), the classifier was validated in 2 independent validation sets (VS1, comprising 28 NSCLC cases (stage I-IV) and 26 hospital based controls; VS2 comprising 32 NSCLC cases (stage I) and 70 healthy controls). The AUC was used to measure the quality of the classifier. In addition, the inventors determined a threshold of the test score in the training set to evaluate sensitivity and specificity in the validation sets. In order not to miss a potential case with NSCLC the inventors maximized the sensitivity to detect NSCLC requiring a minimum specificity (Akobeng, A., Acta Paediatr, 96: 644-7 (2007)). This specificity was defined to be at least 0.5 in its 95% confidence interval. Of note, the threshold fulfilling these criteria was determined in TS. Subsequently, all individuals in VS1 and VS2 reaching an equal or higher test score than the TS based threshold score were diagnosed as NSCLC cases and all others were diagnosed as controls. The sensitivity and specificity of this diagnostic test and its 95 % confidence interval was estimated for VS1 and VS2 (Newcombe, R. G., Stat. Med., 17: 857-72 (1998)) In addition, the inventors compared the probability scores to be a NSCLC case for each case and control using T statistics. To test the specificity of the classifier the whole analysis was repeated thousand times using random feature sets of equal size. For visualisation of the test score obtained by the SVM algorithm, the inventors used the following transformation algorithm (log2(score + 1) + 0.1.

Datamining: To investigate gene ontology of transcripts used for the classifier GeneTrail analysis for over- and underexpressed genes (Backes, C. et al., Nucleic Acids Res., 35: W186-92 (2007)) was performed. To this end, the inventors analyzed the enrichment in genes in the classifier compared to all genes present on the whole array. The inventors analyzed under- respectively over-expressed genes using the hypergeometric test with a minimum of 2 genes per category.

In addition, the inventors performed datamining by Gene Set Enrichment Analysis (GSEA) (Subramanian, A. et al., Proc. Natl. Acad. Sci. USA, 102: 15545-50 (2005)). As indicated, the inventors compared the respective list of genes obtained in the inventors' expression profiling experiment with datasets deposited in the Molecular Signatures Database (MSigDB). The power of the gene set analysis is derived from its focus on groups of genes that share common biological functions. In GSEA an overlap between predefined lists of genes and the newly identified genes can be identified using a running sum statistics that leads to attribution of a score. The significance of this score is tested using a permutation design which is adapted for multiple testing (Subramanian, A. et al., Proc. Natl. Acad. Sci. USA, 102: 15545-50 (2005)). Groups of genes, called gene sets were deposited in the MSigDB database and ordered in different biological dimensions such as cancer modules, canonical pathways, miRNA targets, GO-terms etc. (http://www.broadinstitute.org/gsea/-msigdb/index.jsp). In the analysis, the inventors focused on cancer modules. The cancer modules integrated into the MSigDB are derived from a compendium of 1975 different published microarrays spanning several different tumor entities (Segal, E. et al., Nat. Genet., 36: 1090-8 (2004)).

Experimental design: Expression profiles were generated from PAXgene stabilized blood samples from 3 independent groups consisting of NSCLC cases and controls (n = 77, 54, 102) using the Illumina WG6-VS2 system.

Results: Several genes are consistently differentially expressed in whole blood of NSCLC patients and controls. These expression profiles were used to build a diagnostic classifier for NSCLC which was validated in an independent validation set of NSCLC patients (stage I-IV) and hospital based controls. The area under the receiver operator curve (AUC) was calculated to be 0.824 (p<0.001). In a further independent dataset of stage I NSCLC patients and healthy controls the AUC was 0.977 (p<0.001). Specificity of the classifier was validated by permutation analysis in both validation cohorts. Genes within the classifier are enriched in immune associated genes and demonstrate specificity for non-small cell lung cancer. Conclusions: The inventors' results show that gene expression profiles of whole blood allow for detection of manifest NSCLC. These results prompted further development of gene expression based biomarker tests in peripheral blood for the diagnosis and early detection i.e. stage I lung cancer of NSCLC.

Establishment of a gene expression profiling-based classifier for blood-based diagnosis of NSCLC: The classifier was build based on an initial training set (TS) containing 35 NSCLC cases of different stages (stage I: n=5, stage II: n=5, stage III: n=17, stage IV: n=8) and 42 hospital based controls suffering in part from severe comorbidities such as COPD, hypertension, cardiac diseases as well as malignancies other than lung cancer. The inventors first evaluated three different approaches, namely support vector machine (SVM), linear discrimination analysis (LDA) and prediction analysis of microarrays (PAM) to identify the best algorithm to build a classifier for the diagnosis of NSCLC in a 10 fold cross-validation design. To this end, the inventors used 36 different feature lists extracted from the list of differentially expressed genes according to 36 different cut-off p-values of the T-statistics. In this setting the SVM algorithm performed best by reaching the highest AUC (mean AUC = 0.754) at a cut-off p-value of the T-statistics of 0.003 (Figure 2A). Thus for subsequent classification, the inventors applied SVM by using the 484 feature list obtained at a cut-off p-value of the T-statistics of p≤0.003 for differential expressed genes between cases and controls based on the entire training set. Fold-changes of genes with most significant p-values are shown in Figure 2B and all transcripts used in the classifier are summarized in Table 2. The inventors next maximized the sensitivity of the classifier requiring the 95% confidence interval of the specificity to still comprise 0.5. Using these criteria the threshold of the test score was determined to be 0.082. At this threshold of the test score sensitivity was determined to be 0.91 (0.75-0.97) and the specificity 0.38 (0.23 - 0.54), i.e. the 95 % confidence interval comprising 0.5.

Use of the diagnostic NSCLC classifier to detect NSCLC cases in an independent validation set of NSCLC cases and hospital based controls: First it was validated whether the classifier can be used to discriminate NSCLC cases of early and advanced stages among hospital based controls. Therefore, in the first independent validation set cases and controls were chosen in a similar setting as in the training set, i.e. patients with NSCLC stage I-IV and clinical symptoms associated with lung cancer and hospital based controls with relevant comorbidities (n=26). The AUC for the diagnostic test of NSCLC in this first validation set was calculated to be 0.824 (p<0.001) (Figure 3A). In addition, the inventors observed a significant difference between the SVM based probability scores to be a NSCLC case for actual NSCLC cases and controls in VS1 (p<0.001, T-test). Using the threshold determined in TS, the inventors observed a sensitivity of 0.61 (range 0.41 - 0.78) and a specificity of 0.85 (range 0.64 - 0.95) in VS1. Regarding only patients with stage III / IV non-small cell lung cancer (n = 20) in VS1 the sensitivity was 0.70 (range 0.46 - 0.87) and the specificity 0.85 (range 0.64 - 0.95; data not shown). The inventors observed that three out of three stage I NSCLC cases had a low score in this cohort of patients with a high degree of comorbidity (Figure 3E). Patients with NSCLC of advanced stages in VS1 were identified among hospital based controls using the threshold determined in the training set.

The diagnostic NSCLC classifier identifies stage 1 NSCLC patients in an independent second validation set comprising stage I NSCLC cases and healthy blood donors: After demonstrating that the classifier can be used to detect NSCLC cases among individuals with comorbidities it was also investigated whether this test can be used to distinguish NSCLC cases presenting at stage I with no or only minor symptoms from healthy individuals. Therefore, the inventors recruited a second independent validation set consisting of 32 NSCLC cases at stage I and 70 healthy blood donors (VS2). By applying the identical classifier to VS2 the AUC was determined to be 0.977 (p<0.001) (Figure 3C). Again the classifier was used as a diagnostic test thereby applying the TS-based threshold of the test score. At this threshold the sensitivity was 0.97 (0.82 - 0.99) and the specificity 0.89 (0.78 - 0.95). The inventors also observed a highly significant difference in the probability values to be a NSCLC patient for cases in contrast to controls (p<0.001, T-test). Healthy controls without significant comorbidity (VS2) tend to have lower probability scores compared to hospital based controls (VS1 and TS) although this finding was not statistically significant (Figure 3E). Of note, the difference in the probability score between healthy controls and patients with stage I lung cancer is more pronounced compared to the difference of probability scores between patients with NSCLC stage III/IV and patients with a similar high load of comorbidity.

Permutation test to analyse the specificity of the classifier: To further underline the specificity of this classifier, the inventors used 1000 random feature lists each comprising 484 features to likewise build a SVM-based classifier in the training set (TS) which then were applied to validation set 1 (VS1) and validation set 2 (VS2), respectively. For VS1 the mean AUC obtained by using these random feature lists was 0.49 (range 0.1346 - 0.8633) with only 2 AUCs being ≥ 0.824, the AUC obtained using the NSCLC specific classifier (Figure. 3B). This corresponds to a p-value of less than 0.002 for the permutation test further confirming the specificity of the NSCLC classifier. Similarly, by applying the permuted classifiers to VS2, only 1.8 % of random feature lists lead to an AUC of ≥ 0.977, the AUC obtained using the NSCLC-specific classifier (Figure. 3D). Furthermore, by merging TS and VS1 and randomly generating new dataset splitting in TS' and VS1' it could be demonstrated that highly specific classifiers can be built independently of the initial composition of the training set (data not shown). In conclusion, a NSCLC-specific blood-based classifier was build that was successfully used to identify NSCLC cases among hospital based controls as well as NSCLC cases of early stage among healthy individuals.

Mining of expression profiles: To analyze the biological significance of the extracted 484 features derived as classifier by the SVM approach different strategies were used. First, the inventors used GeneTrail (Backes, C. et al., Nucleic Acids Res., 35: W186-92 (2007)) to analyze an enrichment in GO-terms of the genes associated with NSCLC in the inventors' study. The inventors observed 112 GO categories demonstrating a significant (p-value FDR corrected < 0.05) enrichment of genes in the inventors' extracted gene list of which 25 were associated with the immune system (Table 3). These data indicate an impact of immune cells to the genes involved in the classifier.

Next, the inventors performed a gene set enrichment analysis ((Subramanian, A. et al., Proc. Natl. Acad. Sci. USA, 102: 15545-50 (2005); Segal, E. et al., Nat. Genet., 36: 1090-8 (2004)), thereby focusing on cancer modules which comprise groups of genes participating in biological processes related to cancer. Initially, the power of such modules has been demonstrated exemplarily for single genes such as cyclin D1 or PGC-1alpha (Lamb, J. et al., Cell, 114: 323-34 (2003); Mootha, V. K. et al., Nat. Genet., 34: 267-73 (2003)) and a more comprehensive view on such modules has been introduced recently (Segal, E. et al., Nat. Genet., 36: 1090-8 (2004)). This comprehensive collection of modules allows the identification of similarities across different tumor entities, such as the common ability of a tumor to metastasize to the bone e.g. in subsets of breast, lung and prostate cancer (Segal, E. et al., Nat. Genet., 36: 1090-8 (2004)). Overall, 456 such modules are described in the database spanning several biological processes such as metabolism, transcription, cell cycle and others.

When analysing the identified 484 NSCLC specific features, 199 cancer modules including 26 % of all NSCLC associated modules where identified to show a significant enrichment. This indicates that genes used to build a classifier for NSCLC cases in the inventors' study represent in part a subset of biologically cooperating genes that are also differentially expressed in primary lung cancer.

To further investigate the specificity of the extracted list of 484 features obtained from the analysis for the classification of NSCLC, the inventors also calculated the overlap between this extracted gene set and a set of genes differentially expressed in the blood of patients with renal cell cancer (Twine, N. C. et al., Cancer Res., 63: 6069-75 (2003); Sharma, P. et al., Breast Cancer Res., 7: R634-44 (2005)). No significant overlap was observed for both gene sets. Similarly, no overlap was observed between the inventors' NSCLC specific gene set and gene sets obtained from blood-based expression profiles specific for melanoma (Critchley-Thorne, R. J. et al., PLoS Med., 4: e176 (2007)), breast ( Sharma, P. et al., Breast Cancer Res., 7: R634-44 (2005)) and bladder (Osman, I. et al., Clin. Cancer Res., 12:3374-80 (2006)), respectively. In summary, these data point to a NSCLC specific gene set present in the inventors' classifier.

**Table 1: Clinical and epidemiological characteristics of cases with NSCLC and respective controls. TS = training set, VS1 = validation set 1, VS 2 = validation set 2, NSCLC = non-small lung cancer cases, T = Total number of cases resp. controls per data set, F = Females, M = Males, mA = median age, S = Stage, NA = not applicable, All = all cases, SCC = squamous cell cancer, AC = adenocarcinoma, LCC = large cell carcinoma**

| | | TS1 | | VS1 | | VS2 | |
|---|---|---|---|---|---|---|---|
| | | NSCLC | controls | NSCLC | controls | NSCLC | controls |
| T | | 35 | 42 | 28 | 26 | 32 | 70 |
| F | | 10 | 14 | 9 | 10 | 16 | 35 |
| M | | 25 | 28 | 19 | 16 | 16 | 35 |
| mA | | 61 | 61 | 62 | 65 | 67 | 44 |
| | | | | | | | |
| S1 | All | 5 | NA | 6 | NA | 32 | NA |
| | SCC | 2 | | 2 | | 4 | |
| | AC | 3 | | 4 | | 23 | |
| | LCC | 0 | | 0 | | 5 | |
| S2 | All | 5 | NA | 2 | NA | 0 | NA |
| | SCC | 1 | | 1 | | 0 | |
| | AC | 4 | | 1 | | 0 | |
| | LCC | 0 | | 0 | | 0 | |
| S3 | All | 17 | NA | 12 | NA | 0 | NA |
| | SCC | 4 | | 7 | | 0 | |
| | AC | 12 | | 5 | | 0 | |
| | LCC | 1 | | 0 | | 0 | |
| S4 | All | 8 | NA | 8 | | 0 | NA |
| | SCC | 3 | | 0 | | 0 | |
| | AC | 5 | | 7 | | 0 | |
| | LCC | 0 | | 1 | | 0 | |

**Table 2: RNAs whose abundance may be measured in the invention**

| RNA NO/ | | | | | | | |
|---|---|---|---|---|---|---|---|
| SEQ ID NO | ProbeId | Gid | Accession | Symbol | Diff | Quot | Ttest_p_value |
| 1 | 6290561 | 88998614 | XM_936120.1 | HLA-DQA1 | 315,8964785 | 1,64433008 | 0,000329596 |
| 2 | 2900463 | 7108345 | NM_012483.1 | GNLY | 926,3133937 | 1,612886994 | 0,002862239 |
| 3 | 3990639 | 36748 | X00437 | | 771,7174513 | 1,555765766 | 4,83E-05 |
| 4 | 670041 | 50477326 | CR596519 | | 548,9905075 | 1,522206107 | 3,32E-05 |
| 5 | 4850192 | 51036597 | NM_006725.2 | CD6 | 216,5093624 | 1,520125461 | 0,000132444 |
| 6 | 6480500 | 24797073 | NM_033554.2 | HLA-DPA1 | 588,5023681 | 1,477667953 | 0,00013302 |
| 7 | 460754 | 47078254 | NM_212530.1 | CDC25B | 218,4805426 | 1,466578356 | 0,000195968 |
| 8 | 5960035 | 24850108 | NM_170699.1 | GPBAR1 | 100,9412072 | 1,463343623 | 0,00015651 |
| 9 | 3420630 | 45331203 | NM_007181.3 | MAP4K1 | 141,7841323 | 1,45743426 | 0,000621345 |
| 10 | 1110215 | 14149679 | NM_015292.1 | FAM62A | 222,9573258 | 1,428150347 | 3,25E-05 |
| 11 | 1110575 | 51173716 | NM_006720.3 | ABLIM1 | 178,3940667 | 1,414951976 | 0,000520001 |
| 12 | 2760092 | 34147517 | NM_005449.3 | FAIM3 | 201,7565646 | 1,409529365 | 0,000634628 |
| 13 | 1170307 | 23238195 | NM_000878.2 | IL2RB | 146,6316959 | 1,40578311 | 0,000500983 |
| 14 | 6960746 | 55774984 | NM_018384.3 | GIMAP5 | 223,2919717 | 1,382086438 | 4,02E-05 |
| 15 | 2680370 | 52426773 | NM_019111.3 | HLA-DRA | 817,1291554 | 1,377401879 | 0,000326414 |
| 16 | 1820598 | 12545398 | NM_000873.2 | ICAM2 | 182,5185371 | 1,351682752 | 3,89E-05 |
| 17 | 4010053 | 18641371 | NM_022555.3 | HLA-DRB3 | 131,0547184 | 1,349610681 | 0,001995927 |
| 18 | 6760487 | 47778936 | NM_014164.4 | FXYD5 | 393,3630931 | 1,33904597 | 0,001262983 |
| 19 | 5560280 | 29029549 | NM_017424.2 | CECR1 | 301,0469939 | 1,32885363 | 0,002167663 |
| 20 | 4900053 | 17978510 | NM_014281.3 | SIAHBP1 | 189,9410753 | 1,323848811 | 2,21E-05 |
| 21 | 4760543 | 34335248 | NM_153719.2 | NUP62 | 118,4147225 | 1,307105002 | 1,39E-05 |
| 22 | 4590082 | 17999536 | NM_006445.2 | PRPF8 | 125,5348006 | 1,304518217 | 0,000927196 |
| 23 | 2690609 | 62526019 | NM_001014433.2 | CUTA | 223,2296564 | 1,301630178 | 0,000120692 |
| 24 | 650164 | 4503240 | NM_000896.1 | CYP4F3 | -315,5464725 | 0,699584557 | 3,55E-05 |
| 25 | 6220450 | 40548399 | NM_005771.3 | DHRS9 | -181,3140503 | 0,694871194 | 0,001532317 |
| 26 | 3460674 | 47419935 | NM_003137.3 | SRPK1 | -139,764433 | 0,694069927 | 3,60E-05 |
| 27 | 3990296 | 9951925 | NM_000717.2 | CA4 | -213,0886356 | 0,693534636 | 0,001400277 |
| 28 | 160132 | 59938769 | NM_182898.2 | CREB5 | -262,1114924 | 0,69200725 | 0,00019583 |
| 29 | 6110088 | 21536375 | NM_005502.2 | ABCA1 | -111,4628782 | 0,659789914 | 0,002134427 |
| 30 | 6350364 | 41872408 | NM_002704.2 | PPBP | -761,2674927 | 0,651771499 | 0,000630226 |
| 31 | 2640609 | 45827727 | NM_005980.2 | S100P | -390,18098 | 0,625545099 | 0,000253144 |
| 32 | 1430762 | 6005791 | NM_007199.1 | IRAK3 | -155,6188523 | 0,616800598 | 0,000474504 |
| 33 | 5130475 | 27477087 | NM_003853.2 | IL18RAP | -632,4410294 | 0,583984119 | 0,000851483 |
| 34 | 1500328 | 27477086 | NM_003855.2 | IL18R1 | -122,3006055 | 0,528089291 | 0,001627845 |
| 35 | 2370524 | 26051242 | NM_007115.2 | TNFAIP6 | -189,5036814 | 0,489548363 | 1,09E-06 |
| 36 | 7550537 | 4502098 | NM_001152.1 | SLC25A5 | 326,6719362 | 1,197818187 | 1,44E-05 |
| 37 | 4280332 | 14251213 | NM_020414.3 | DDX24 | 75,4163163 | 1,309570343 | 3,02E-05 |
| 38 | 3360364 | 7662339 | NM_014941.1 | MORC2 | 54,87417822 | 1,317774309 | 4,03E-05 |
| 39 | 1780348 | 70908369 | NM_018285.2 | IMP3 | 148,6064801 | 1,240632612 | 5,80E-05 |
| 40 | 450037 | 55925577 | NM_002348.2 | LY9 | 64,18668944 | 1,337080038 | 7,31E-05 |
| 41 | 6980070 | 13929470 | NM_004776.2 | B4GALT5 | -121,2712825 | 0,720729919 | 9,61E-05 |
| 42 | 7000133 | 12597634 | NM_022898.1 | BCL11B | 87,76854653 | 1,366299623 | 0,000470008 |
| 43 | 1400270 | 20336247 | NM_004716.2 | PCSK7 | 94,29387128 | 1,302793128 | 0,000716583 |
| 44 | 150632 | 48762708 | NM_022136.3 | SAMSN1 | -80,93158519 | 0,591012921 | 0,000781956 |
| 45 | 6420541 | 34535726 | AK128384 | | 87,13245532 | 1,405980431 | 0,001078556 |
| 46 | 7650025 | 40806176 | NM_004949.2 | DSC2 | -93,38175241 | 0,642737193 | 0,001292334 |
| 47 | 5960128 | 21327699 | NM_003487.2 | TAF15 | 87,80187716 | 1,346679516 | 0,001679561 |
| 48 | 6420079 | 89027874 | XM_291277.4 | DKFZp761P0423 | 80,22901015 | 1,337704578 | 0,001921658 |
| 49 | 4590646 | 67089144 | NM_001024667.1 | FCRL3 | 94,2507356 | 1,380488839 | 0,002010981 |
| 50 | 510450 | 29789089 | NM_018715.1 | RCC2 | 88,67276932 | 1,267465318 | 1,24E-05 |
| 51 | 7380288 | 34147585 | NM_016237.3 | ANAPC5 | 91,54539975 | 1,176438251 | 8,97E-05 |
| 52 | 5900156 | 57013275 | NM_006082.2 | K-ALPHA-1 | 543,1415197 | 1,219305455 | 0,000108016 |
| 53 | 7100136 | 41352720 | NM_078483.2 | SLC36A1 | -133,4968039 | 0,700431238 | 0,000133553 |
| 54 | 2630451 | 22027639 | NM_003528.2 | HIST2H2BE | -106,9694117 | 0,715024497 | 0,000134858 |
| 55 | 6280754 | 18426908 | NM_080816.1 | SIRPB2 | 30,07920138 | 1,308367153 | 0,000139409 |
| 56 | 4210647 | 13375800 | NM_024600.1 | C16orf30 | 31,66193318 | 1,346066522 | 0,000170914 |
| 57 | 5700403 | 31982913 | NM_032118.2 | WDR54 | 67,77289263 | 1,331399486 | 0,000188678 |
| 58 | 6270021 | 14141153 | NM_031203.1 | HNRPM | 175,5084406 | 1,233305528 | 0,00023295 |
| 59 | 1710286 | 21536460 | NM_002405.2 | MFNG | 186,80371 | 1,213068636 | 0,000240549 |
| 60 | 2630154 | 56676368 | NM_031412.2 | GABARAPL1 | -174,3602129 | 0,768240176 | 0,000244873 |
| 61 | 4060131 | 4758669 | NM_004811.1 | LPXN | 169,3070648 | 1,22742049 | 0,000265747 |
| 62 | 610563 | 14141173 | NM_002129.2 | HMGB2 | -47,59152899 | 0,643902556 | 0,000271029 |
| 63 | 6290431 | 49472821 | NM_003755.2 | EIF3S4 | 289,038097 | 1,265068842 | 0,000284449 |
| 64 | 7150017 | 58331183 | NM_006429.2 | CCT7 | 38,59039574 | 1,316676229 | 0,000311432 |
| 65 | 3170092 | 52851435 | NM_015710.3 | GLTSCR2 | 951,7801905 | 1,265887651 | 0,000319621 |
| 66 | 6940242 | 13128859 | NM_004964.2 | HDAC1 | 149,0464951 | 1,226649864 | 0,000355245 |
| 67 | 5130082 | 20149593 | NM_007355.2 | HSP90AB1 | 101,2393963 | 1,291963927 | 0,000372701 |
| 68 | 5310653 | 6912575 | NM_012387.1 | PADI4 | -903,4667472 | 0,718802076 | 0,000399258 |
| 69 | 380685 | 34147607 | NM_015953.3 | NOSIP | 201,9140045 | 1,2941855 | 0,00044799 |
| 70 | 3440431 | 29337284 | NM_012110.2 | CHIC2 | -117,5328049 | 0,822270062 | 0,000549514 |
| 71 | 4180142 | 51093838 | NM_016091.2 | EIF3S6IP | 296,2042703 | 1,221453733 | 0,000621455 |
| 72 | 1260228 | 10863876 | NM_021105.1 | PLSCR1 | -53,52413775 | 0,686231584 | 0,00064387 |
| 73 | 840730 | 40786401 | NM_199337.1 | LOC374395 | 170,1866859 | 1,178701902 | 0,000665379 |
| 74 | 3370605 | 45237199 | NM_020808.1 | SIPA1L2 | -58,97844017 | 0,687553371 | 0,000697156 |
| 75 | 5310136 | 75991708 | NM_001033667.1 | LY9 | 33,64428254 | 1,319047944 | 0,000716611 |
| 76 | 6380148 | 15431305 | NM_033301.1 | RPL8 | 855,395502 | 1,170817986 | 0,000768045 |
| 77 | 4050398 | 47132594 | NM_213611.1 | SLC25A3 | 190,2461506 | 1,160808106 | 0,000837252 |
| 78 | 6940246 | 292820 | M97723 | | 20,54719112 | 1,328176844 | 0,000841772 |
| 79 | 1690189 | 24234685 | NM_153201.1 | HSPA8 | 155,7459284 | 1,215840724 | 0,000855179 |
| 80 | 3890095 | 58696421 | NM_002304.1 | LFNG | 232,3486066 | 1,232868306 | 0,001042389 |
| 81 | 6770348 | 19920316 | NM_006825.2 | CKAP4 | -161,4085766 | 0,746907843 | 0,001086179 |
| 82 | 2000551 | 10092611 | NM_016326.2 | CKLF | -325,605478 | 0,801639824 | 0,001103698 |
| 83 | 6350360 | 50593531 | NM_005176.4 | ATP5G2 | 516,754743 | 1,243669891 | 0,00114794 |
| 84 | 160240 | 21040370 | NM_005804.2 | DDX39 | 101,9627944 | 1,230286148 | 0,001187406 |
| 85 | 360066 | 7019566 | NM_013378.1 | VPREB3 | 58,73278863 | 1,504196763 | 0,001230244 |
| 86 | 3800253 | 89035372 | XM_932678.1 | LOC387841 | 189,24229 | 1,278629303 | 0,001252967 |
| 87 | 3400328 | 47078294 | NM_000022.2 | ADA | 67,32199518 | 1,313091331 | 0,001361042 |
| 88 | 3800243 | 5031976 | NM_005746.1 | PBEF1 | -348,2606482 | 0,718301277 | 0,001419039 |
| 89 | 650541 | 45935370 | NM_002727.2 | PRG1 | -1496,501337 | 0,775793901 | 0,00150564 |
| 90 | 2100221 | 40353209 | NM_017758.2 | ALKBH5 | 161,2343238 | 1,161468316 | 0,001576054 |
| 91 | 6280576 | 21614543 | NM_002964.3 | S100A8 | -3148,764309 | 0,740939542 | 0,001592006 |
| 92 | 4780672 | 68216098 | NM_012413.3 | QPCT | -224,4635907 | 0,775514046 | 0,001600979 |
| 93 | 2510133 | 4506782 | NM_003864.1 | SAP30 | -78,20837572 | 0,607275759 | 0,001706434 |
| 94 | 770458 | 70995210 | NM_001398.2 | ECH1 | 140,6533489 | 1,216657159 | 0,001780796 |
| 95 | 2970768 | 4503528 | NM_001416.1 | EIF4A1 | 408,0155473 | 1,170835965 | 0,001793442 |
| 96 | 2320309 | 89037018 | XM_941325.1 | LOC652025 | 117,8979784 | 1,237697761 | 0,001829317 |
| 97 | 4220181 | 4505370 | NM_002496.1 | NDUFS8 | 171,4535635 | 1,260630153 | 0,001853457 |
| 98 | 7380170 | 28178815 | NM_174855.1 | IDH3B | 104,9723088 | 1,205100893 | 0,001927203 |
| 99 | 520647 | 20149601 | NM_012229.2 | NT5C2 | -220,1185076 | 0,806670139 | 0,001970138 |
| 100 | 2710711 | 23200005 | NM_149379.1 | NSUN5C | 34,34062961 | 1,320832601 | 0,002020028 |
| 101 | 2680682 | 47157327 | NM_213636.1 | PDLIM7 | -106,9173695 | 0,774721384 | 0,002138706 |
| 102 | 3610634 | 41327713 | NM_020531.2 | C20orf3 | -169,2466369 | 0,762496036 | 0,002151473 |
| 103 | 5860075 | 27436923 | NM_003517.2 | HIST2H2AC | -148,5459367 | 0,787951043 | 0,002154776 |
| 104 | 1030039 | 21328454 | NM_003516.2 | HIST2H2AA | -122,2515261 | 0,785390725 | 0,002180554 |
| 105 | 5900021 | 11072092 | NM_018031.2 | WDR6 | 110,7543183 | 1,289322955 | 0,002181731 |
| 106 | 2650390 | 33598947 | NM_002660.2 | PLCG1 | 65,59369512 | 1,328004119 | 0,002261605 |
| 107 | 20154 | 33624782 | NM_145799.2 | | 74,78050355 | 1,302850102 | 0,002265789 |
| 108 | 830400 | 9845520 | NM_002965.2 | S100A9 | -1676,927338 | 0,75302877 | 0,002267466 |
| 109 | 3990328 | 37577120 | NM_080387.4 | CLEC4D | -47,99765081 | 0,635020703 | 0,002285359 |
| 110 | 2970537 | 15431299 | NM_000980.2 | RPL18A | 1096,761159 | 1,208535779 | 0,002326571 |
| 111 | 5420538 | 6552334 | NM_000852.2 | GSTP1 | 265,0758172 | 1,212234469 | 0,002377771 |
| 112 | 240608 | 6042201 | NM_007288.1 | MME | -221,958069 | 0,761262136 | 0,002402057 |
| 113 | 10025 | 40788009 | NM_000574.2 | CD55 | -178,8297083 | 0,72532828 | 0,002428894 |
| 114 | 290452 | 47519615 | NM_213674.1 | TPM2 | 30,21632141 | 1,308702924 | 0,002451226 |
| 115 | 7400121 | 13435358 | NM_001923.2 | DDB1 | 229,4803921 | 1,262296307 | 0,002565468 |
| 116 | 3130300 | 50345983 | NM_001001937.1 | ATP5A1 | 225,076896 | 1,147238723 | 0,002626228 |
| 117 | 6660709 | 6006013 | NM_000632.2 | ITGAM | -167,4535448 | 0,769754788 | 0,002684536 |
| 118 | 2900482 | 4503518 | NM_003754.1 | EIF3S5 | 175,2963427 | 1,186295643 | 0,002708745 |
| 119 | 6350376 | 24234684 | NM_006597.3 | HSPA8 | 245,6853166 | 1,206537243 | 0,002730009 |
| 120 | 2070152 | 32130534 | NM_017801.2 | CMTM6 | -188,8631566 | 0,768636189 | 0,002746935 |
| 121 | 6650020 | 4501944 | NM_001124.1 | ADM | -270,3778539 | 0,770943537 | 0,002748971 |
| 122 | 5720458 | 8051633 | NM_004585.2 | RARRES3 | 228,1363976 | 1,255356881 | 0,002841093 |
| 123 | 5720347 | 14589868 | NM_032966.1 | BLR1 | 33,77133084 | 1,352619823 | 0,002843529 |
| 124 | 4890192 | 21396481 | NM_003512.3 | HIST1H2AC | -112,3541472 | 0,718192715 | 0,002850535 |
| 125 | 940348 | 21614521 | NM_012198.2 | GCA | -659,6345279 | 0,731881578 | 0,002891609 |
| 126 | 2750626 | 25453476 | NM_001961.2 | EEF2 | 556,3584728 | 1,181343779 | 0,002930668 |
| 127 | 1780035 | 31377794 | NM_006888.2 | CALM1 | 119,0389403 | 1,140092427 | 0,002955045 |
| 128 | 840564 | 23510386 | NM_153050.1 | MTMR3 | -134,6471185 | 0,837440801 | 0,002971284 |
| 129 | 6270128 | 58331233 | NM_000074.2 | CD40LG | 19,30987052 | 1,299106772 | 1,76E-05 |
| 130 | 2630484 | 88986422 | XM_927593.1 | ATP10B | -3,310535431 | 0,927138841 | 1,93E-05 |
| 131 | 520332 | 22165415 | NM_000155.2 | GALT | 15,96763751 | 1,182575744 | 2,05E-05 |
| 132 | 4730195 | 21264599 | NM_003543.3 | HIST1H4H | -35,48000424 | 0,721118446 | 2,49E-05 |
| 133 | 3120301 | 51173749 | NM_031488.4 | L3MBTL2 | 23,21749718 | 1,214091824 | 4,02E-05 |
| 134 | 6110768 | 39725714 | NM_004044.4 | ATIC | 72,99433158 | 1,297103547 | 4,63E-05 |
| 135 | 7150634 | 5902011 | NM_006901.1 | MY09A | 15,79647532 | 1,176790608 | 4,89E-05 |
| 136 | 6590386 | 20149679 | NM_024619.2 | FN3KRP | 63,48415847 | 1,276544672 | 4,98E-05 |
| 137 | 5690037 | 31543829 | NM_013270.2 | TSP50 | -4,520475047 | 0,914368497 | 5,40E-05 |
| 138 | 4540082 | 58331160 | NM_015651.1 | PHF19 | 27,47614989 | 1,23686341 | 7,56E-05 |
| 139 | 6380598 | 15529981 | NM_033416.1 | IMP4 | 34,49530681 | 1,205303684 | 7,96E-05 |
| 140 | 3520192 | 47132578 | NM_004401.2 | DFFA | 13,05479272 | 1,196479136 | 7,97E-05 |
| 141 | 2450497 | 314309 | T03068 | | 2,937619578 | 1,076034692 | 9,15E-05 |
| 142 | 7320576 | 4503512 | NM_003757.1 | EIF3S2 | 92,09473895 | 1,176647021 | 0,000308301 |
| 143 | 1300768 | 24497575 | NM_006066.2 | AKR1A1 | 80,92050915 | 1,237851126 | 0,000645996 |
| 144 | 5890327 | 24497579 | NM_001628.2 | AKR1B1 | 83,65924065 | 1,273311325 | 0,000719704 |
| 145 | 460273 | 35493970 | NM_003345.3 | UBE2I | 90,10026341 | 1,134131115 | 0,001168429 |
| 146 | 4920577 | 76781490 | NM_007047.3 | BTN3A2 | 93,80802363 | 1,266607439 | 0,001191858 |
| 147 | 650358 | 71773149 | NM_000485.2 | APRT | 84,63692212 | 1,280817312 | 0,001674124 |
| 148 | 1190647 | 18375504 | NM_080649.1 | APEX1 | 89,44973593 | 1,233840385 | 0,001687003 |
| 149 | 5960301 | 38026968 | NM_004649.4 | C21orf33 | 84,66892254 | 1,184726723 | 0,001806028 |
| 150 | 6290239 | 33469921 | NM_182776.1 | MCM7 | 85,43037244 | 1,23369979 | 0,002059443 |
| 151 | 4280373 | 11496980 | NM_003174.2 | SVIL | -86,77630886 | 0,817298661 | 0,002251248 |
| 152 | 5570750 | 31742497 | NM_015374.1 | UNC84B | 95,70834076 | 1,215821376 | 0,002516183 |
| 153 | 1780152 | 68161540 | NM_001024912.1 | CEACAM1 | -98,14974577 | 0,731931222 | 0,00292068 |
| 154 | 5390131 | 33469915 | NM_003906.3 | MCM3AP | 32,02638688 | 1,143678564 | 0,000106053 |
| 155 | 1240603 | 34452172 | NM_000887.3 | ITGAX | -22,98226817 | 0,818214716 | 0,000107483 |
| 156 | 2190673 | 41281472 | NM_014738.2 | KIAA0195 | 45,96224495 | 1,262020095 | 0,000110774 |
| 157 | 1580348 | 25141321 | NM_015039.2 | NMNAT2 | 6,84304916 | 1,124383533 | 0,000120767 |
| 158 | 4220138 | 89057421 | XM_940198.1 | LOC651076 | 10,13757955 | 1,165397115 | 0,000128251 |
| 159 | 3610504 | 38788307 | NM_005476.3 | GNE | 18,30602894 | 1,161672234 | 0,000133964 |
| 160 | 3520082 | 4506752 | NM_003707.1 | RUVBL1 | 11,02056207 | 1,158636454 | 0,000135201 |
| 161 | 7160296 | 70980548 | NM_014976.1 | PDCD11 | 21,33989507 | 1,221159345 | 0,000135859 |
| 162 | 1050128 | 8515897 | AF272739 | | 5,425986954 | 1,113600395 | 0,000136921 |
| 163 | 4730577 | 39890659 | CK300859 | | -3,693681288 | 0,918211879 | 0,000137667 |
| 164 | 5910091 | 30425443 | NM_178510.1 | ANKK1 | 2,967139784 | 1,069192658 | 0,000157493 |
| 165 | 1430292 | 40254427 | NM_000578.2 | SLC11A1 | -53,76208405 | 0,700846833 | 0,000167687 |
| 166 | 3460189 | 31652246 | NM_139244.2 | STXBP5 | -30,43225917 | 0,777631906 | 0,000172103 |
| 167 | 3140093 | 47717138 | NM_006767.2 | LZTR1 | 65,30433309 | 1,271997612 | 0,000186989 |
| 168 | 1440601 | 28872781 | NM_016408.2 | CDK5RAP1 | 25,2613611 | 1,174006584 | 0,000202701 |
| 169 | 3830390 | 89035828 | XM_930898.1 | KIAA0692 | -2,717873701 | 0,926116051 | 0,000205305 |
| 170 | 5860196 | 23659890 | BU733214 | | -2,809152673 | 0,934353946 | 0,000218212 |
| 171 | 3420735 | 40556369 | NM_015288.4 | PHF15 | 69,78943303 | 1,295589549 | 0,000222283 |
| 172 | 7050543 | 32967306 | NM_182480.1 | COQ6 | 13,29427898 | 1,129169056 | 0,000225323 |
| 173 | 5290289 | 34147412 | NM_032312.2 | YIPF4 | -50,40343731 | 0,765323005 | 0,000234467 |
| 174 | 6590523 | 56119213 | NM_024711.3 | GIMAP6 | 61,57201639 | 1,19443786 | 0,000236996 |
| 175 | 6020402 | 24497619 | NM_014230.2 | SRP68 | 28,09091984 | 1,155068368 | 0,000260218 |
| 176 | 5550746 | 63029931 | NM_152272.2 | CHMP7 | 43,03363654 | 1,222857885 | 0,00026052 |
| 177 | 160736 | 7706484 | NM_016292.1 | TRAP1 | 20,89088823 | 1,24310651 | 0,000270249 |
| 178 | 3840022 | 12707569 | NM_004092.2 | ECHS1 | 48,95693214 | 1,230257197 | 0,000274708 |
| 179 | 20647 | 40807451 | NM_015062.3 | PPRC1 | 20,40411099 | 1,172512955 | 0,000283424 |
| 180 | 3370687 | 16445420 | NM_052837.1 | SCAMP3 | 67,26265306 | 1,266481175 | 0,000291089 |
| 181 | 5310754 | 55956897 | NM_015935.4 | KIAA0859 | 13,16095145 | 1,140721813 | 0,000296022 |
| 182 | 2710397 | 24497489 | NM_003059.2 | SLC22A4 | -40,49246287 | 0,777411881 | 0,000298583 |
| 183 | 4850541 | 75750528 | NM_016552.2 | ANKMY1 | 2,735334806 | 1,065736829 | 0,000317215 |
| 184 | 6100220 | 6005951 | NM_007255.1 | B4GALT7 | 29,14447929 | 1,228247363 | 0,000318886 |
| 185 | 10541 | 13194198 | NM_023003.1 | TM6SF1 | -42,63413583 | 0,75244874 | 0,000319057 |
| 186 | 5720398 | 31341089 | NM_173791.2 | PDZD8 | -20,13545497 | 0,805850726 | 0,000321296 |
| 187 | 4540301 | 45827722 | NM_206914.1 | DKFZP586D0919 | 11,93196859 | 1,166543095 | 0,000322081 |
| 188 | 2100035 | 31543661 | NM_004226.2 | STK17B | -56,28618761 | 0,726985134 | 0,000323173 |
| 189 | 5090477 | 20336255 | NM_138687.1 | PIP5K2B | 4,198748845 | 1,077633516 | 0,000323344 |
| 190 | 130403 | 56550113 | NM_006567.2 | FARS2 | 15,61874075 | 1,144473414 | 0,000328093 |
| 191 | 5390669 | 5453556 | NM_006321.1 | ARIH2 | 43,41521242 | 1,16984795 | 0,000330449 |
| 192 | 2760563 | 77404351 | NM_001414.2 | EIF2B1 | 26,53165567 | 1,152983904 | 0,000332202 |
| 193 | 1440382 | 33359700 | NM_182688.1 | UBE2G2 | 39,95896434 | 1,258246254 | 0,000339206 |
| 194 | 2260446 | 89027447 | XM_935843.1 | LOC653832 | -3,084729823 | 0,927141 | 0,000346447 |
| 195 | 2030544 | 89026490 | XM_936353.1 | LOC642196 | 6,598817511 | 1,139296555 | 0,000346604 |
| 196 | 1440546 | 73486657 | NM_002080.2 | GOT2 | 48,7873308 | 1,230560635 | 0,00035799 |
| 197 | 5270500 | 16936531 | NM_000075.2 | CDK4 | 39,00645813 | 1,28126712 | 0,000367439 |
| 198 | 2750408 | 88971019 | XM_939707.1 | RUVBL1 | 16,22168355 | 1,191413103 | 0,000375432 |
| 199 | 6980274 | 16199327 | BI915661 | | 3,572189393 | 1,088541696 | 0,000376532 |
| 200 | 4200538 | 7657121 | NM_015721.1 | GEMIN4 | 29,79545919 | 1,244092425 | 0,000387475 |
| 201 | 6550142 | 6552335 | NM_003819.2 | PABPC4 | 43,32832748 | 1,217948334 | 0,000387574 |
| 202 | 7000408 | 13376877 | NM_025263.1 | PRR3 | 10,71405587 | 1,132753454 | 0,000391734 |
| 203 | 3140246 | 40255000 | NM_033111.2 | LOC88523 | -78,75175311 | 0,776953228 | 0,000391929 |
| 204 | 4230653 | 24497586 | NM_139247.2 | ADCY4 | -35,6351327 | 0,787114348 | 0,000397239 |
| 205 | 3420343 | 46409267 | NM_207311.1 | CCDC64 | 30,70426568 | 1,287470221 | 0,00040278 |
| 206 | 4150402 | 2963496 | AA868051 | | 2,919731911 | 1,06730094 | 0,000407376 |
| 207 | 2100333 | 38683839 | NM_198196.1 | CD96 | 51,45503197 | 1,281782623 | 0,000407931 |
| 208 | 4150161 | 4502676 | NM_001779.1 | CD58 | -48,65350702 | 0,744869256 | 0,000410831 |
| 209 | 4880364 | 13129091 | NM_024092.1 | TMEM109 | 61,87509923 | 1,260473608 | 0,0004333 |
| 210 | 2230661 | 20428651 | NM_005356.2 | LCK | 28,15648785 | 1,240489001 | 0,000447815 |
| 211 | 2450343 | 27828388 | BX096685 | | 4,718261846 | 1,099375057 | 0,000459923 |
| 212 | 6840408 | 2354214 | AA576740 | | 2,46572254 | 1,061515186 | 0,000464263 |
| 213 | 3140039 | 21314659 | NM_016230.2 | CYB5R4 | -69,25618425 | 0,757186116 | 0,000474673 |
| 214 | 2900524 | 41352701 | NM_001384.3 | DPH2 | 30,82374927 | 1,211102665 | 0,000489612 |
| 215 | 6480170 | 52632380 | NM_001005333.1 | MAGED1 | 44,33620059 | 1,280407525 | 0,000496684 |
| 216 | 4120133 | 31542722 | NM_022827.2 | SPATA20 | 22,43434012 | 1,234512657 | 0,000496736 |
| 217 | 4780743 | 4885224 | NM_005243.1 | EWSR1 | 65,31825131 | 1,149756619 | 0,000520842 |
| 218 | 7150601 | 7661785 | NM_014165.1 | C6orf66 | 6,495115777 | 1,112031506 | 0,000522811 |
| 219 | 5310050 | 12232384 | NM_022730.1 | COPS7B | 20,43313854 | 1,159974145 | 0,000523392 |
| 220 | 3130091 | 46358357 | NM_017905.3 | TMCO3 | -52,34722897 | 0,774257356 | 0,000525503 |
| 221 | 4860132 | 7662451 | NM_015239.1 | AGTPBP1 | -78,07936872 | 0,798836295 | 0,000529903 |
| 222 | 3780689 | 70608210 | NM_016489.11 | NT5C3 | -33,80520978 | 0,771229 | 0,000530225 |
| 223 | 1030692 | 27436906 | NM_004927.2 | MRPL49 | 41,36497592 | 1,196106353 | 0,000541915 |
| 224 | 4830435 | 39812071 | NM_030811.3 | MRPS26 | 24,14041112 | 1,209465158 | 0,000546594 |
| 225 | 4830563 | 33620778 | NM_012321.2 | LSM4 | 46,65393084 | 1,19050163 | 0,000557036 |
| 226 | 3940368 | 89057924 | XM_928933.1 | FLJ32154 | 2,730511524 | 1,07060656 | 0,000558628 |
| 227 | 1980424 | 13129119 | NM_024108.1 | TRAPPC6A | 30,5100061 | 1,204130632 | 0,000560504 |
| 228 | 3130711 | 22748936 | NM_020750.1 | XPO5 | 12,09222819 | 1,135424285 | 0,000572639 |
| 229 | 620722 | 4506444 | NM_002896.1 | RBM4 | 43,5385138 | 1,203671935 | 0,000578613 |
| 230 | 3400504 | 66933015 | NM_000884.2 | IMPDH2 | 69,11593701 | 1,25700787 | 0,000583684 |
| 231 | 2750521 | 57634533 | NM_015135.1 | NUP205 | 29,42766456 | 1,221266897 | 0,000585225 |
| 232 | 4060056 | 55749643 | NM_015054.1 | KIAA0701 | -12,83668481 | 0,883051575 | 0,000597219 |
| 233 | 4640044 | 21040335 | NM_138931.1 | BCL6 | -5,775066733 | 0,890317809 | 0,00060429 |
| 234 | 6110537 | 88967388 | XM_931928.1 | LOC642816 | 2,637522553 | 1,065666772 | 0,000612974 |
| 235 | 4640528 | 13435126 | NM_025228.1 | TRAF3IP3 | 75,87438371 | 1,216408031 | 0,000615875 |
| 236 | 5860400 | 19557649 | NM_021052.2 | HIST1H2AE | -11,74106542 | 0,820379932 | 0,000618118 |
| 237 | 990273 | 34222323 | NM_015380.3 | SAMM50 | 47,44338392 | 1,204119339 | 0,00063726 |
| 238 | 3990465 | 89038158 | XM_496059.2 | LOC440261 | 2,890329302 | 1,065568707 | 0,000641851 |
| 239 | 4860296 | 42558244 | NM_032320.4 | BTBD10 | -25,33553958 | 0,830860508 | 0,000644443 |
| 240 | 1110358 | 27469749 | BC041984 | | -5,896873872 | 0,901523403 | 0,000669438 |
| 241 | 5130767 | 52352808 | NM_005902.3 | SMAD3 | 27,20027806 | 1,165895949 | 0,00067007 |
| 242 | 6980609 | 40255221 | NM_178839.3 | LRRTM1 | -3,098540364 | 0,930415728 | 0,000677469 |
| 243 | 2630719 | 57222567 | NM_005937.2 | MLLT6 | 67,91872541 | 1,270330562 | 0,000681074 |
| 244 | 7380274 | 68509917 | NM_007167.2 | ZMYM6 | 52,66472418 | 1,182129748 | 0,000687399 |
| 245 | 6480328 | 39725684 | NM_025150.3 | TARSL1 | 6,593485366 | 1,123027001 | 0,000690243 |
| 246 | 5820348 | 48314819 | NM_017570.1 | OPLAH | -45,18509526 | 0,713924556 | 0,000703103 |
| 247 | 130113 | 56699400 | NM_052901.2 | SLC25A25 | 18,42042548 | 1,167822465 | 0,000707163 |
| 248 | 6860243 | 24430138 | NM_015540.2 | RPAP1 | 7,937422972 | 1,134855062 | 0,00071637 |
| 249 | 5900414 | 5031814 | NM_005548.1 | KARS | 23,41578115 | 1,134554539 | 0,000730069 |
| 250 | 1340338 | 89041598 | XM_377476.3 | MGC57346 | 17,93153367 | 1,232438001 | 0,000733975 |
| 251 | 2850576 | 68160936 | NM_005082.4 | TRIM25 | -23,69158863 | 0,821748384 | 0,000737703 |
| 252 | 6960328 | 75677605 | NM_001033515.1 | LOC389833 | 5,635808116 | 1,113339339 | 0,000738593 |
| 253 | 20224 | 47894448 | NM_206967.1 | MGC17624 | 5,464481452 | 1,099129587 | 0,000741223 |
| 254 | 4560463 | 4290896 | AI433683 | | -4,20815397 | 0,91832369 | 0,000764668 |
| 255 | 5490142 | 50363239 | NM_002685.2 | EXOSC10 | 64,67454838 | 1,261126978 | 0,000769548 |
| 256 | 240653 | 19603939 | BM981444 | | -2,329460443 | 0,946403267 | 0,000785871 |
| 257 | 2750070 | 18375517 | NM_001854.2 | COL11A1 | -3,554107591 | 0,934385923 | 0,000788658 |
| 258 | 6020575 | 14249551 | NM_032839.1 | DIRC2 | -35,91490208 | 0,820367822 | 0,000797516 |
| 259 | 3710189 | 38201688 | NM_002883.2 | RANGAP1 | 65,259255 | 1,257018161 | 0,000797921 |
| 260 | 2710278 | 52851445 | NM_005412.4 | SHMT2 | 65,41415964 | 1,2526449 | 0,000800169 |
| 261 | 360343 | 17105402 | NM_005103.3 | FEZ1 | 12,70533853 | 1,203271429 | 0,00082364 |
| 262 | 2690576 | 55953121 | NM_015705.4 | RUTBC3 | 14,20156258 | 1,166207066 | 0,00084996 |
| 263 | 2120681 | 13376061 | NM_024740.1 | ALG9 | 12,03252127 | 1,167495262 | 0,000852968 |
| 264 | 1410369 | 41350203 | NM_016286.2 | DCXR | 36,20150781 | 1,200362836 | 0,000857671 |
| 265 | 4850438 | 39777595 | NM_021809.4 | TGIF2 | 16,93614892 | 1,169575173 | 0,000858382 |
| 266 | 7100392 | 16753208 | NM_017481.2 | UBQLN3 | 4,445833834 | 1,083359837 | 0,000865917 |
| 267 | 1340731 | 21361349 | NM_006643.2 | SDCCAG3 | 29,22941312 | 1,187927522 | 0,00086772 |
| 268 | 2450603 | 32967290 | NM_181803.1 | UBE2C | -13,65767785 | 0,824486907 | 0,000875883 |
| 269 | 3390458 | 25777636 | NM_002243.3 | KCNJ15 | -68,77822725 | 0,761196715 | 0,000892396 |
| 270 | 1990673 | 52851448 | NM_173659.2 | RPUSD3 | 26,38750171 | 1,177225317 | 0,000894737 |
| 271 | 4150632 | 21618346 | NM_002756.2 | MAP2K3 | 3,058914147 | 1,065607186 | 0,000899878 |
| 272 | 130451 | 10835100 | NM_021078.1 | GCN5L2 | 45,23903059 | 1,24691539 | 0,000902329 |
| 273 | 10338 | 72534727 | NM_001031717.1 | CRELD1 | 22,42405663 | 1,23243055 | 0,000913686 |
| 274 | 1500040 | 30581134 | NM_006306.2 | SMC1L1 | 7,353409474 | 1,122782283 | 0,00092398 |
| 275 | 730725 | 40068037 | NM_016524.2 | SYT17 | 3,962856298 | 1,08382056 | 0,000932685 |
| 276 | 3800270 | 4757937 | NM_000648.1 | CCR2 | -2,41229231 | 0,939356527 | 0,000934322 |
| 277 | 5090204 | 7661547 | NM_015702.1 | C2orf25 | -79,93887985 | 0,842935199 | 0,000959059 |
| 278 | 3170468 | 7706726 | NM_016089.1 | ZNF589 | 28,88231896 | 1,256897786 | 0,000963048 |
| 279 | 50164 | 41281436 | NM_014669.2 | NUP93 | 28,51197413 | 1,174573985 | 0,000965886 |
| 280 | 4050411 | 34222200 | NM_153329.2 | ALDH16A1 | 53,51905541 | 1,260140642 | 0,000966212 |
| 281 | 380315 | 7705725 | NM_015959.1 | TXNDC14 | 36,56045278 | 1,18069957 | 0,000967375 |
| 282 | 6650541 | 70980545 | NM_176880.4 | TRA16 | 14,18996137 | 1,190005746 | 0,000987075 |
| 283 | 1440291 | 14589867 | NM_001716.2 | BLR1 | 4,157558649 | 1,091968062 | 0,00099509 |
| 284 | 4880373 | 27842881 | BX096603 | | 10,23388569 | 1,158997206 | 0,000998463 |
| 285 | 2120356 | 39888074 | CK299581 | | 2,430006552 | 1,065215654 | 0,001020522 |
| 286 | 160682 | 54112120 | NM_012426.3 | SF3B3 | 44,15684822 | 1,219213777 | 0,001029969 |
| 287 | 4280047 | 34577088 | NM_183382.1 | RNF13 | -75,68323448 | 0,750155587 | 0,001037306 |
| 288 | 1740576 | 41281526 | NM_014871.2 | USP52 | 19,95561799 | 1,213504932 | 0,001055174 |
| 289 | 2480082 | 35493922 | NM_006031.3 | PCNT2 | 29,27063181 | 1,159621239 | 0,001070467 |
| 290 | 6110328 | 23111065 | NM_003645.2 | SLC27A2 | -4,068829061 | 0,923612267 | 0,001075869 |
| 291 | 6660056 | 13743863 | BG217842 | | -33,02507288 | 0,769709246 | 0,001076096 |
| 292 | 1500717 | 73765543 | NM_000314.3 | PTEN | -65,04739132 | 0,804962827 | 0,001088331 |
| 293 | 7330487 | 52485986 | NM_002566.4 | P2RY11 | 28,34353619 | 1,266107577 | 0,00109239 |
| 294 | 2510324 | 7253951 | AW578902 | | -5,837784587 | 0,909758543 | 0,001097496 |
| 295 | 2760110 | 31542241 | NM_017917.2 | C14orf10 | -58,83249801 | 0,816539548 | 0,001102809 |
| 296 | 3140142 | 32698741 | NM_020925.1 | CACHD1 | -2,700091737 | 0,936782452 | 0,001113125 |
| 297 | 6400563 | 14150188 | NM_032369.1 | MGC15619 | 27,91382697 | 1,187624618 | 0,001114572 |
| 298 | 7000768 | 63003900 | NM_017773.2 | LAX1 | 36,42003819 | 1,258339525 | 0,001121704 |
| 299 | 2030450 | 6912355 | NM_012155.1 | EML2 | 42,34533266 | 1,168946841 | 0,001122558 |
| 300 | 3930687 | 71061448 | NM_024326.2 | FBXL15 | 41,8339122 | 1,225791909 | 0,00112491 |
| 301 | 770452 | 89029874 | XM_928878.1 | LOC392364 | 3,076035108 | 1,070389607 | 0,001136035 |
| 302 | 4390615 | 19924152 | NM_138557.1 | TLR4 | -47,03190471 | 0,76415391 | 0,001140352 |
| 303 | 2190524 | 46370053 | NM_019011.4 | TRIAD3 | 24,07686324 | 1,177162245 | 0,001145386 |
| 304 | 5360674 | 32455240 | NM_178232.2 | HAPLN3 | 17,16381664 | 1,244549462 | 0,001189577 |
| 305 | 4760364 | 62912469 | NM_001017403.1 | LGR6 | 15,47756491 | 1,23125561 | 0,001208655 |
| 306 | 4150768 | 7661817 | NM_014181.1 | HSPC159 | -19,17476534 | 0,78372152 | 0,001216429 |
| 307 | 2970332 | 50593004 | NM_006328.2 | RBM14 | 73,69517521 | 1,214022777 | 0,001227948 |
| 308 | 2370341 | 88982372 | XM_932015.1 | LOC643401 | 2,094536796 | 1,05887807 | 0,001232055 |
| 309 | 940754 | 7657332 | NM_014358.1 | CLEC4E | -29,11656126 | 0,74583825 | 0,00124297 |
| 310 | 1090048 | 48976062 | NM_153611.3 | CYBASC3 | 49,70244398 | 1,217479345 | 0,001247126 |
| 311 | 5910343 | 88953692 | XM_929793.1 | LOC646836 | 6,392732823 | 1,113777423 | 0,001249592 |
| 312 | 2810674 | 88970691 | XM_942926.1 | LOC647649 | 2,321532121 | 1,054742893 | 0,001252869 |
| 313 | 6350017 | 4505462 | NM_003632.1 | CNTNAP1 | 8,432099288 | 1,161986349 | 0,001272112 |
| 314 | 2000390 | 32130539 | NM_012395.2 | PFTK1 | -11,44388985 | 0,844274751 | 0,001272449 |
| 315 | 360402 | 12383073 | NM_022830.1 | RBM21 | 12,81737281 | 1,157133003 | 0,001289751 |
| 316 | 6270364 | 77157783 | NM_015044.3 | GGA2 | 54,90600546 | 1,283424211 | 0,001302584 |
| 317 | 7510224 | 89037451 | XM_929560.1 | ACOT1 | 5,220050681 | 1,097059274 | 0,001314489 |
| 318 | 3850025 | 21536366 | NM_139353.1 | TAF1C | 75,95063347 | 1,204746923 | 0,001320044 |
| 319 | 2640373 | 71040087 | NM_000449.3 | RFX5 | 40,58475673 | 1,195639991 | 0,001352801 |
| 320 | 1940543 | 62243651 | NM_001013848.1 | EXOC6 | -35,01537314 | 0,76458234 | 0,001357439 |
| 321 | 1820142 | 34147665 | NM_006374.3 | STK25 | 30,72099994 | 1,157095215 | 0,001358231 |
| 322 | 1470332 | 21450778 | NM_145036.1 | MGC33887 | 2,451633821 | 1,057340207 | 0,001380734 |
| 323 | 2070241 | 23397461 | NM_153211.1 | C18orf17 | 26,57907091 | 1,195520367 | 0,001380825 |
| 324 | 6200746 | 39725655 | NM_032376.2 | TMEM101 | 20,79524943 | 1,135857258 | 0,001392087 |
| 325 | 5130154 | 2115625 | AA431917 | | 2,687068685 | 1,060681089 | 0,001397186 |
| 326 | 7650605 | 13489098 | NM_024681.1 | KCTD17 | 42,15691645 | 1,219877312 | 0,001398325 |
| 327 | 990224 | 40538798 | NM_020131.3 | UBQLN4 | 44,15987403 | 1,241656798 | 0,001409781 |
| 328 | 6110142 | 21389554 | NM_144666.1 | DNHD1 | 10,18272952 | 1,176487041 | 0,001414669 |
| 329 | 520154 | 34527067 | AK130294 | | 21,74202891 | 1,237530114 | 0,001416293 |
| 330 | 2650164 | 57164941 | NM_001008938.1 | CKAP5 | 29,33938351 | 1,191276253 | 0,001417373 |
| 331 | 6370463 | 89047258 | XM_925913.1 | LOC653063 | 2,808209897 | 1,065349534 | 0,00141842 |
| 332 | 6020500 | 27477133 | NM_024923.2 | NUP210 | 37,64775789 | 1,196401091 | 0,001418525 |
| 333 | 7380328 | 11141876 | NM_021805.1 | SIGIRR | 25,06501581 | 1,245654007 | 0,001427724 |
| 334 | 4200451 | 88983572 | XM_068632.5 | LOC133993 | 3,341967043 | 1,065344844 | 0,001430887 |
| 335 | 1430156 | 13129099 | NM_024096.1 | XTP3TPA | 21,55693285 | 1,173886359 | 0,001430912 |
| 336 | 2490747 | 4501840 | NM_001605.1 | AARS | 77,76894401 | 1,276356344 | 0,001432837 |
| 337 | 1010612 | 31542677 | NM_031206.2 | LAS1L | 12,21303662 | 1,172490221 | 0,001437935 |
| 338 | 2630181 | 38570053 | NM_020408.3 | C6orf149 | 8,470600619 | 1,14906581 | 0,001455672 |
| 339 | 1450082 | 29789121 | NM_021117.1 | CRY2 | 17,52300597 | 1,161214169 | 0,001456531 |
| 340 | 3940020 | 38149169 | CF887308 | | 2,194774646 | 1,057872899 | 0,001465205 |
| 341 | 2970440 | 51592099 | NM_014675.2 | CROCC | 5,396550969 | 1,094770922 | 0,001466061 |
| 342 | 3990192 | 6912279 | NM_012111.1 | AHSA1 | 28,18197892 | 1,15069417 | 0,001481716 |
| 343 | 3830278 | 12751496 | NM_023080.1 | C8orf33 | 11,18572632 | 1,129068947 | 0,001502437 |
| 344 | 2070131 | 22035596 | NM_031420.2 | MRPL9 | 31,85307452 | 1,167431606 | 0,001511617 |
| 345 | 6040259 | 31652258 | NM_032853.2 | MUM1 | 21,48103217 | 1,229453813 | 0,001518048 |
| 346 | 650014 | 37237779 | CF619198 | | 2,327816537 | 1,063259079 | 0,0015214 |
| 347 | 1690504 | 27831422 | BX101820 | | 2,143929406 | 1,056737612 | 0,00152161 |
| 348 | 5080056 | 39841072 | NM_018079.3 | FLJ10379 | -24,26466131 | 0,832390434 | 0,001525093 |
| 349 | 7100504 | 39652617 | NM_004623.2 | TTC4 | 36,08459854 | 1,184629696 | 0,001561337 |
| 350 | 5890273 | 21071040 | NM_014141.3 | CNTNAP2 | 6,595271148 | 1,146748332 | 0,001599007 |
| 351 | 7610546 | 40255053 | NM_032717.3 | HMFN0839 | -54,94911949 | 0,781061678 | 0,001603755 |
| 352 | 6020132 | 41281777 | NM_152943.1 | ZNF268 | 2,648966818 | 1,059765454 | 0,001607376 |
| 353 | 6110133 | 15042936 | NM_007365.1 | PADI2 | -9,370076681 | 0,86480149 | 0,001614081 |
| 354 | 2000474 | 17965839 | BM272556 | | -2,55867557 | 0,936510151 | 0,001622817 |
| 355 | 2030747 | 34222148 | NM_032138.3 | KBTBD7 | -29,27404586 | 0,760659869 | 0,001628042 |
| 356 | 5560093 | 34531894 | AK125708 | | 3,295857766 | 1,075280337 | 0,001644271 |
| 357 | 610465 | 41393562 | NM_015074.2 | KIF1B | -69,35956203 | 0,733288248 | 0,001645719 |
| 358 | 3400372 | 53692181 | NM_015002.2 | FBXO21 | 26,3299661 | 1,15897828 | 0,001663991 |
| 359 | 6580075 | 20149575 | NM_006164.2 | NFE2L2 | -62,000525 | 0,78335815 | 0,001666828 |
| 360 | 5130747 | 23238724 | BU587445 | | 5,206540958 | 1,107947177 | 0,001673048 |
| 361 | 6650576 | 24787676 | CA424950 | | 2,962661443 | 1,070519384 | 0,001676488 |
| 362 | 4120086 | 13569871 | NM_030915.1 | LBH | 4,189390797 | 1,079490285 | 0,001706774 |
| 363 | 3130019 | 20143980 | NM_014234.3 | HSD17B8 | 21,75170555 | 1,196488957 | 0,001711828 |
| 364 | 4200148 | 42660194 | XM_378421 | | 14,16364107 | 1,189819518 | 0,001713891 |
| 365 | 940706 | 88989505 | XM_939144.1 | LOC650058 | 2,002405673 | 1,048229223 | 0,001739768 |
| 366 | 20553 | 42789728 | NM_006152.2 | LRMP | -74,82036091 | 0,849300145 | 0,001740334 |
| 367 | 5550270 | 27838297 | BX113537 | | 3,941936997 | 1,086914048 | 0,00175354 |
| 368 | 4570612 | 51477715 | NM_006122.2 | MAN2A2 | -44,39209005 | 0,795587215 | 0,001768914 |
| 369 | 3390008 | 24475884 | NM_003475.2 | RASSF7 | 57,42044838 | 1,233262979 | 0,001787024 |
| 370 | 4730088 | 50484029 | CR603222 | | 3,836354648 | 1,077928085 | 0,001799439 |
| 371 | 670376 | 38158012 | NM_182830.2 | MAMDC1 | 2,798906913 | 1,068655473 | 0,0018053 |
| 372 | 7400431 | 24234746 | NM_004515.2 | ILF2 | 63,52810868 | 1,160207487 | 0,001806039 |
| 373 | 520360 | 40254941 | NM_020448.2 | NPAL3 | 20,03362452 | 1,165178541 | 0,00181287 |
| 374 | 1240440 | 48762680 | NM_000701.6 | ATP1A1 | 37,53285116 | 1,204524584 | 0,001815625 |
| 375 | 6380358 | 73912719 | NM_001032363.1 | C1orf151 | 3,003480865 | 1,065127964 | 0,001856986 |
| 376 | 7400240 | 50541941 | NM_000488.2 | SERPINC1 | 2,88345468 | 1,067256547 | 0,001886392 |
| 377 | 6110672 | 68303646 | NM_001025077.1 | CUGBP2 | -49,70921613 | 0,826255573 | 0,001902574 |
| 378 | 7050196 | 88988812 | XM_943393.1 | LOC647784 | 2,371716902 | 1,058914165 | 0,001918129 |
| 379 | 1070377 | 66346718 | NM_207106.2 | UIP1 | 32,34029956 | 1,192004187 | 0,0019244 |
| 380 | 50347 | 57862814 | NR_002196.1 | H19 | 4,098623993 | 1,09014366 | 0,001939512 |
| 381 | 6510452 | 7794142 | AW779539 | | 2,742193271 | 1,058591264 | 0,001982133 |
| 382 | 5130142 | 50301239 | NM_001001998.1 | EXOSC10 | 23,91241934 | 1,189832508 | 0,001984951 |
| 383 | 6770142 | 37693992 | NM_002513.2 | NME3 | 51,08647128 | 1,205555171 | 0,001991096 |
| 384 | 3400176 | 39725947 | NM_015517.3 | MIZF | 13,64333077 | 1,107326504 | 0,001995042 |
| 385 | 610653 | 4826650 | NM_004928.1 | C21orf2 | 33,52242941 | 1,236381199 | 0,001998985 |
| 386 | 3940386 | 39995110 | NM_000849.3 | GSTM3 | 8,079852703 | 1,155645076 | 0,002004006 |
| 387 | 2100519 | 54144638 | NM_006469.4 | IVNS1ABP | -63,08035217 | 0,81392201 | 0,002018303 |
| 388 | 5130553 | 19001887 | BM688622 | | 2,603201451 | 1,065274697 | 0,002038439 |
| 389 | 5690008 | 4885584 | NM_005500.1 | SAE1 | 75,82328648 | 1,157051883 | 0,002042091 |
| 390 | 7160164 | 40354200 | NM_199184.1 | C6orf108 | 26,45996909 | 1,188835551 | 0,002055282 |
| 391 | 4610619 | 34147351 | NM_024046.2 | CAMKV | -3,847147069 | 0,927934773 | 0,002074291 |
| 392 | 4010673 | 38149909 | NM_198138.1 | SEC31L2 | 10,15672126 | 1,132559406 | 0,00207951 |
| 393 | 3780220 | 8922497 | NM_018129.1 | PNPO | 47,19395989 | 1,242699483 | 0,002083951 |
| 394 | 3440040 | 88989998 | XM_944927.1 | LOC649242 | -2,349265137 | 0,942740447 | 0,002090613 |
| 395 | 6040639 | 4757733 | NM_004833.1 | AIM2 | -39,37522134 | 0,756948245 | 0,002136117 |
| 396 | 430100 | 34222094 | NM_012212.2 | LTB4DH | -2,891199796 | 0,93528499 | 0,002137898 |
| 397 | 5270450 | 31004095 | CD243631 | | -2,682928323 | 0,934932365 | 0,002141697 |
| 398 | 4900497 | 18979657 | BM669760 | | 2,394474482 | 1,055793869 | 0,002144886 |
| 399 | 6250338 | 22748798 | NM_152371.1 | C1orf93 | 20,43951257 | 1,20374194 | 0,002146632 |
| 400 | 5720424 | 54607107 | NM_031449.3 | DKFZp761I2123 | 28,39880817 | 1,24615102 | 0,002146928 |
| 401 | 5570632 | 51587022 | CR738137 | | -3,700734709 | 0,919525529 | 0,002155079 |
| 402 | 2650152 | 29789109 | NM_020698.1 | TMCC3 | -29,36456043 | 0,795720773 | 0,002157529 |
| 403 | 5390100 | 42544135 | NM_015713.3 | RRM2B | -28,5915367 | 0,768583225 | 0,002165583 |
| 404 | 6380050 | 74229007 | NM_001032999.1 | CBFA2T2 | 3,841545355 | 1,0857201 | 0,002166999 |
| 405 | 4260053 | 19747275 | NM_133325.1 | PHF10 | 49,7681192 | 1,179062329 | 0,002169686 |
| 406 | 4810327 | 2046104 | AA393134 | | -2,484180664 | 0,938614611 | 0,002175145 |
| 407 | 5720497 | 25777601 | NM_002808.3 | PSMD2 | 31,15011938 | 1,146090486 | 0,002181127 |
| 408 | 630091 | 21536482 | NM_003518.3 | HIST1H2BG | -13,60500067 | 0,819384796 | 0,00218131 |
| 409 | 2850274 | 88952955 | XM_926366.1 | LOC653181 | -1,935228719 | 0,953381316 | 0,002182192 |
| 410 | 1710100 | 54860104 | NM_018054.4 | ARHGAP17 | 53,494328 | 1,203378443 | 0,002183767 |
| 411 | 6650482 | 27811314 | CB066794 | | 2,266652104 | 1,055934349 | 0,002184843 |
| 412 | 6040196 | 34335276 | NM_015568.2 | PPP1R16B | 28,57922477 | 1,214435517 | 0,002197064 |
| 413 | 2510132 | 34147385 | NM_024844.2 | NUP85 | 25,75983033 | 1,159776012 | 0,002199934 |
| 414 | 3460242 | 20127505 | NM_006556.2 | PMVK | 16,95917741 | 1,145346624 | 0,002201696 |
| 415 | 5870300 | 19592429 | BM974838 | | 3,55266692 | 1,080305117 | 0,00220268 |
| 416 | 1010754 | 34101267 | NM_020803.3 | KLHL8 | -26,74101975 | 0,788060862 | 0,002203432 |
| 417 | 4540088 | 16877451 | BC016972 | | -3,538684311 | 0,925430866 | 0,002221092 |
| 418 | 630768 | 13399321 | NM_024662.1 | NAT10 | 18,28438533 | 1,168615183 | 0,00222234 |
| 419 | 3840131 | 13376746 | NM_025152.1 | NUBPL | 6,311302866 | 1,116147303 | 0,002242144 |
| 420 | 5050390 | 58331183 | NM_006429.2 | CCT7 | 36,9437154 | 1,173816637 | 0,002248928 |
| 421 | 730543 | 54144651 | NM_001517.4 | GTF2H4 | 14,70939523 | 1,167778973 | 0,002249712 |
| 422 | 840139 | 24792039 | CA429313 | | 2,270203986 | 1,054352894 | 0,002252613 |
| 423 | 7380241 | 39812261 | NM_003530.3 | HIST1H3D | -21,86668228 | 0,793726621 | 0,00226776 |
| 424 | 3940338 | 12669910 | NM_005225.1 | E2F1 | -5,162386453 | 0,908474544 | 0,002305931 |
| 425 | 7650458 | 62422567 | NM_001313.3 | CRMP1 | 5,572953708 | 1,098993806 | 0,00231087 |
| 426 | 6110132 | 13376252 | NM_024839.1 | RPP21 | 59,80821658 | 1,179349782 | 0,002310908 |
| 427 | 3130291 | 27886542 | NM_004555.2 | NFATC3 | 15,6758675 | 1,14504507 | 0,002320651 |
| 428 | 7380347 | 55743082 | NM_001006940.1 | ALG3 | 23,9546724 | 1,169231331 | 0,002321091 |
| 429 | 6550762 | 27734876 | NM_173555.1 | TYSND1 | 36,94434887 | 1,185786991 | 0,002333003 |
| 430 | 1030041 | 40788014 | NM_021209.3 | CARD12 | -24,56951249 | 0,818818786 | 0,002358744 |
| 431 | 1820341 | 21396488 | NM_004793.2 | PRSS15 | 22,47364221 | 1,206078489 | 0,002396097 |
| 432 | 2370593 | 66348044 | NM_016478.3 | ZC3HC1 | 19,91283504 | 1,166411255 | 0,002399701 |
| 433 | 6650403 | 7706336 | NM_016057.1 | COPZ1 | 40,88815665 | 1,163358795 | 0,002410936 |
| 434 | 3710228 | 62739164 | NM_182943.2 | PLOD2 | -6,41581762 | 0,875464193 | 0,002417035 |
| 435 | 1090139 | 24475981 | NM_016464.2 | HSPC196 | 22,43444477 | 1,189752629 | 0,002427259 |
| 436 | 2070494 | 24234749 | NM_012218.2 | ILF3 | 66,65536913 | 1,264598066 | 0,00246216 |
| 437 | 6480458 | 71773200 | NM_001030018.1 | APRT | 49,22444358 | 1,266012441 | 0,002496455 |
| 438 | 830754 | 11277467 | NM_006427.2 | SIVA | 15,28911626 | 1,123182292 | 0,002511787 |
| 439 | 5090554 | 38016938 | NM_002150.2 | HPD | -6,000610516 | 0,892380431 | 0,002530391 |
| 440 | 5090047 | 28416952 | NM_032501.2 | ACSS1 | 35,01148376 | 1,184237465 | 0,002539779 |
| 441 | 4880681 | 21361381 | NM_007171.2 | POMT1 | 12,61365289 | 1,174440001 | 0,002541681 |
| 442 | 7200477 | 6640026 | AW263210 | | -1,678426858 | 0,960397391 | 0,002564708 |
| 443 | 2750035 | 65508349 | NM_001018062.1 | LOC51149 | 2,484711498 | 1,062668028 | 0,002577857 |
| 444 | 3290019 | 54607143 | NM_152735.3 | ZBTB9 | 14,11460038 | 1,157880814 | 0,002589178 |
| 445 | 3850020 | 10437827 | AK025332 | | -26,25812165 | 0,759114727 | 0,002632909 |
| 446 | 5670274 | 22035641 | NM_030644.1 | APOL3 | 28,59518167 | 1,194613178 | 0,002633129 |
| 447 | 2760064 | 24234721 | NM_006260.2 | DNAJC3 | -6,571723401 | 0,884214748 | 0,002633237 |
| 448 | 5570661 | 34147500 | NM_020315.3 | PDXP | 25,74017676 | 1,220220548 | 0,002654539 |
| 449 | 5090315 | 51094102 | NM_019082.2 | DDX56 | 52,28321626 | 1,235529494 | 0,002654824 |
| 450 | 3140088 | 45387930 | NM_205548.1 | UNQ9217 | -3,394067971 | 0,932686171 | 0,002657711 |
| 451 | 4010048 | 34304338 | NM_004035.4 | ACOX1 | -43,72066716 | 0,802689627 | 0,002662627 |
| 452 | 7380736 | 40317621 | NM_199176.1 | MRRF | 4,945702746 | 1,084721323 | 0,002666503 |
| 453 | 1170139 | 52421789 | NM_033141.2 | MAP3K9 | 3,726192295 | 1,066369155 | 0,002667438 |
| 454 | 5260184 | 32217345 | CD693563 | | 10,93731216 | 1,194775359 | 0,002672296 |
| 455 | 7320594 | 55956901 | NM_006724.2 | MAP3K4 | 21,59370431 | 1,158641495 | 0,002720574 |
| 456 | 4900398 | 89060532 | XM_946033.1 | LOC652809 | -3,418618822 | 0,935923831 | 0,002738753 |
| 457 | 2850288 | 31077212 | NM_001530.2 | HIF1A | -17,7068821 | 0,797710279 | 0,002742525 |
| 458 | 6660471 | 25306269 | NM_016050.2 | MRPL11 | 28,56600561 | 1,177667177 | 0,002745508 |
| 459 | 1710189 | 5453751 | NM_006424.1 | SLC34A2 | 2,229363981 | 1,050166934 | 0,002749561 |
| 460 | 6620674 | 34147574 | NM_006410.3 | HTATIP2 | -17,29978654 | 0,88693604 | 0,002756336 |
| 461 | 5890494 | 47834345 | NM_012257.3 | HBP1 | -50,56848683 | 0,83510744 | 0,002765307 |
| 462 | 7160767 | 89042800 | XM_945544.1 | UBE2Z | 55,13945078 | 1,130117008 | 0,002775733 |
| 463 | 1510224 | 53729323 | NM_017838.3 | NOLA2 | 35,91106831 | 1,179080572 | 0,002802852 |
| 464 | 380541 | 27436919 | NM_003971.3 | SPAG9 | -16,78481129 | 0,871575447 | 0,00282301 |
| 465 | 1450523 | 89035472 | XM_930820.1 | LRRK2 | -68,96090215 | 0,719755782 | 0,002825347 |
| 466 | 6520241 | 23510351 | NM_020679.2 | MIF4GD | 27,40320804 | 1,159950156 | 0,002830456 |
| 467 | 60468 | 17017983 | NM_001261.2 | CDK9 | 24,99861391 | 1,168629649 | 0,00283774 |
| 468 | 6110228 | 50355989 | NM_173855.3 | MORN3 | 3,709222234 | 1,08270705 | 0,002842034 |
| 469 | 580050 | 31543835 | NM_016616.2 | TXNDC3 | -27,04827067 | 0,793501561 | 0,00284596 |
| 470 | 3780243 | 55953128 | NM_001550.2 | IFRD1 | -50,9467186 | 0,757230602 | 0,002863928 |
| 471 | 5090647 | 2816926 | AA765688 | | -2,251315518 | 0,946398658 | 0,002880931 |
| 472 | 5340162 | 56550038 | NM_005933.2 | MLL | 23,28905135 | 1,270793653 | 0,00290127 |
| 473 | 7560603 | 21071035 | NM_020926.2 | BCOR | 3,835536594 | 1,073378361 | 0,002913866 |
| 474 | 5820465 | 21361852 | NM_020405.3 | PLXDC1 | 10,07727623 | 1,144744368 | 0,002918091 |
| 475 | 7000224 | 20336295 | NM_018380.2 | DDX28 | 12,70400328 | 1,117735353 | 0,002939555 |
| 476 | 3420682 | 9257200 | NM_015722.2 | DRD1IP | 3,175697509 | 1,074847011 | 0,002940764 |
| 477 | 1470541 | 24532964 | CA314866 | | -2,368897154 | 0,945452428 | 0,002942185 |
| 478 | 940450 | 61966858 | NM_001013706.1 | LOC440503 | -6,987092813 | 0,869107661 | 0,002951728 |
| 479 | 6270215 | 89041715 | XM_373742.4 | MGC40489 | 3,326299967 | 1,07180415 | 0,002959387 |
| 480 | 5050653 | 24432071 | NM_145032.2 | FBXL13 | -18,83916692 | 0,810627797 | 0,002971531 |
| 481 | 1070189 | 5729975 | NM_006608.1 | PHTF1 | -24,33989005 | 0,794747722 | 0,002986487 |
| 482 | 650767 | 30795205 | NM_006243.2 | PPP2R5A | -67,1556536 | 0,847031344 | 0,002994704 |
| 483 | 6280168 | 21450799 | NM_145043.1 | NEIL2 | 19,88048242 | 1,19544672 | 0,002995077 |
| 484 | 2640278 | 22027508 | NM_017955.2 | CDCA4 | 6,698447811 | 1,111031409 | 0,002997021 |

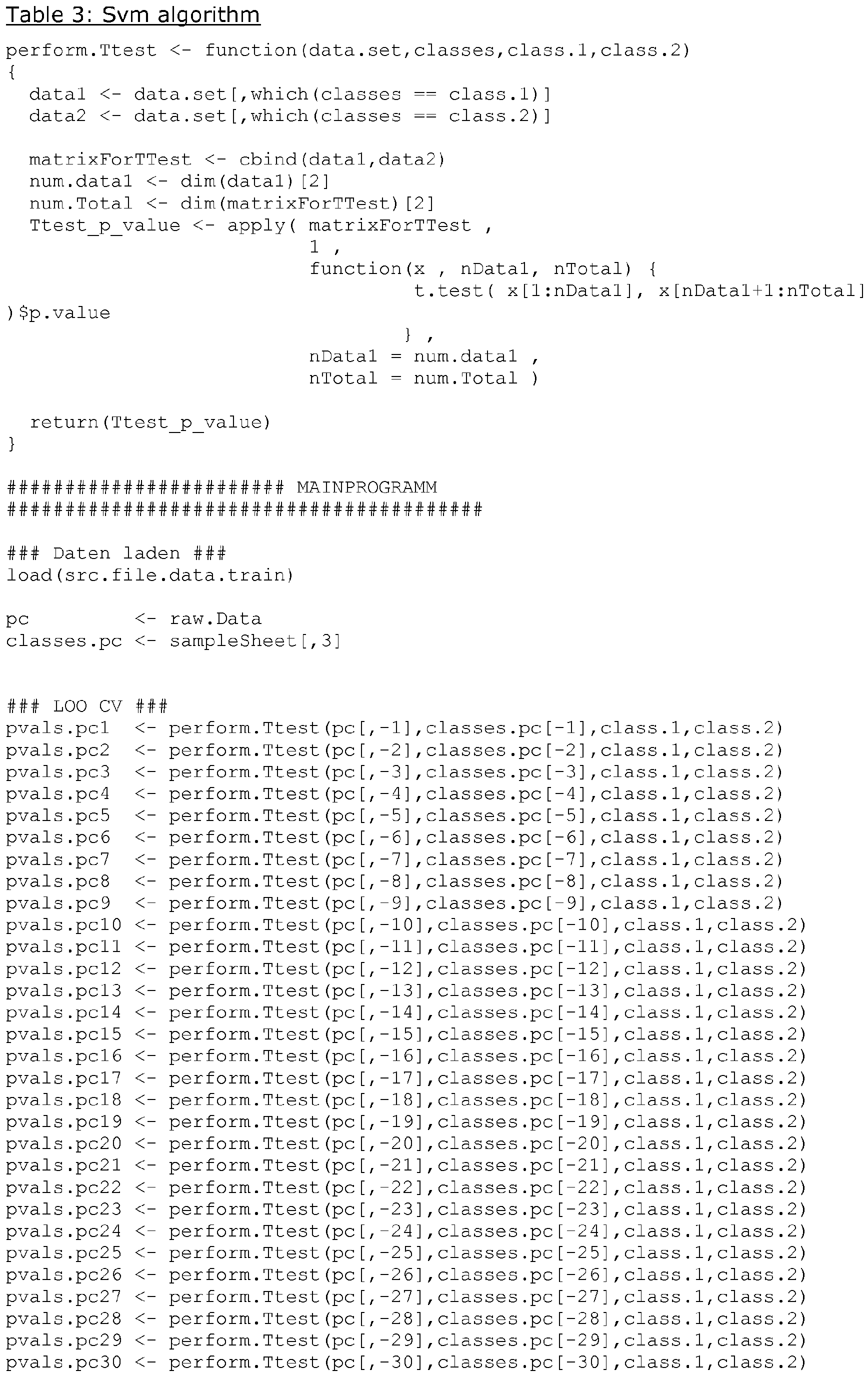

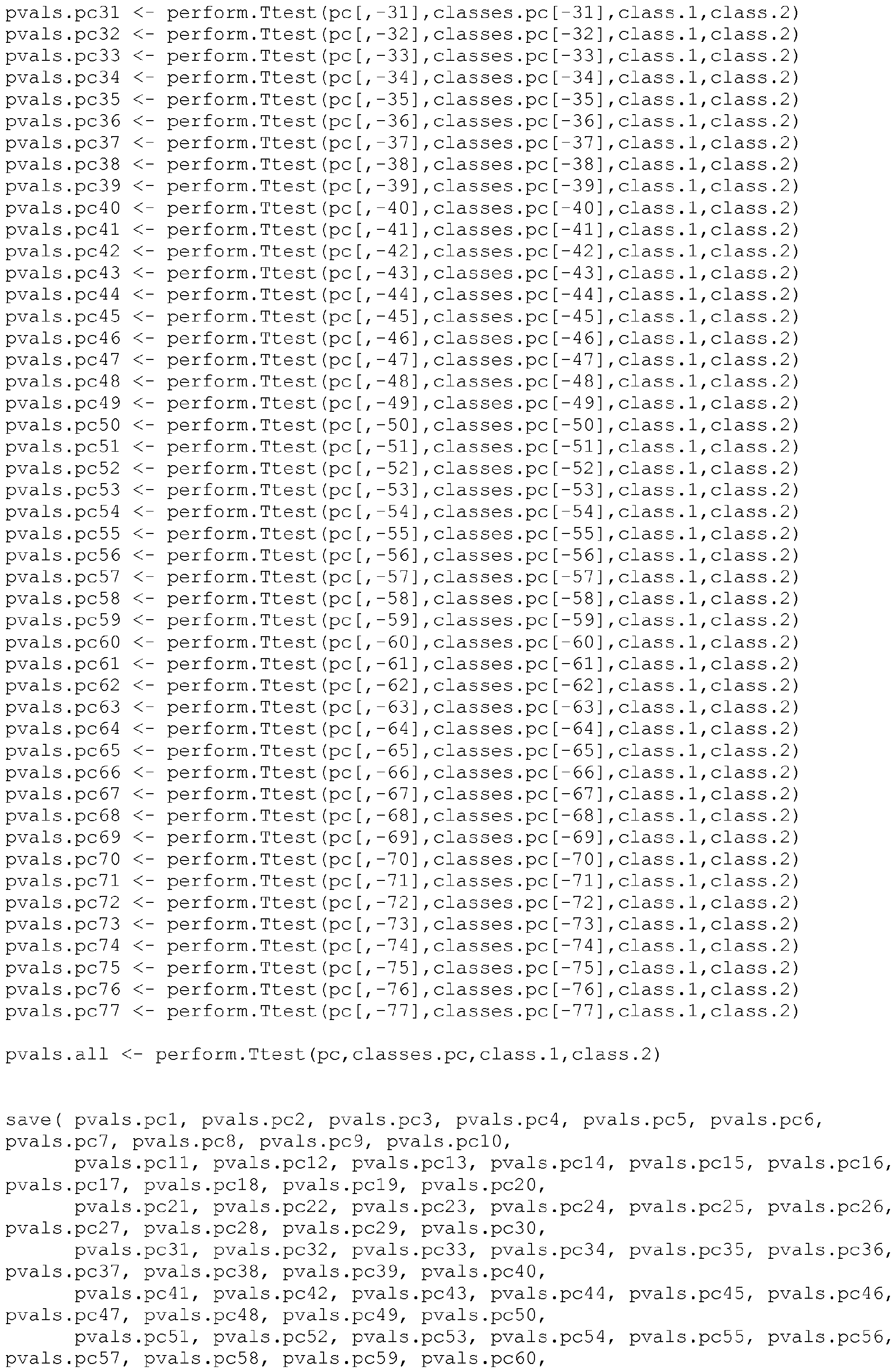

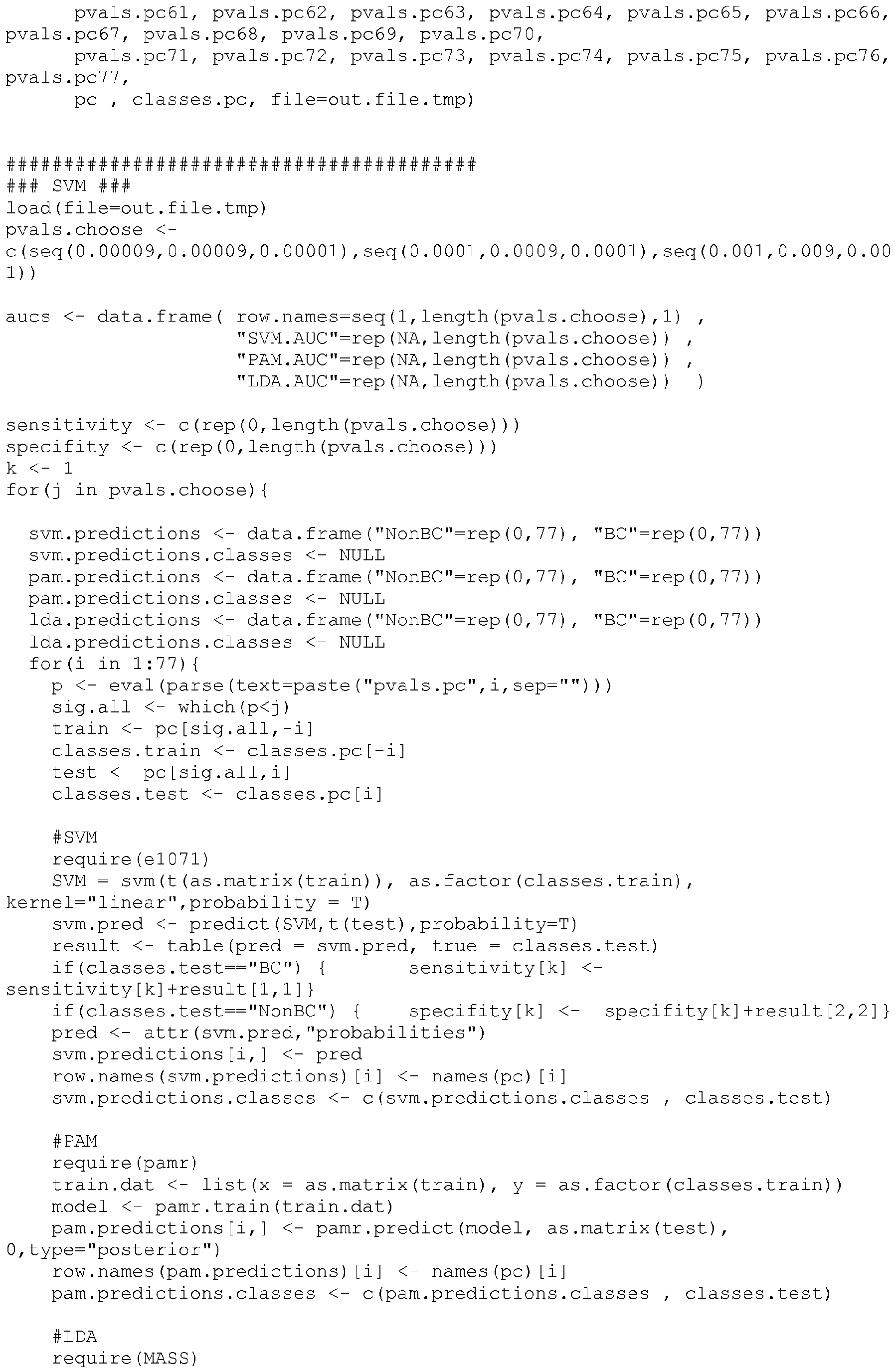

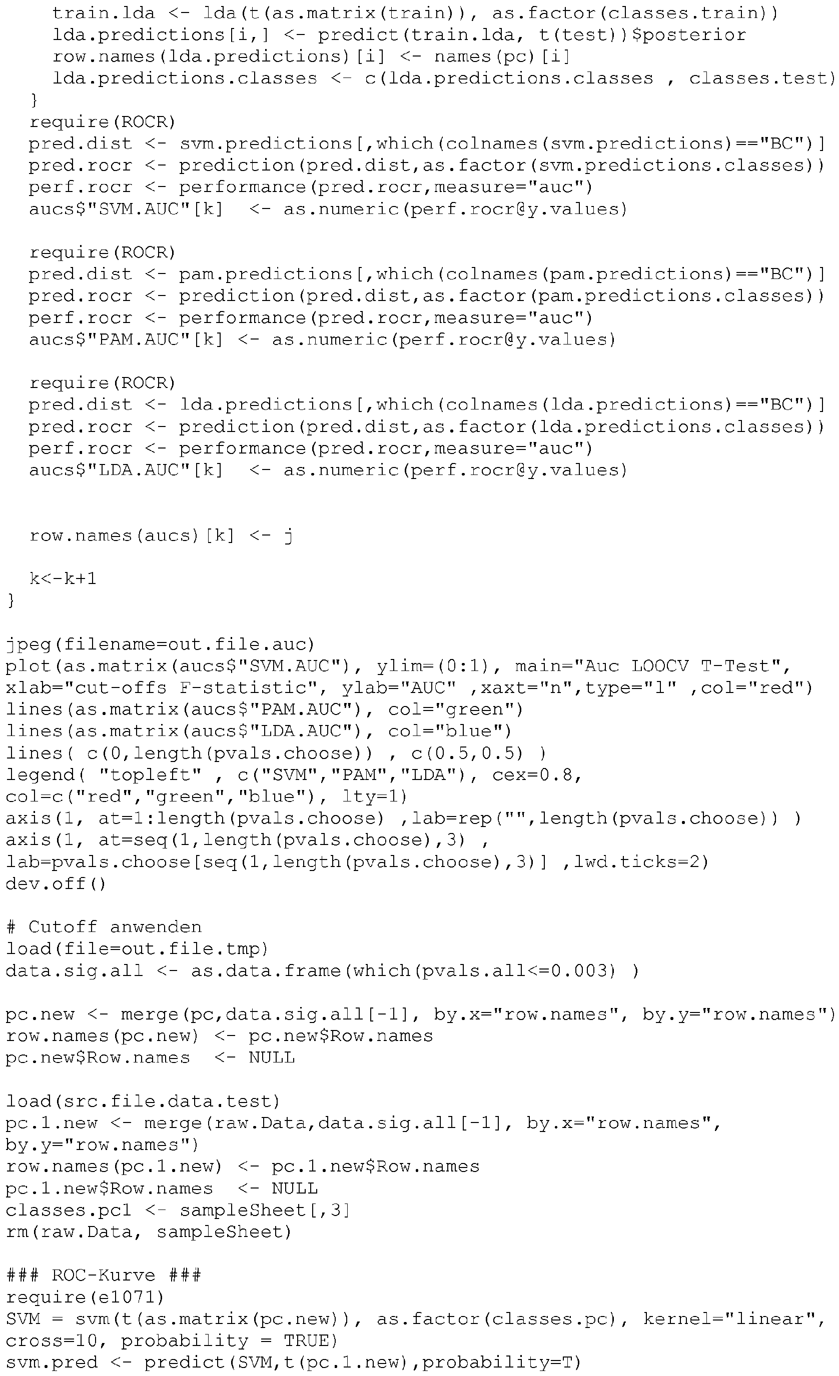

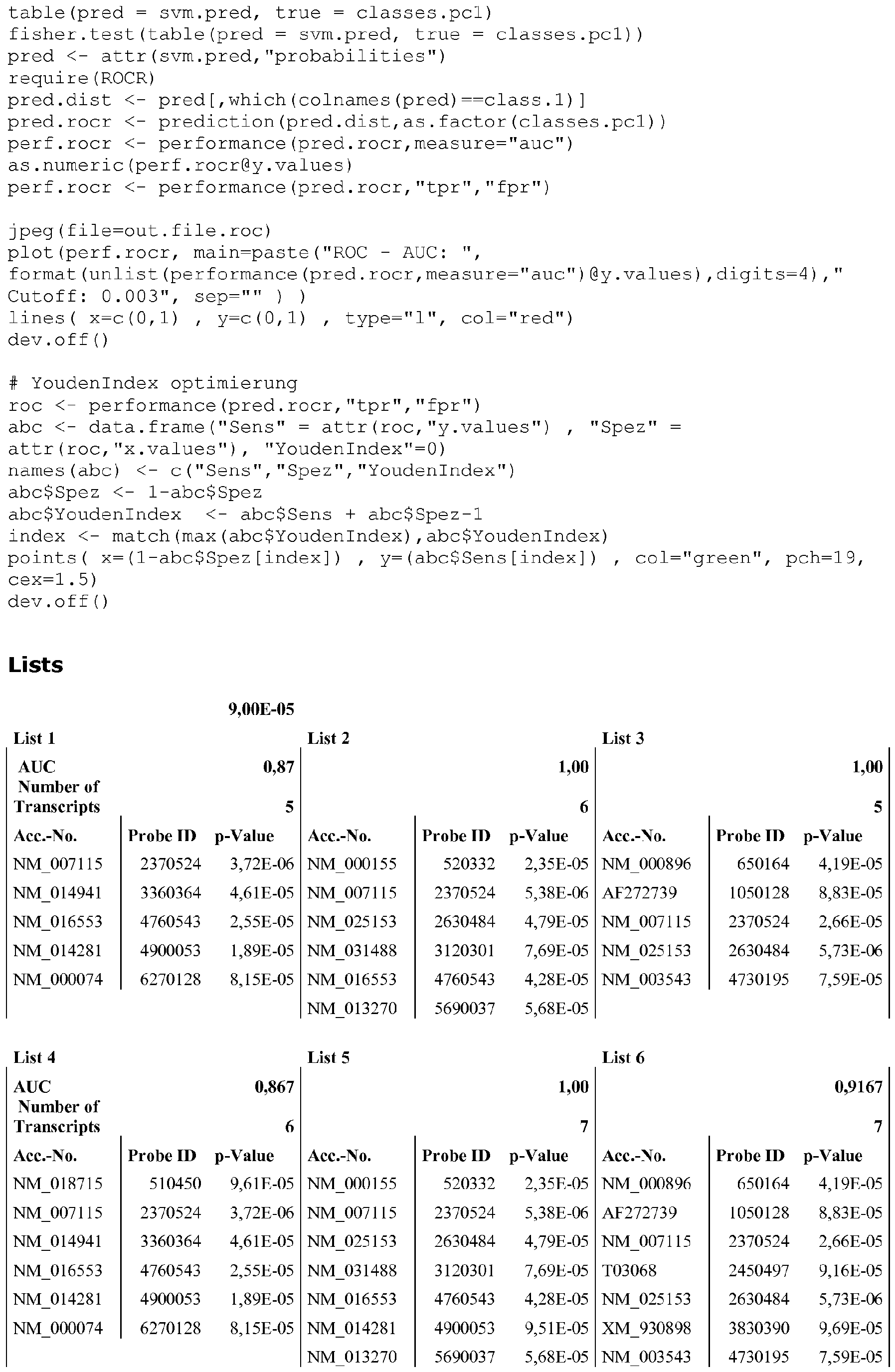

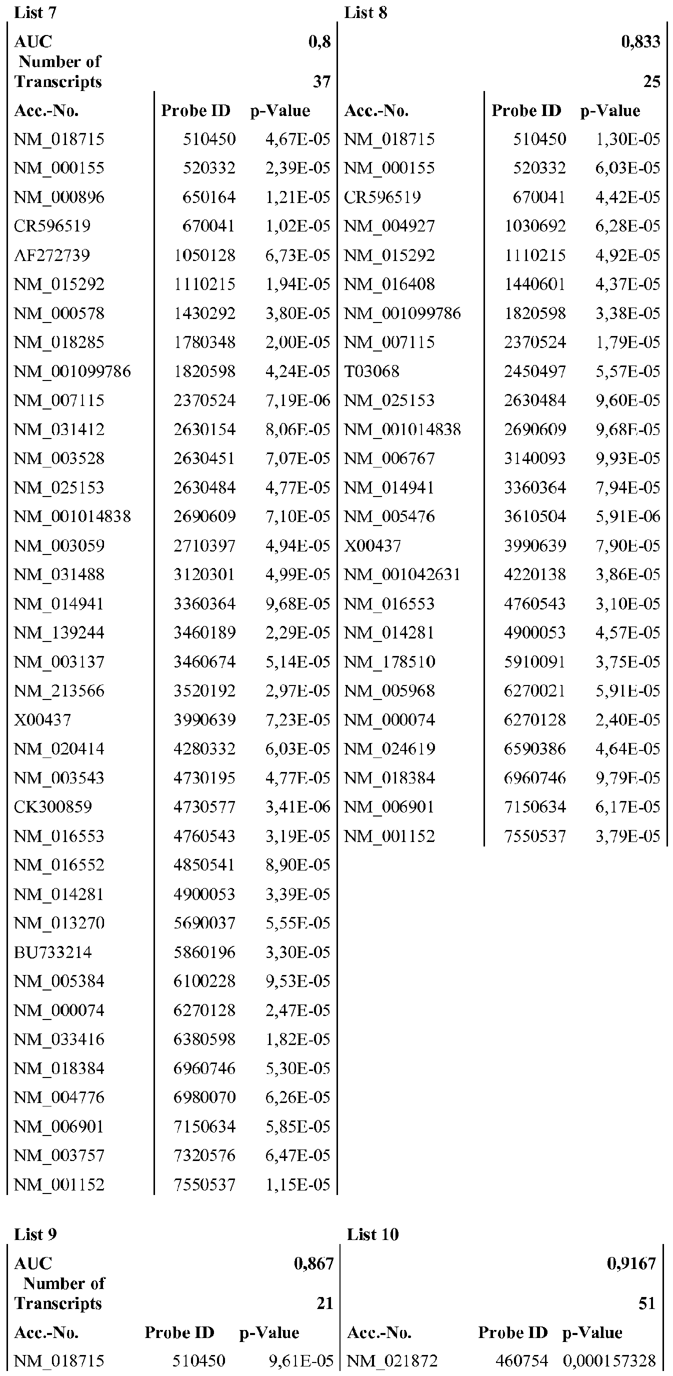

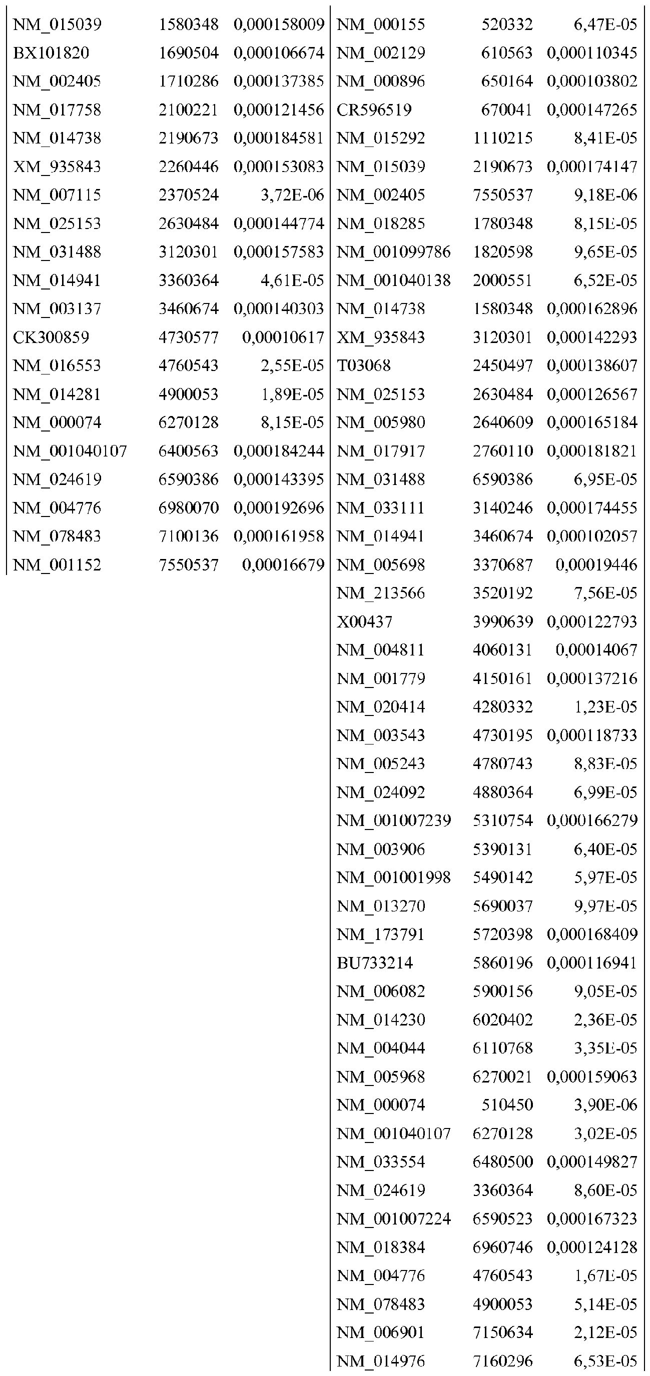

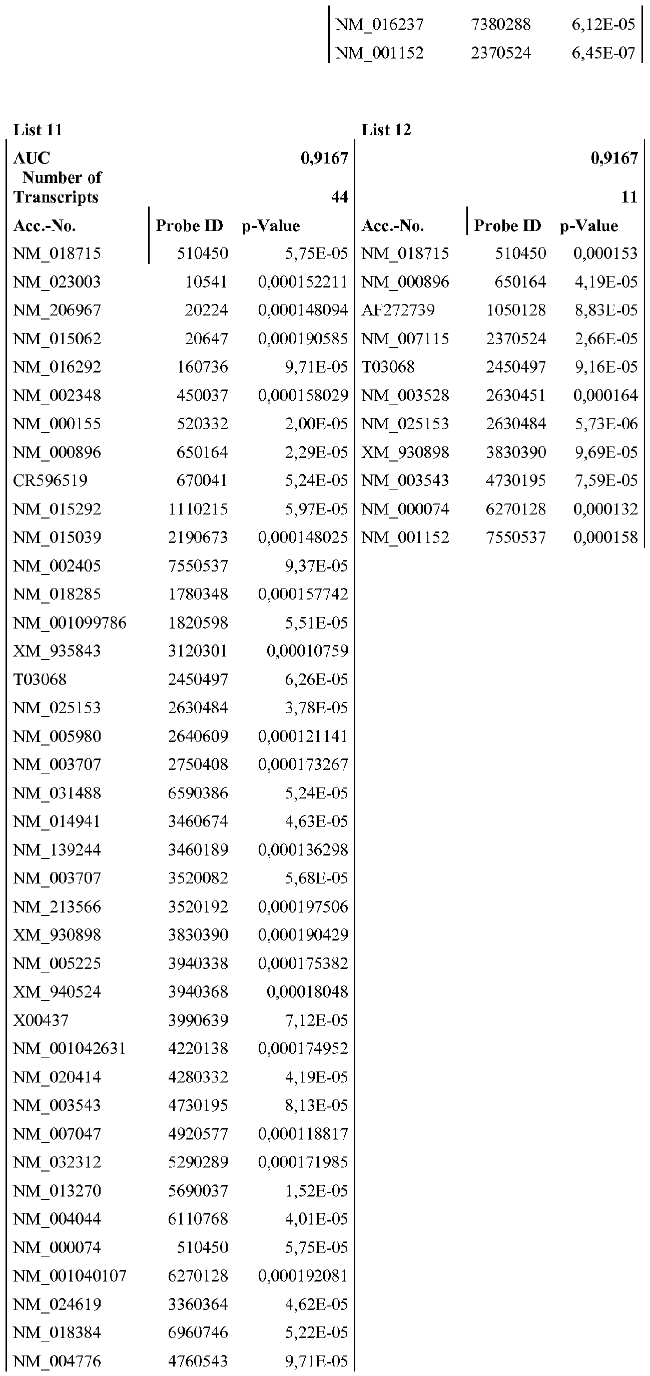

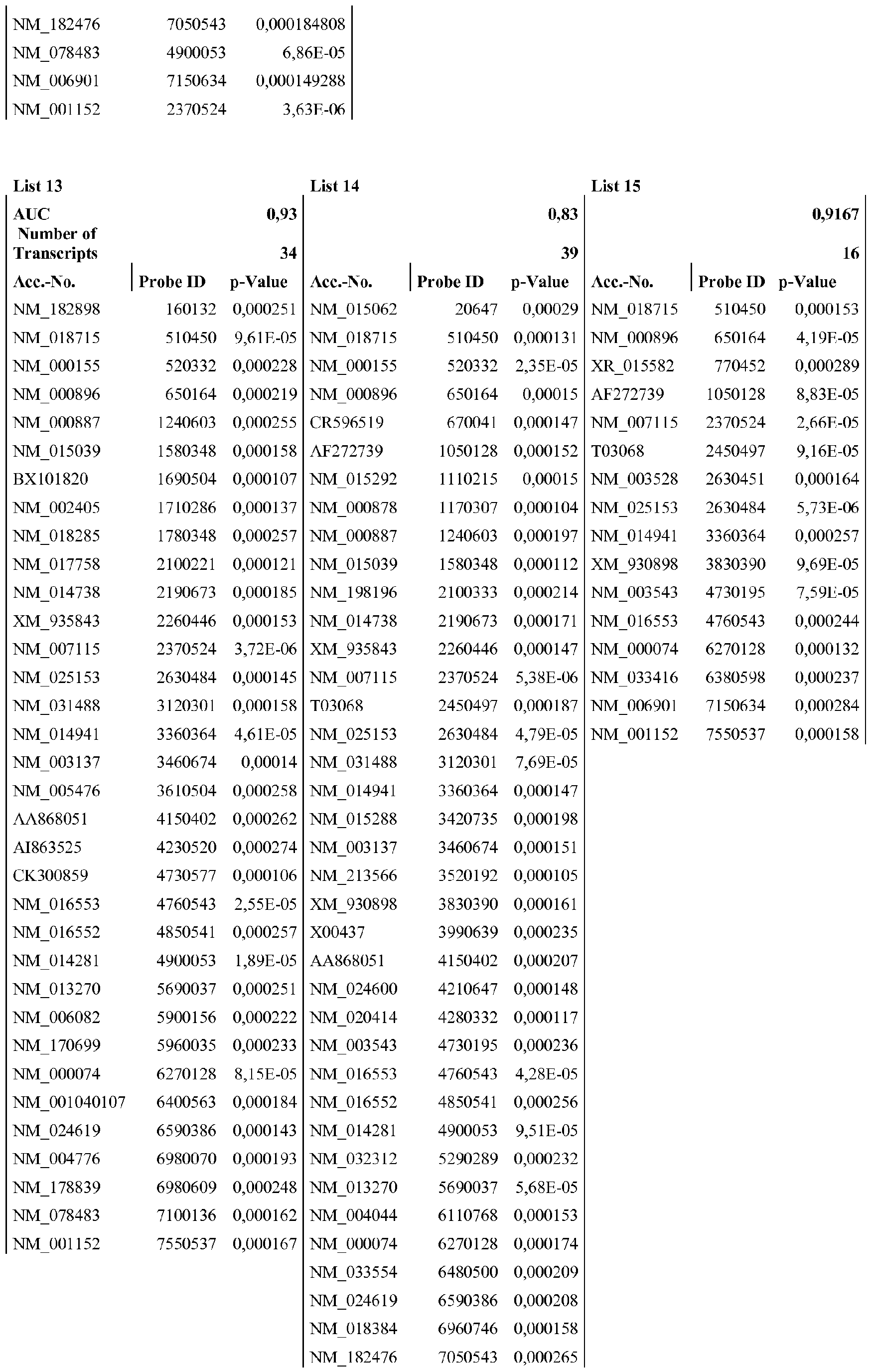

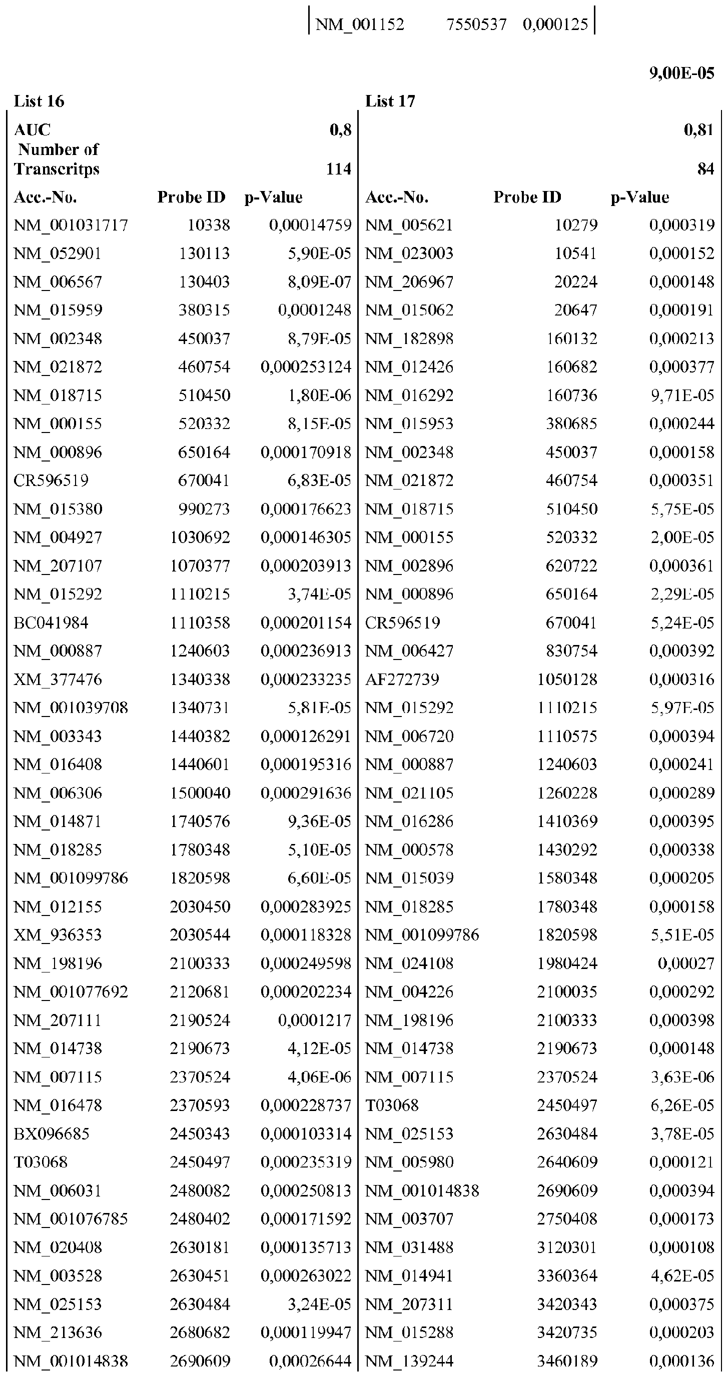

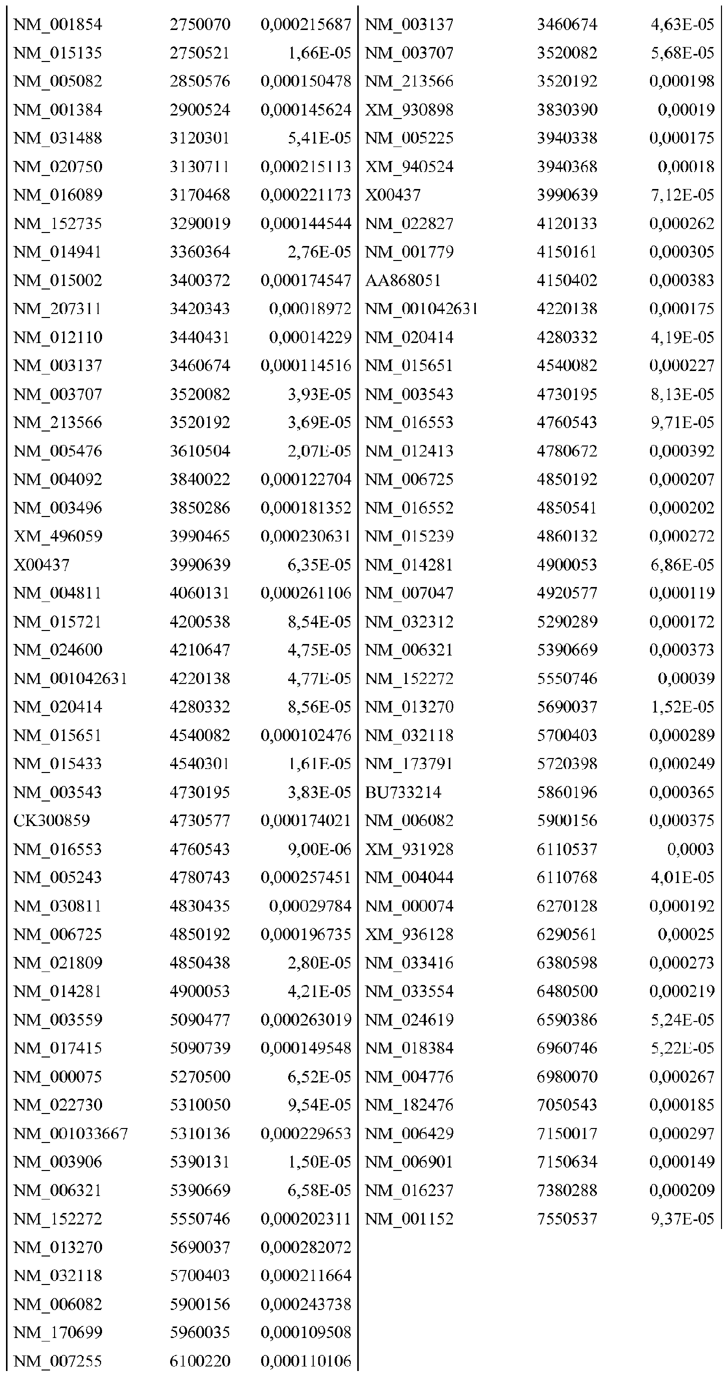

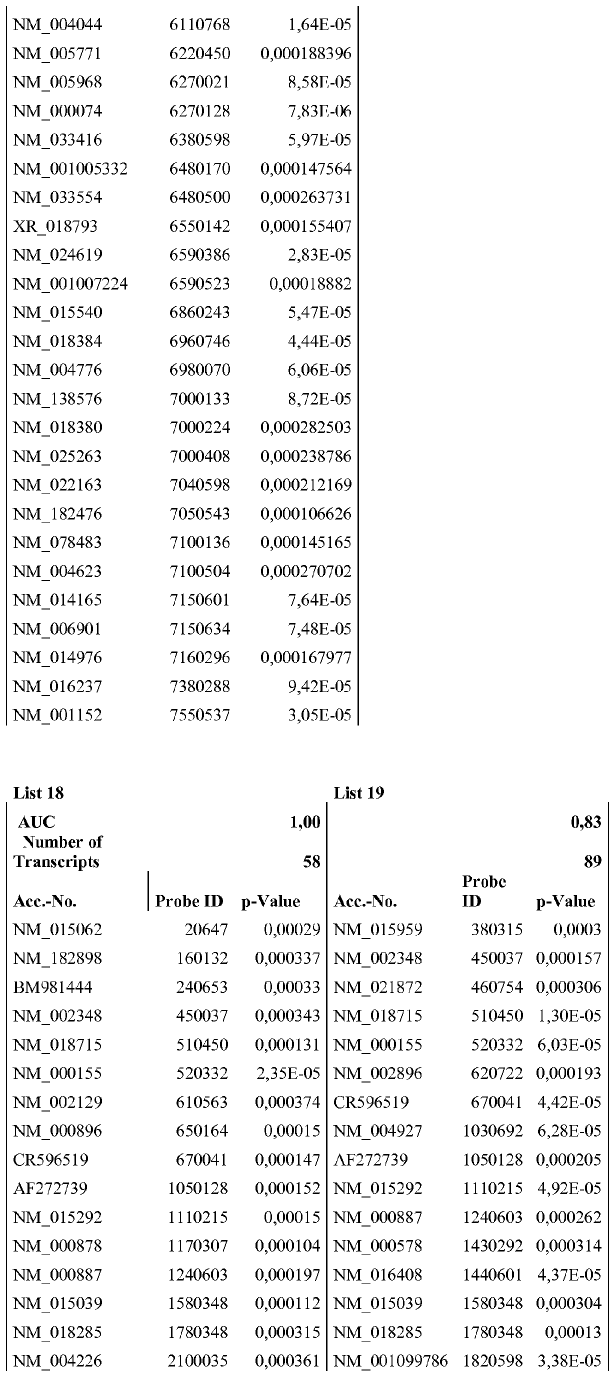

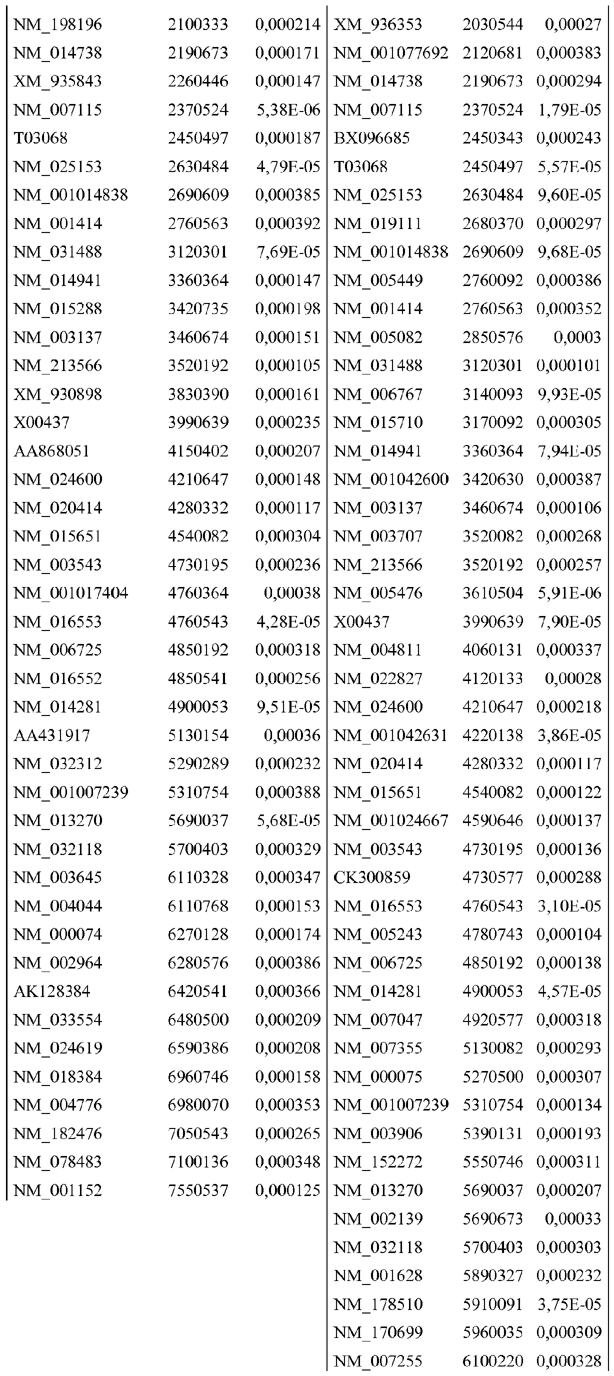

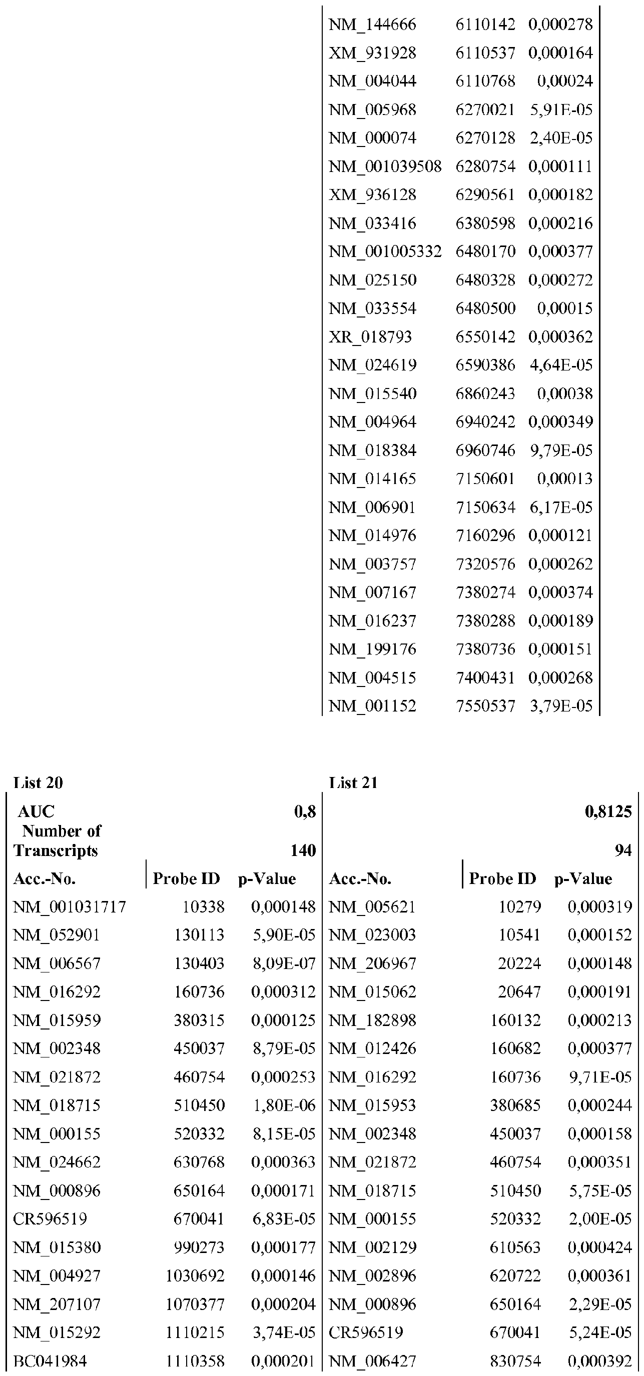

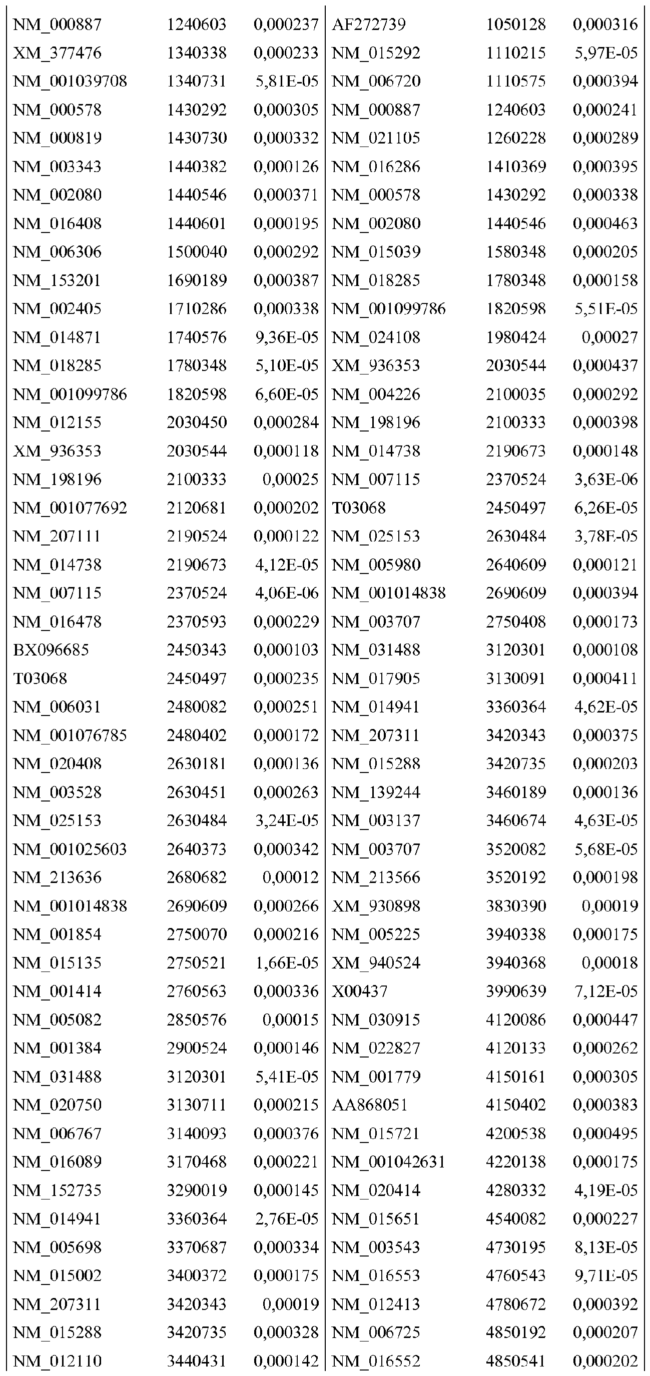

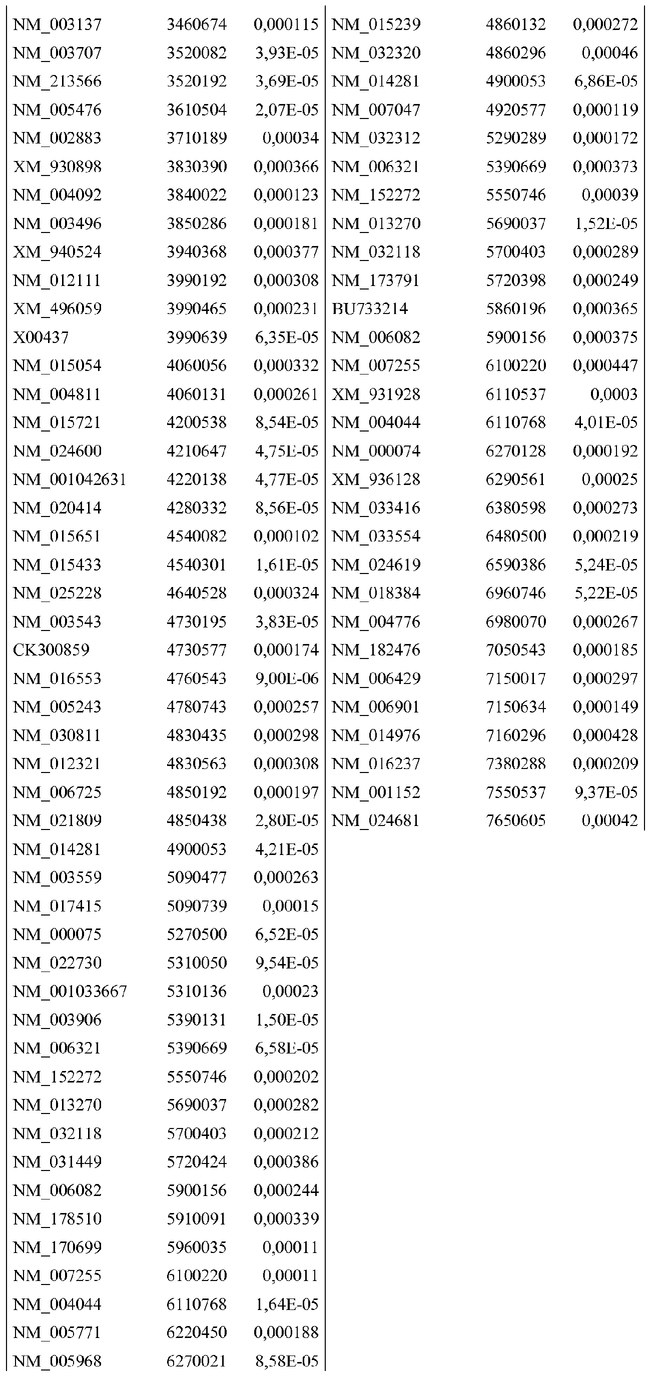

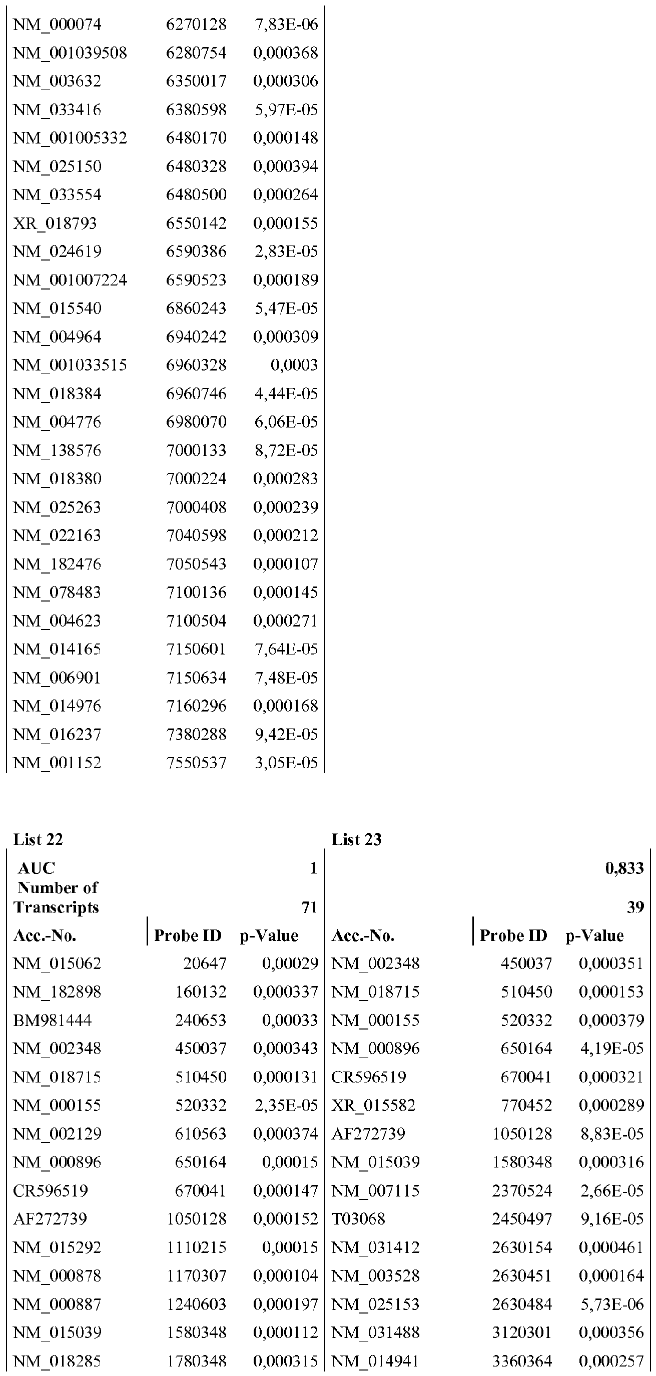

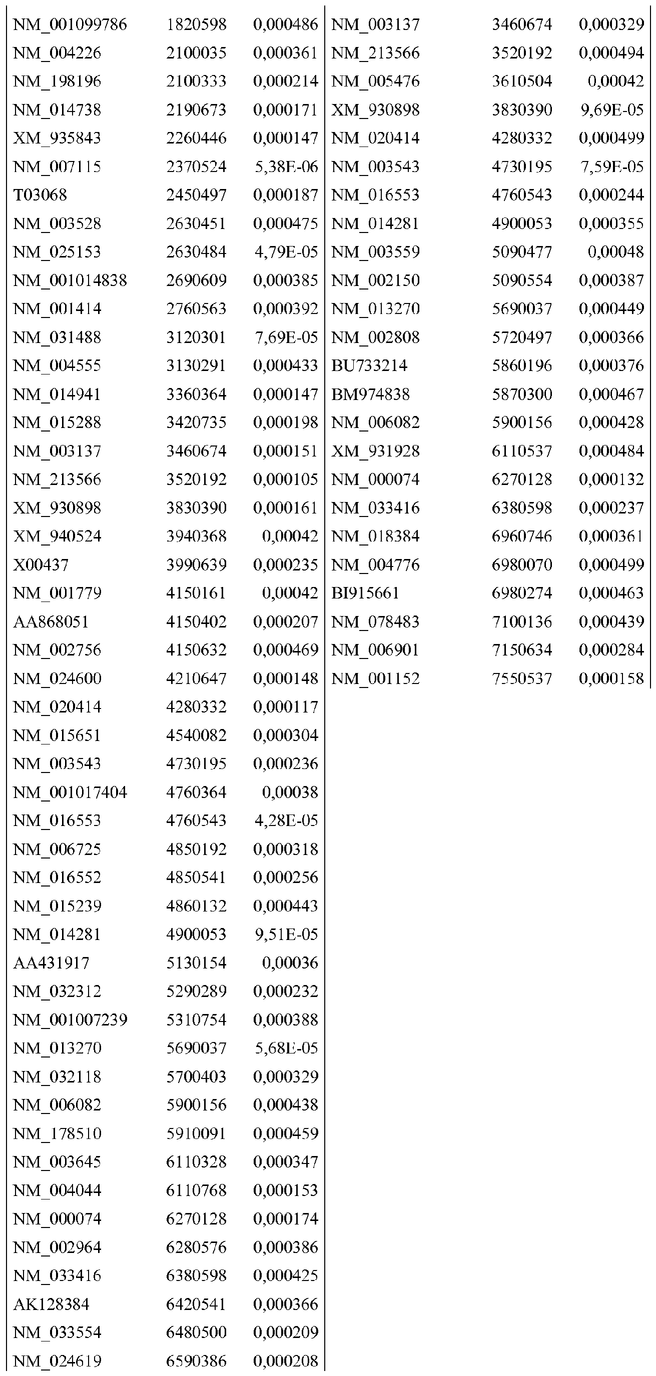

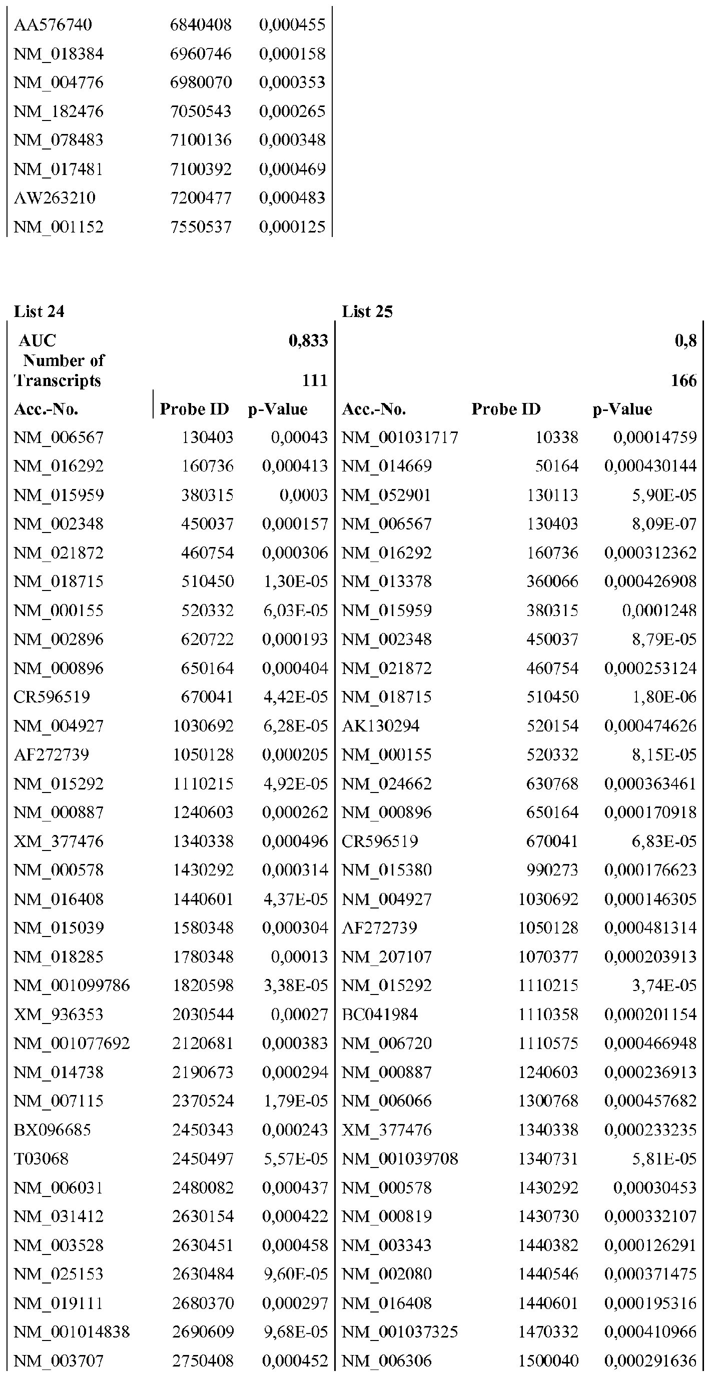

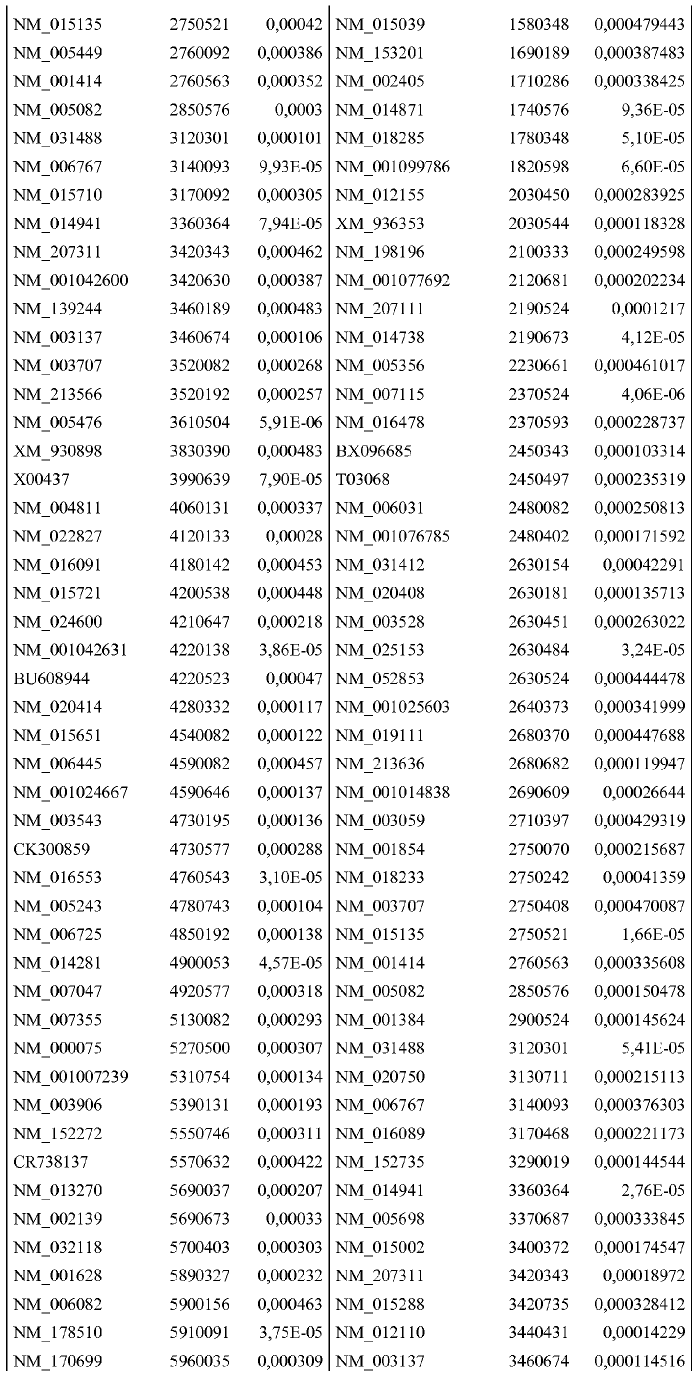

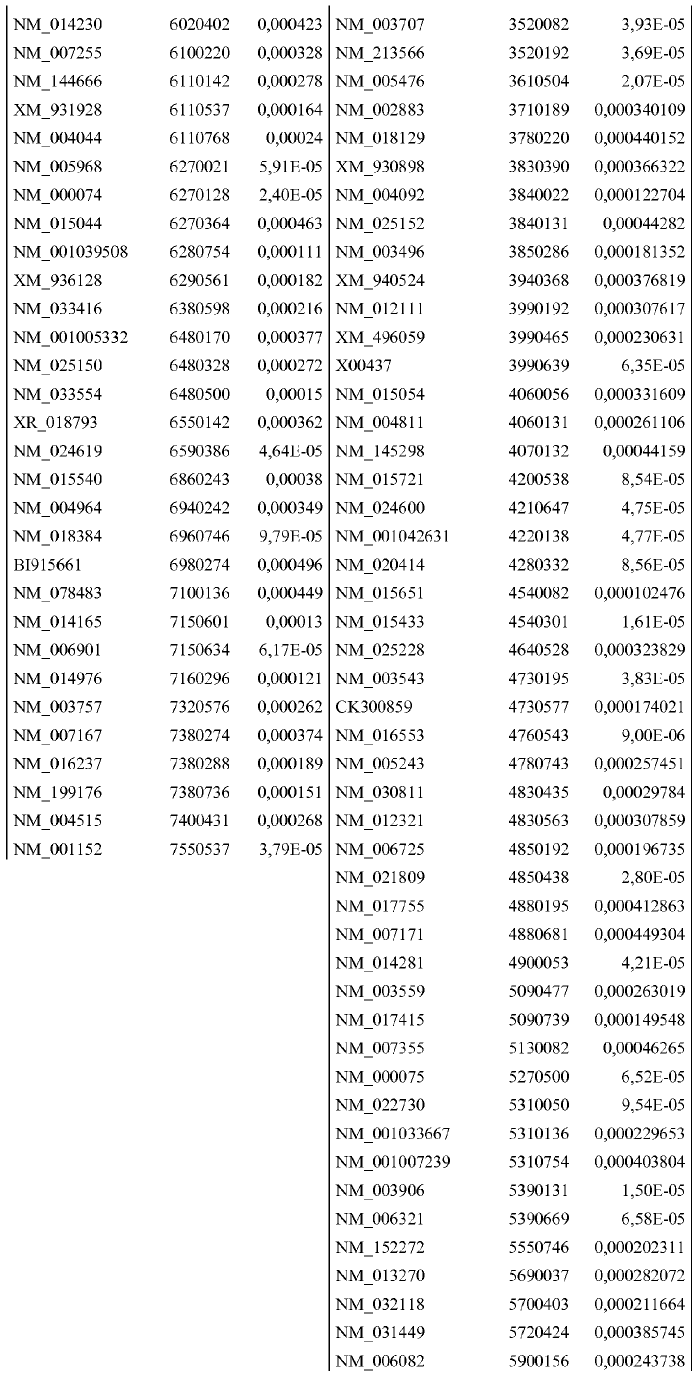

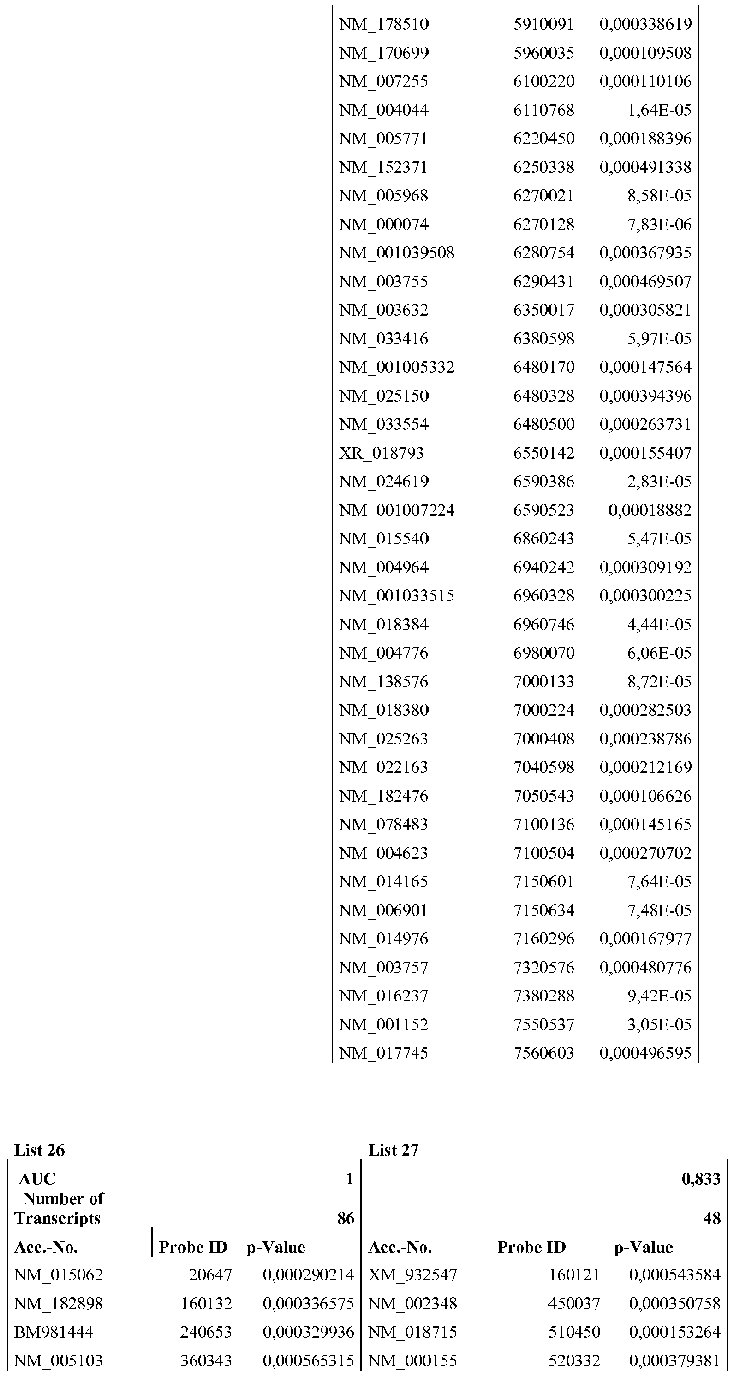

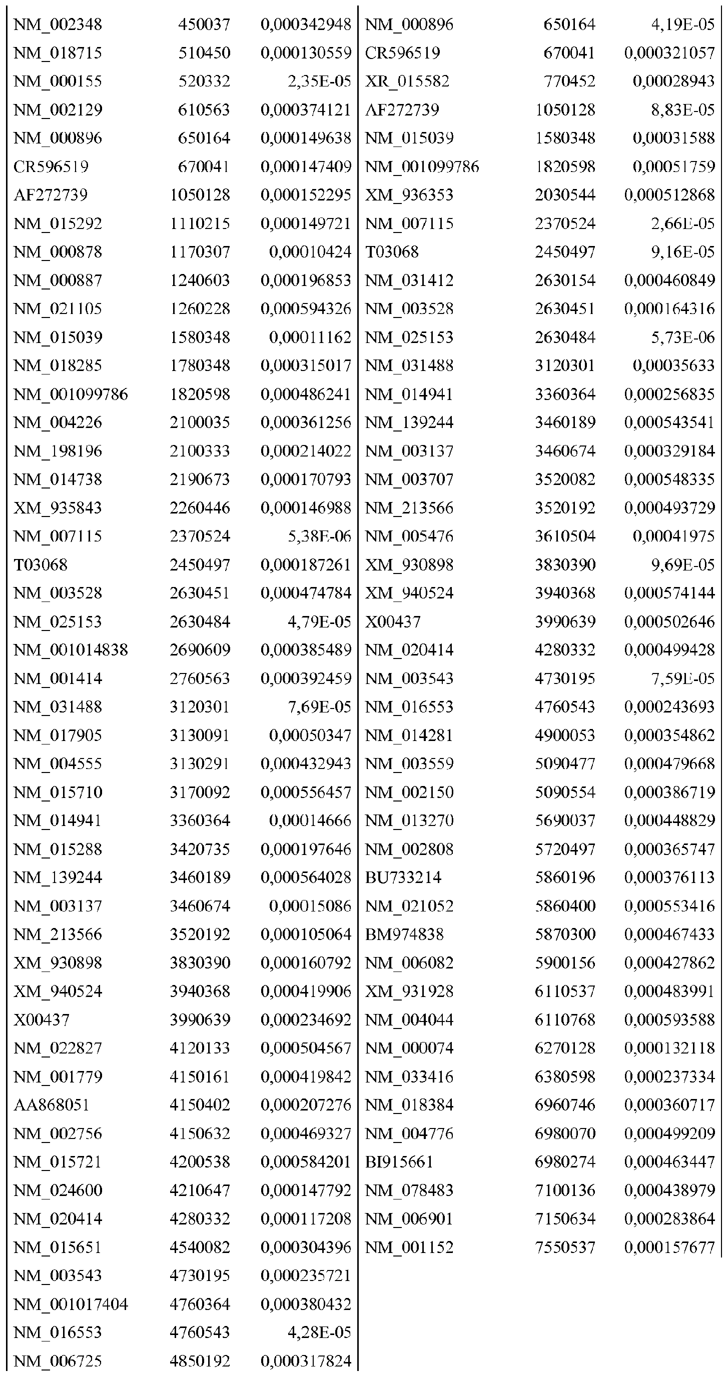

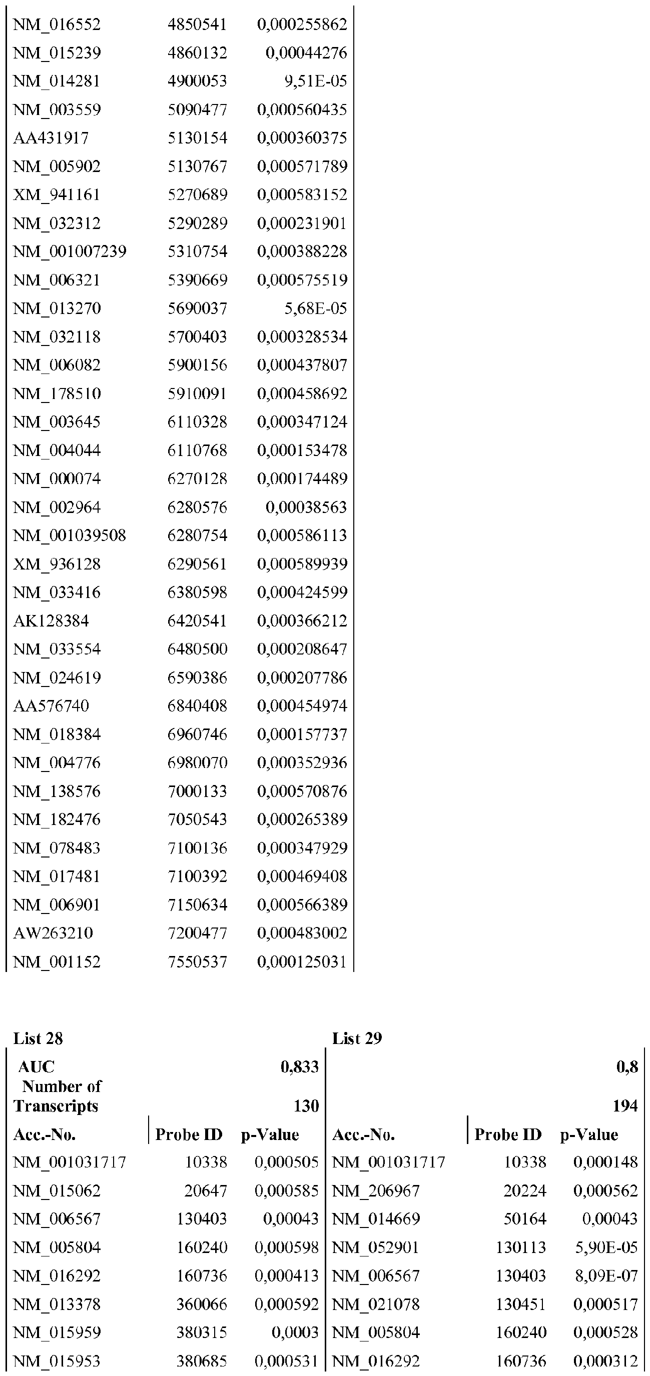

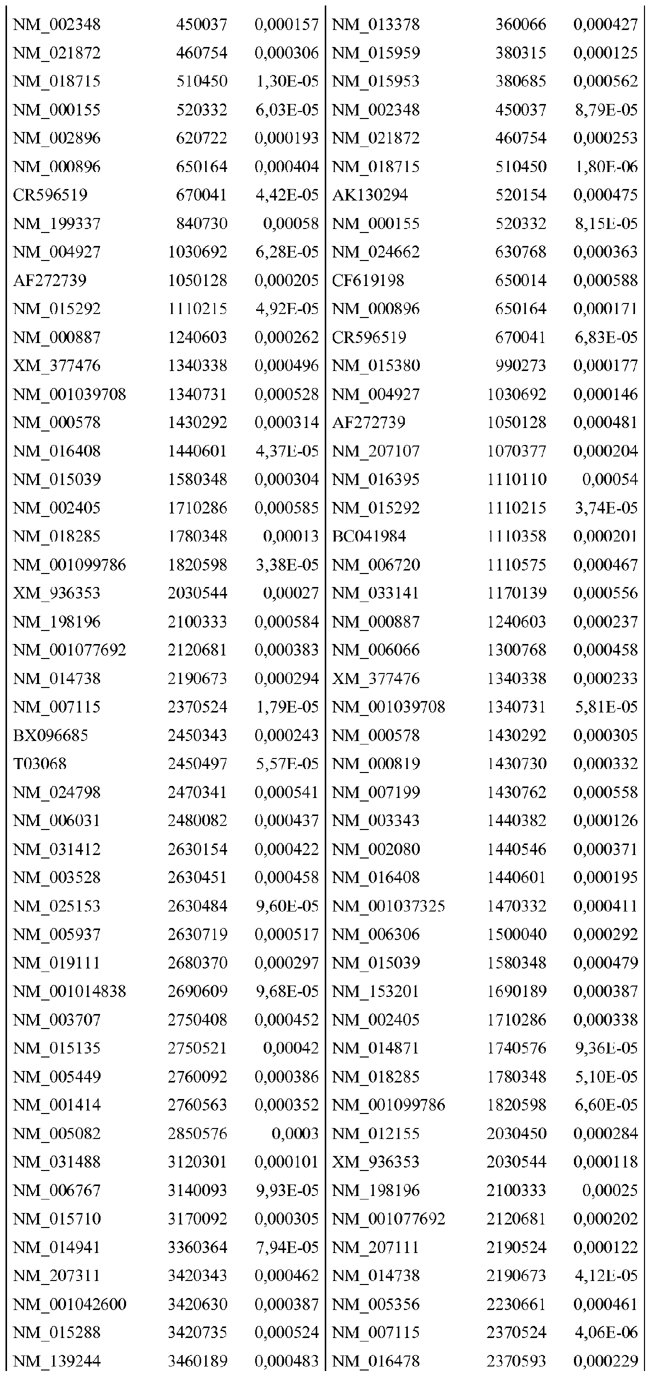

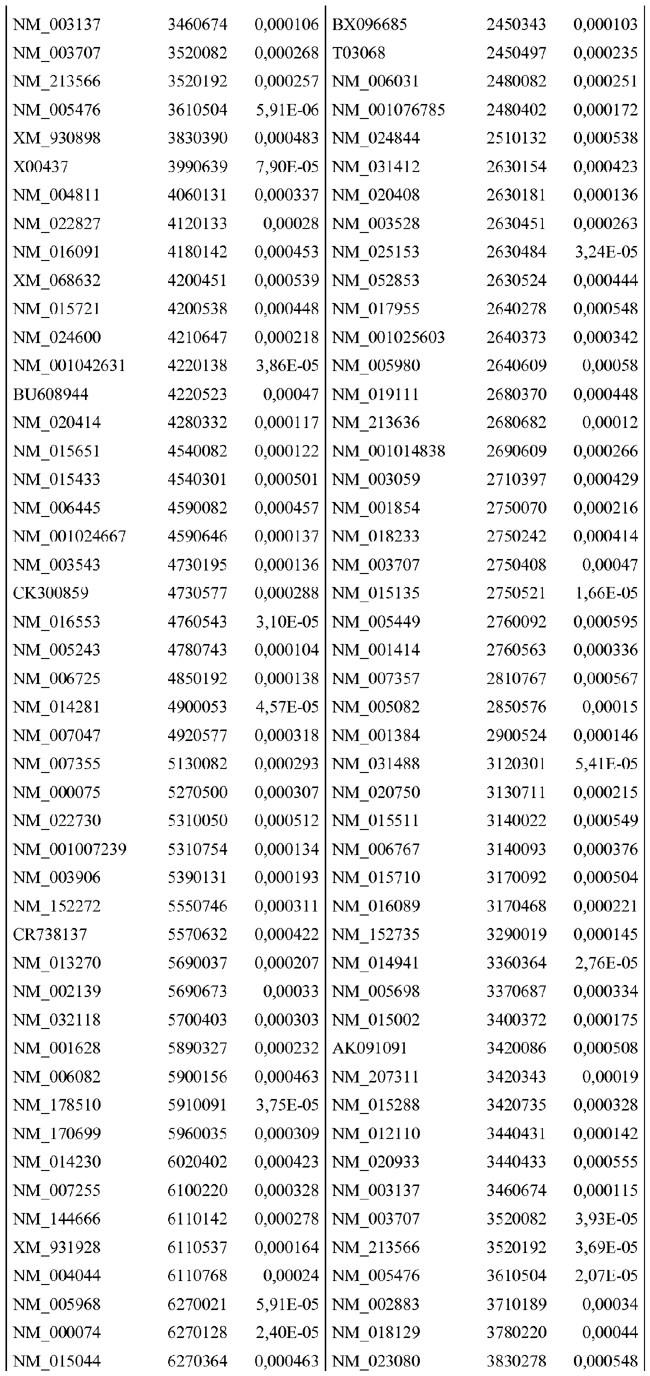

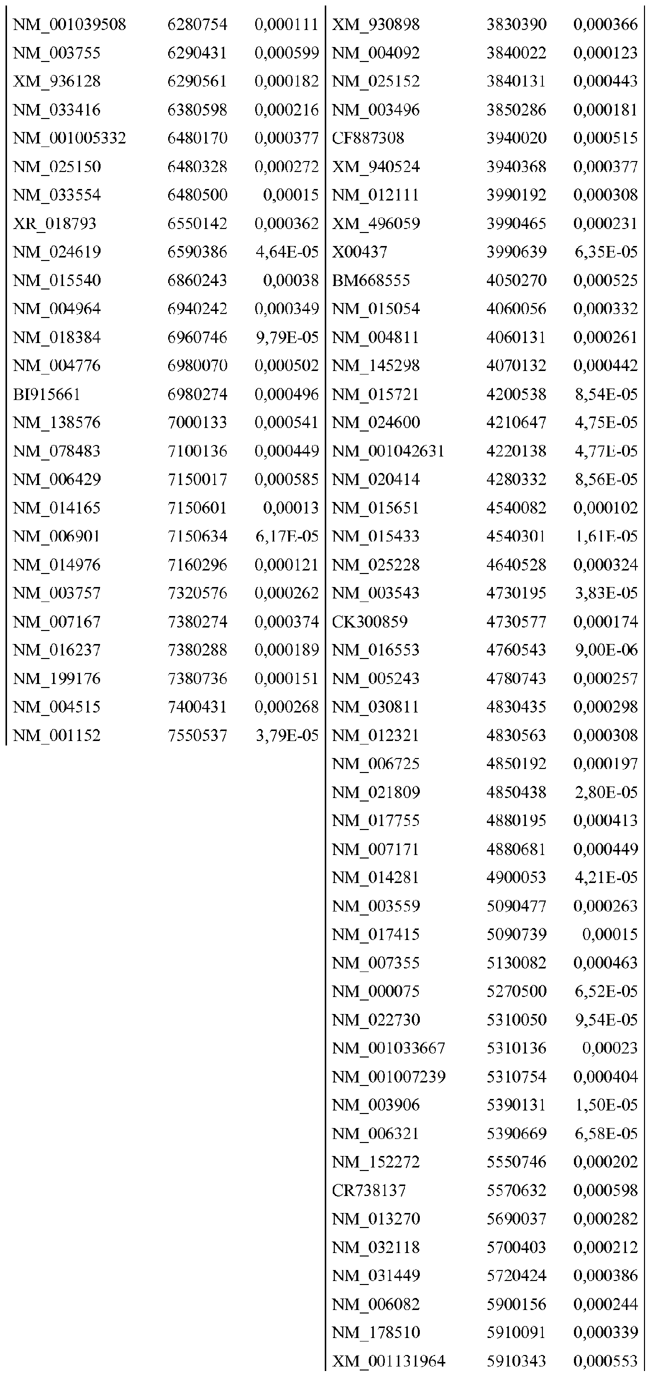

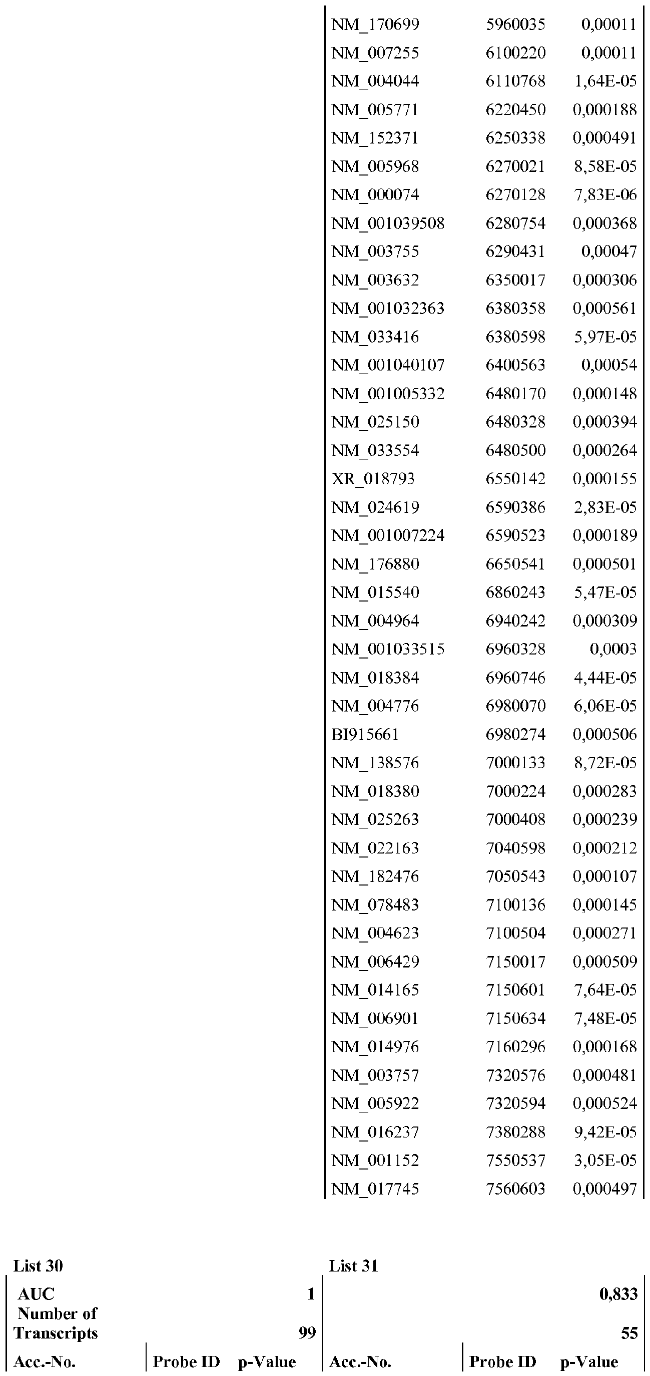

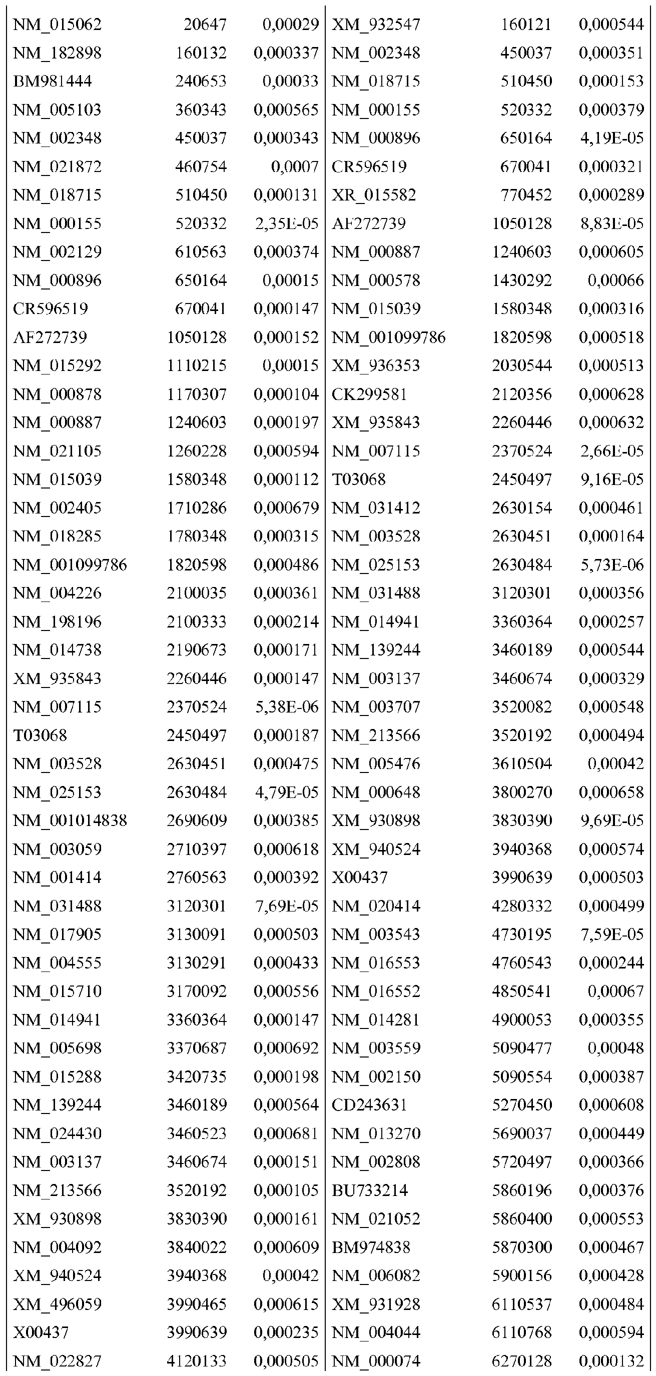

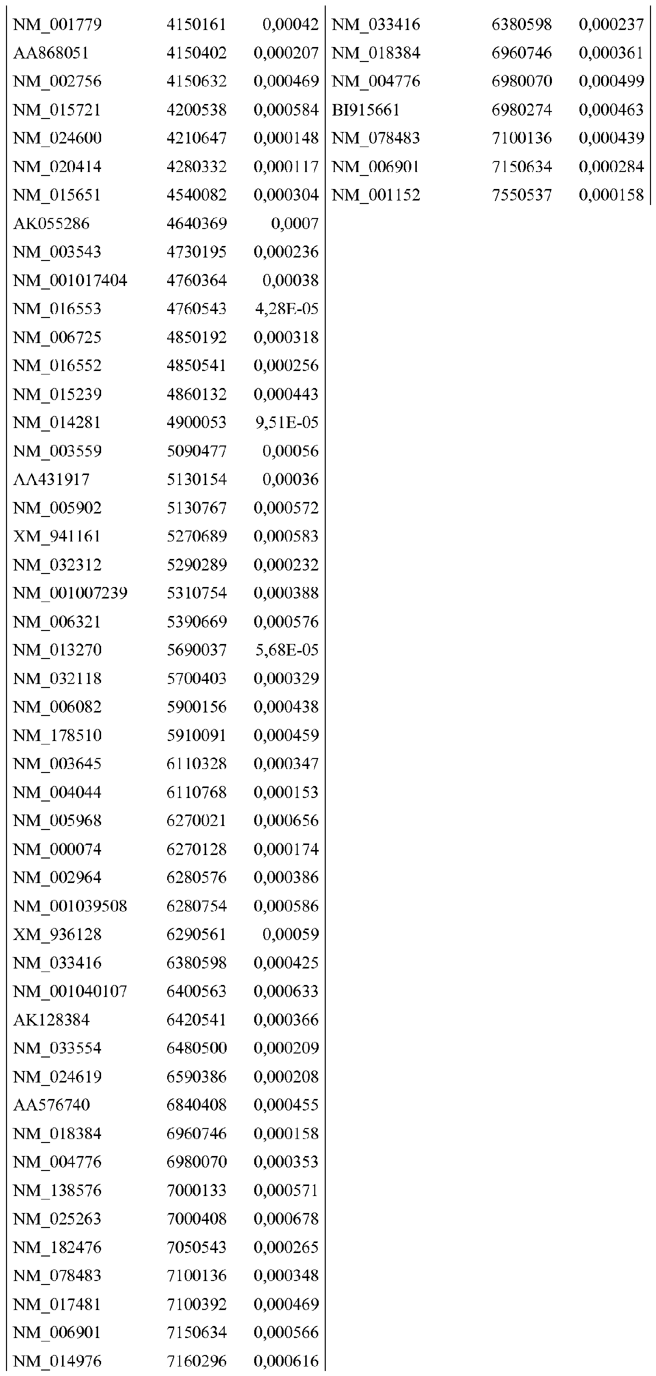

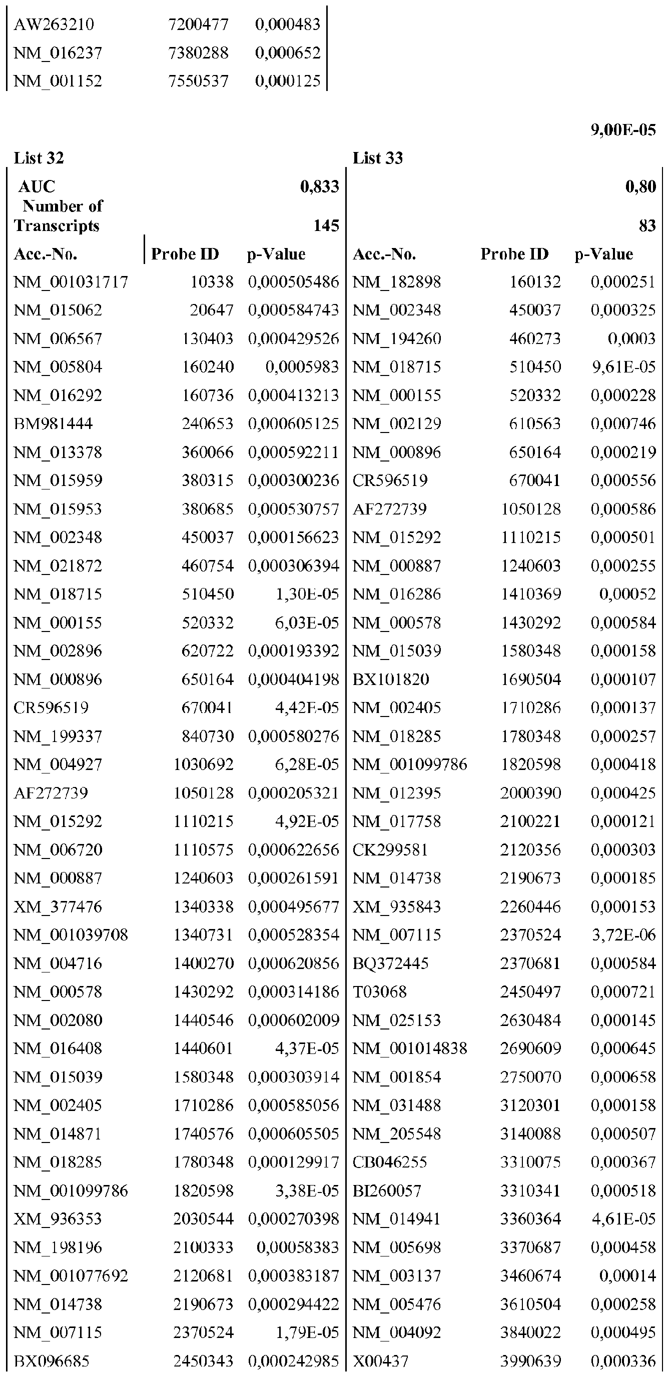

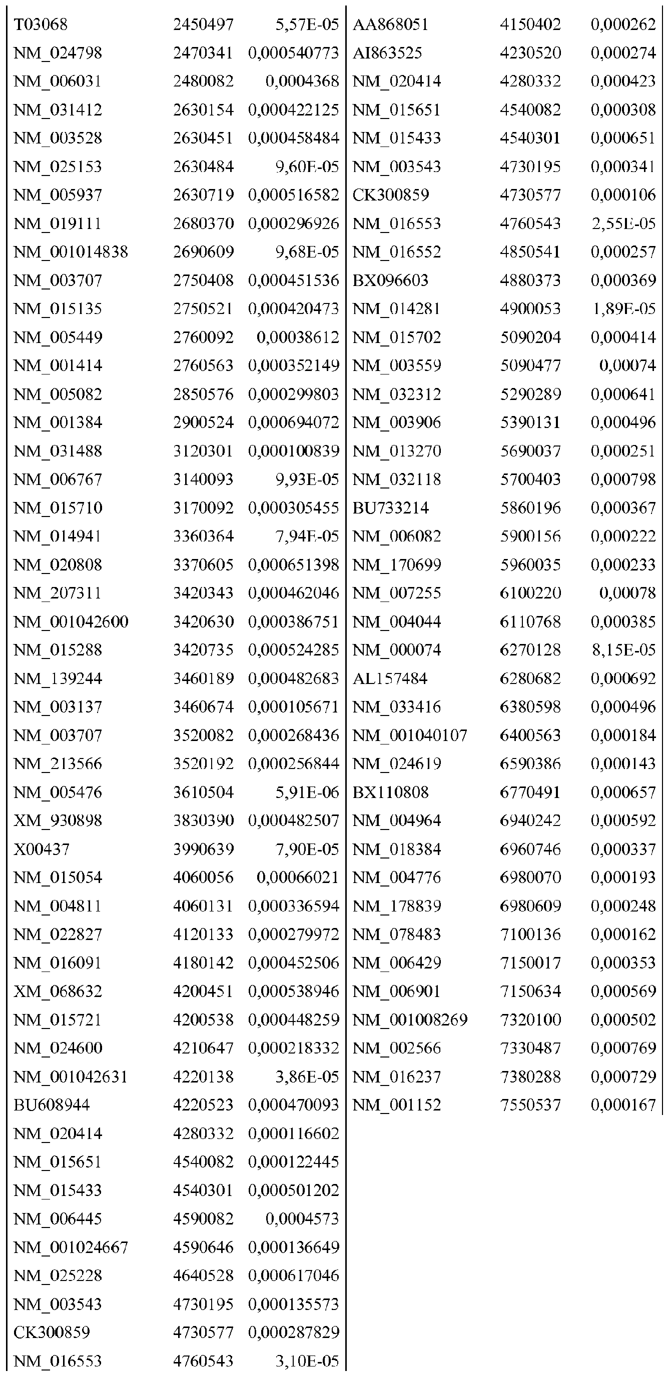

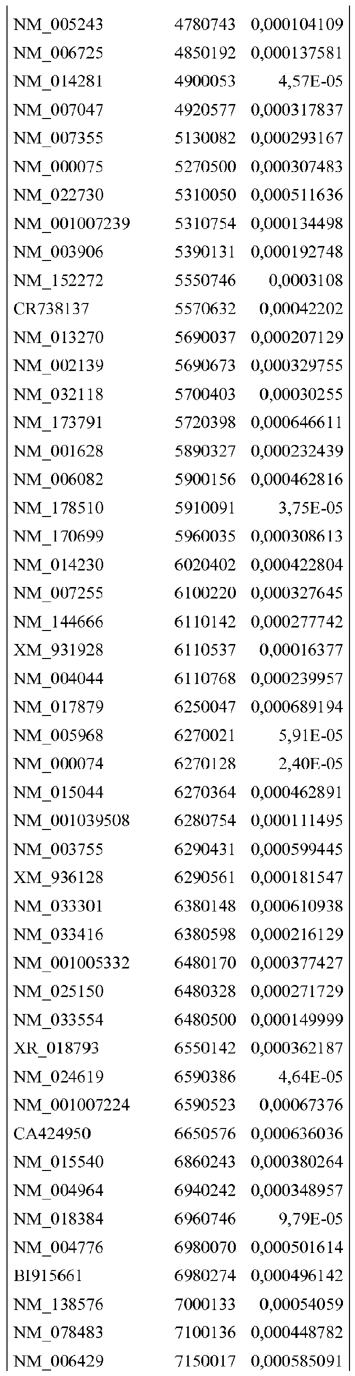

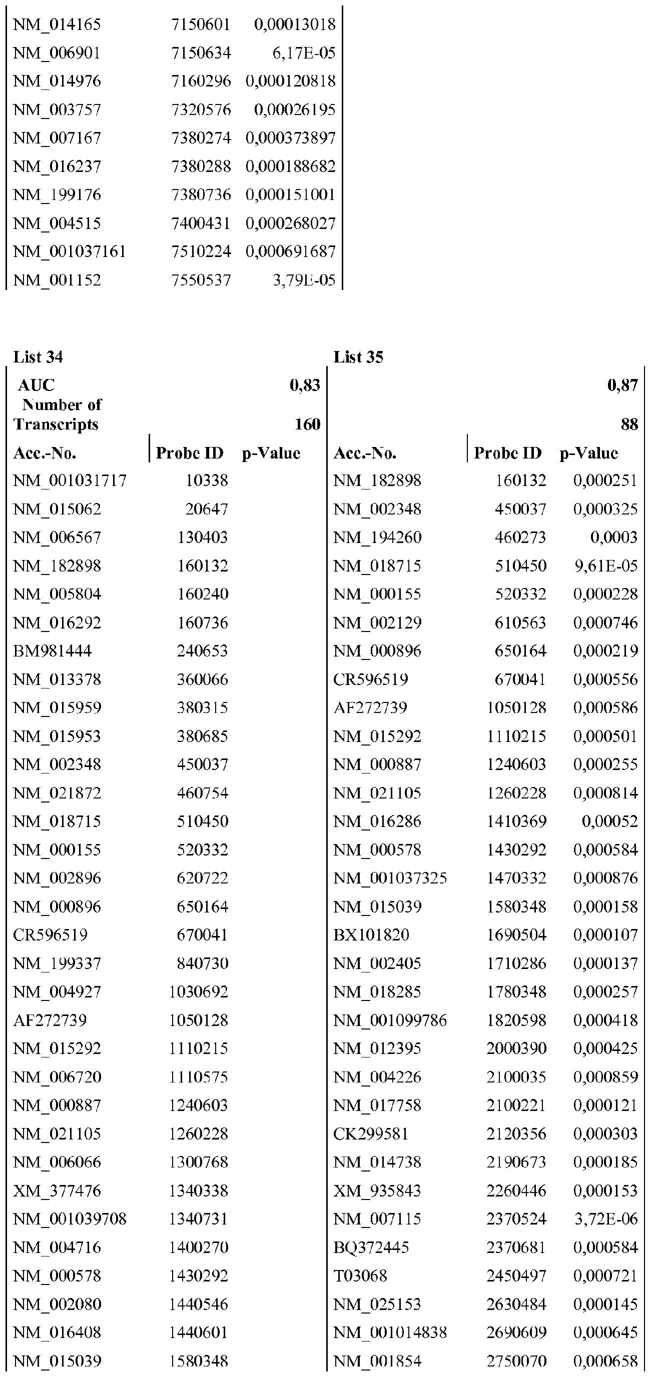

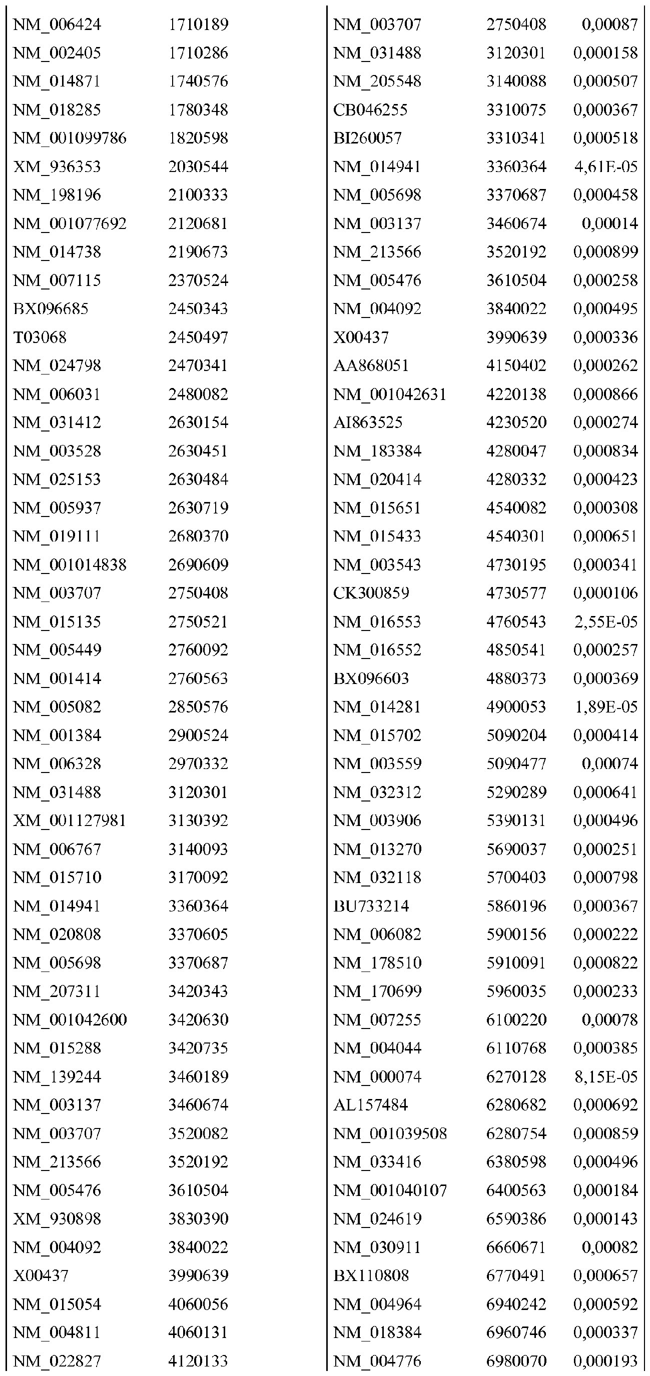

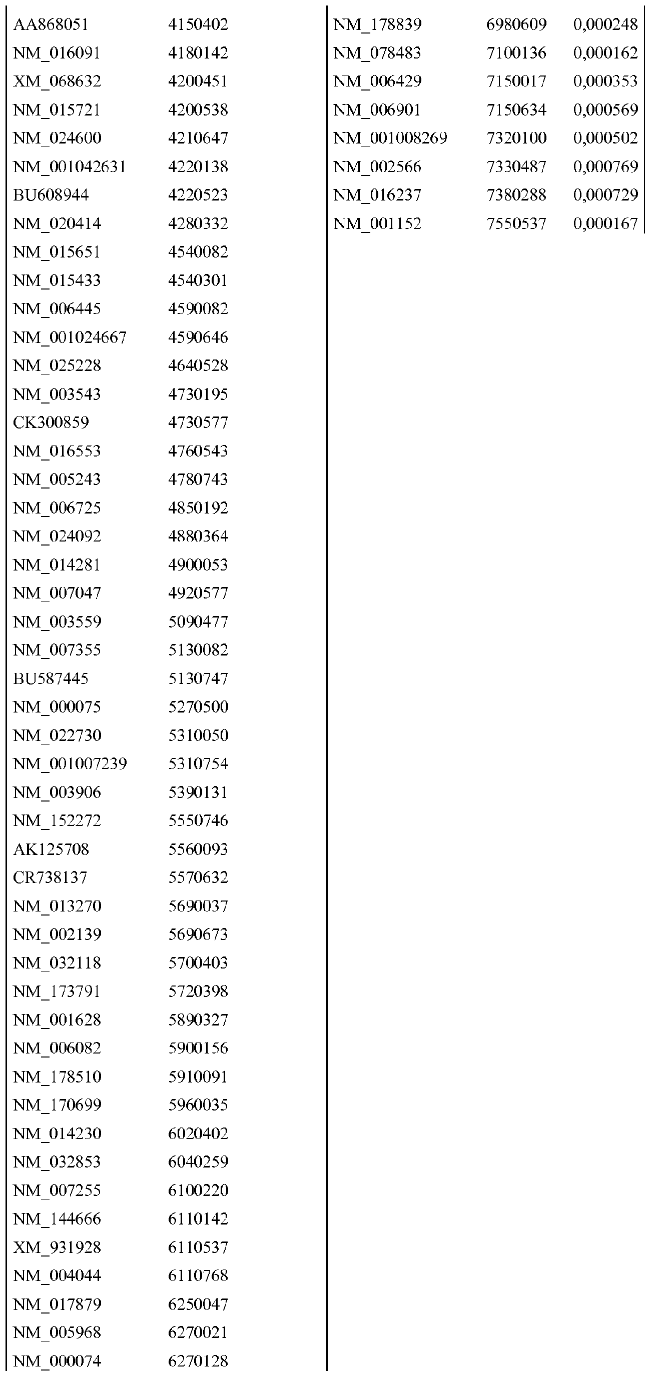

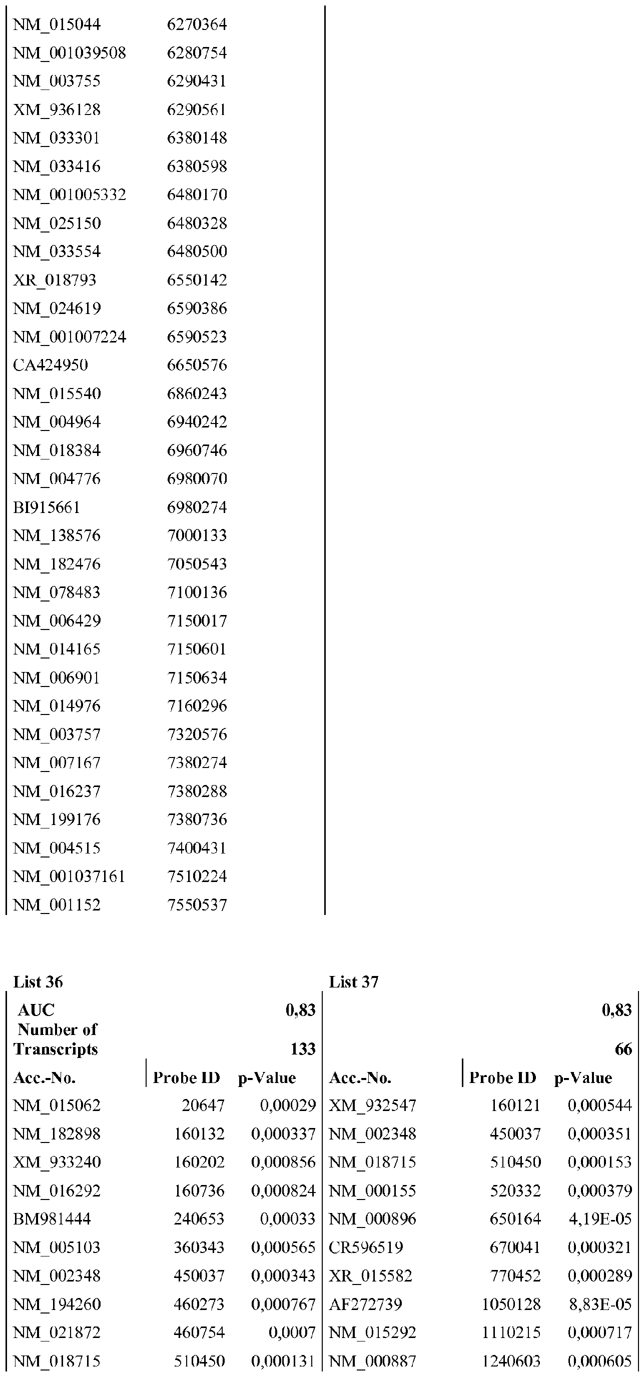

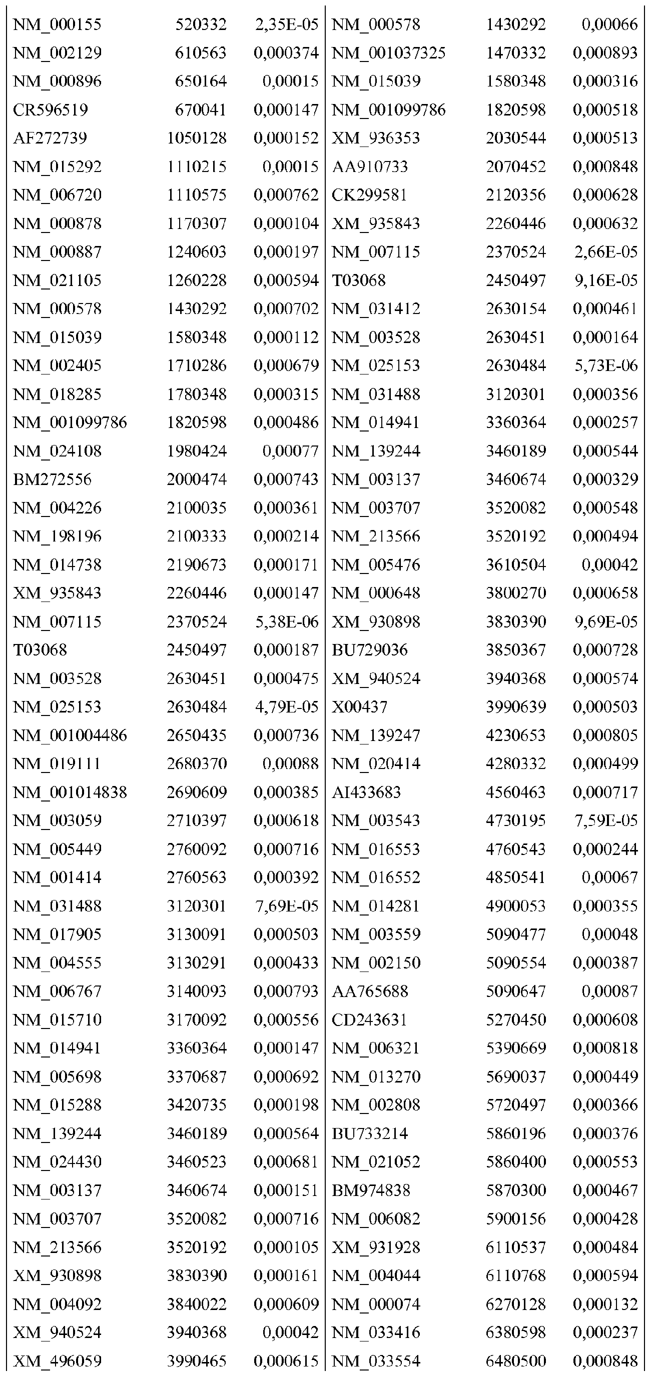

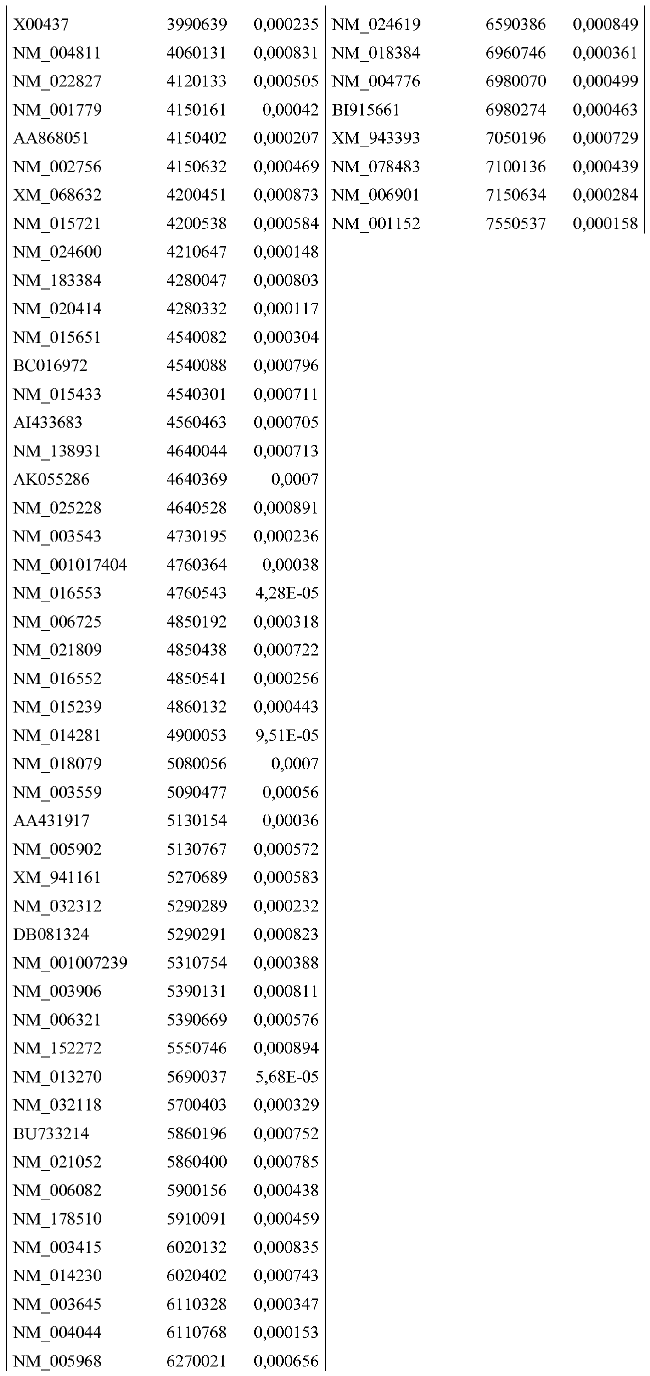

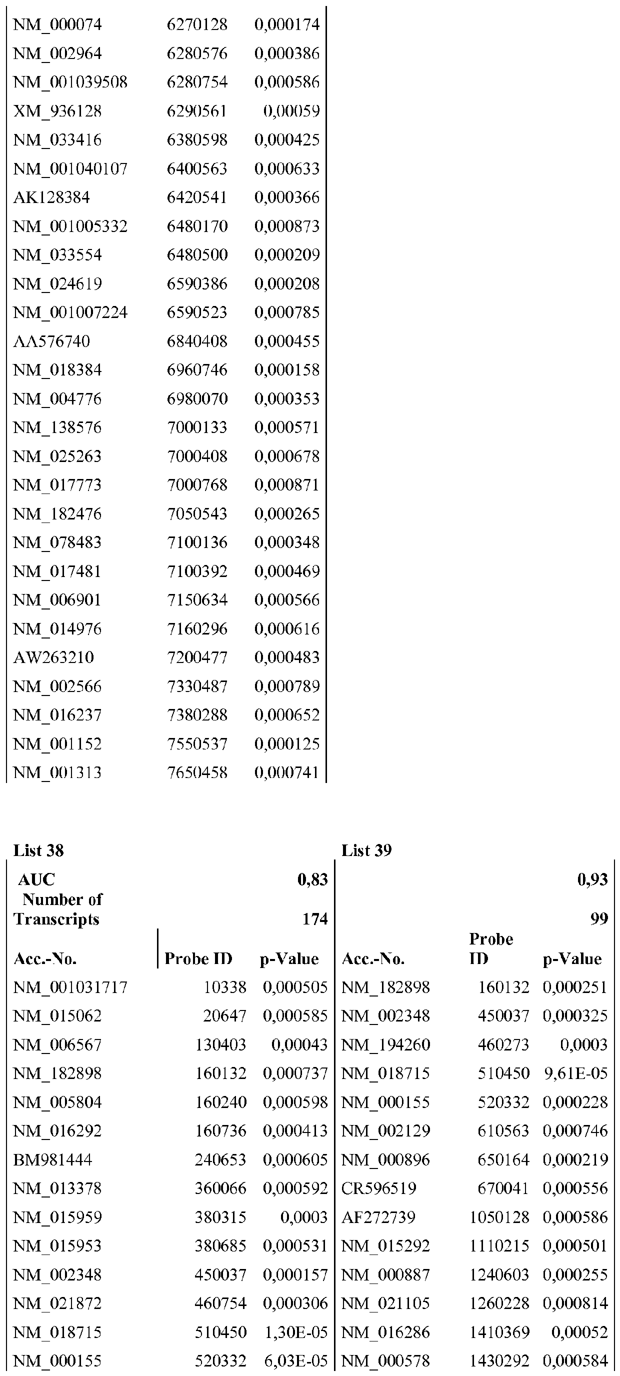

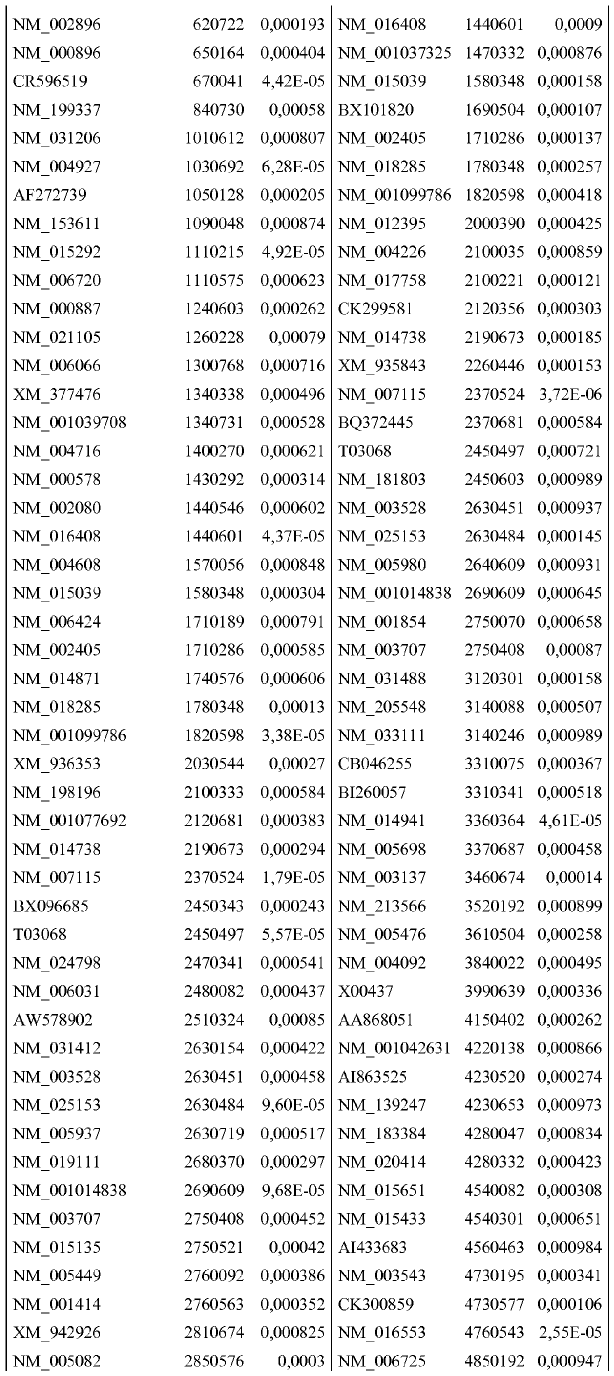

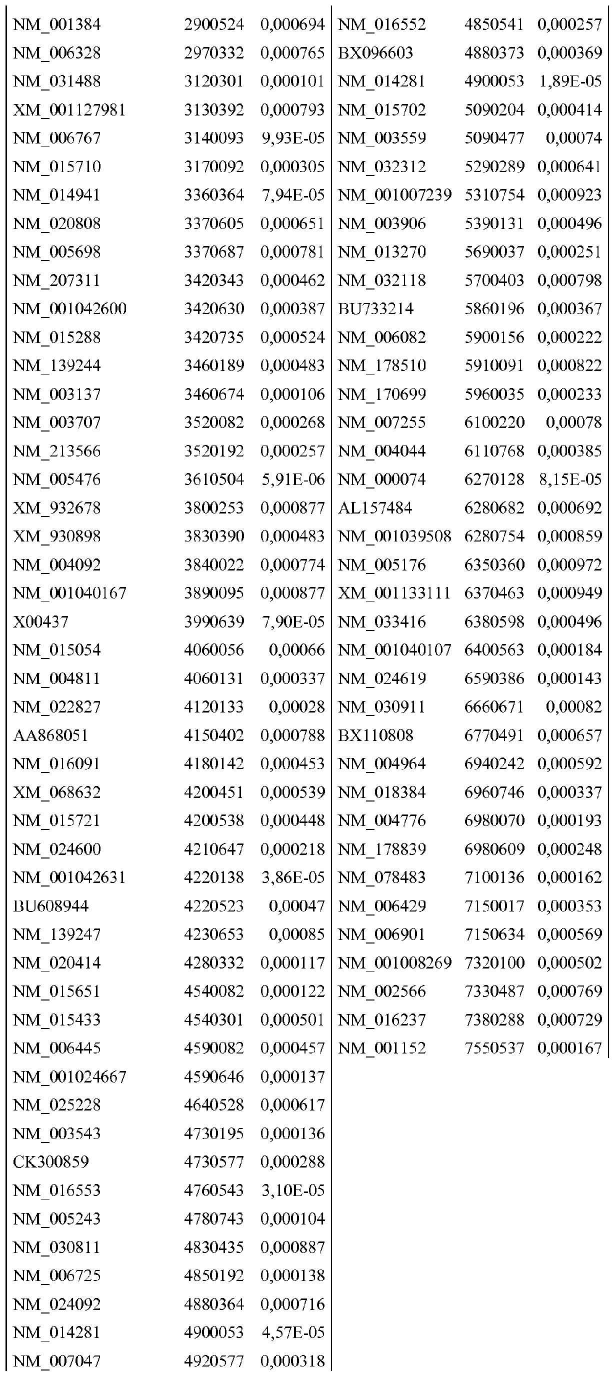

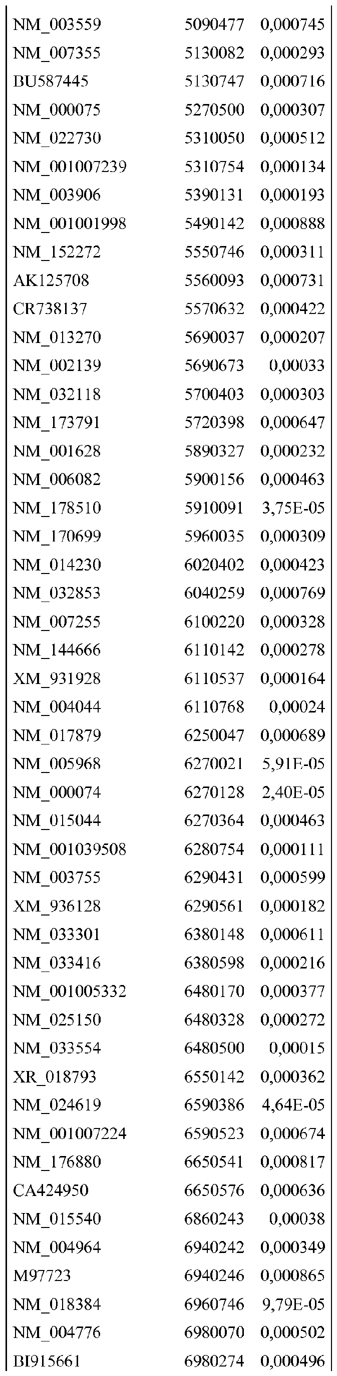

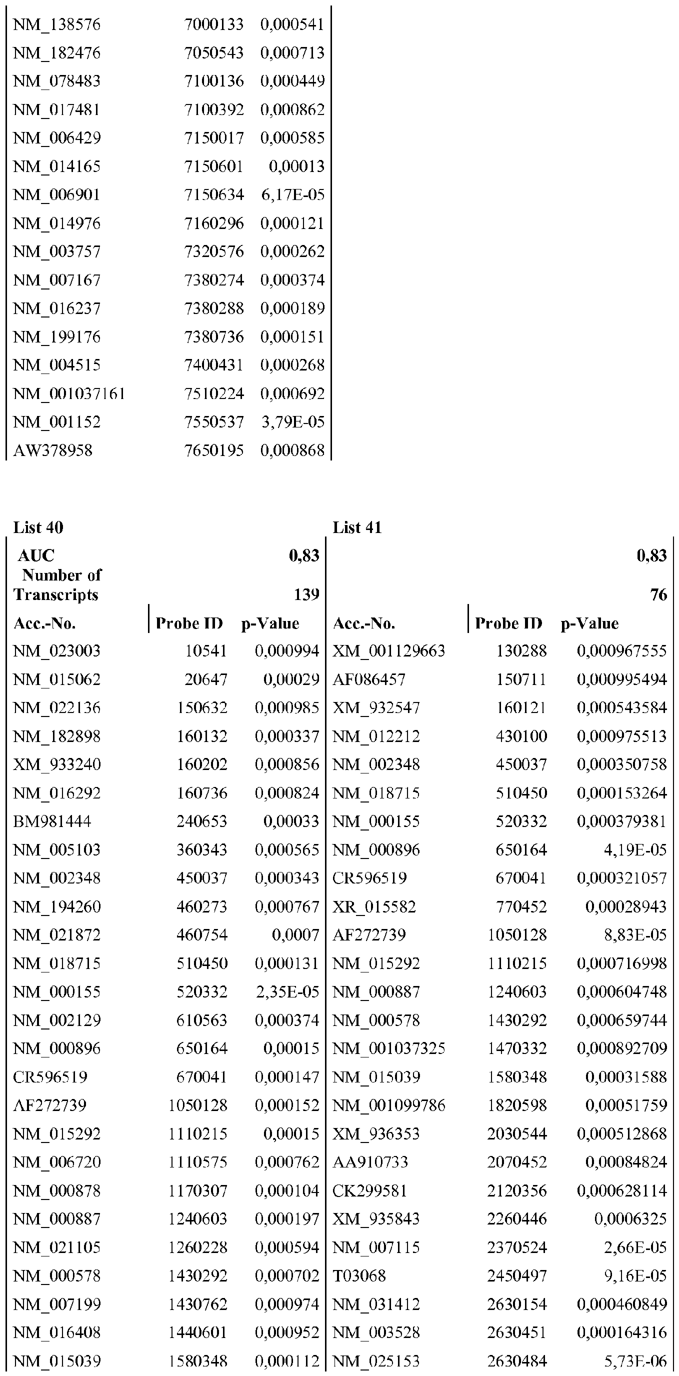

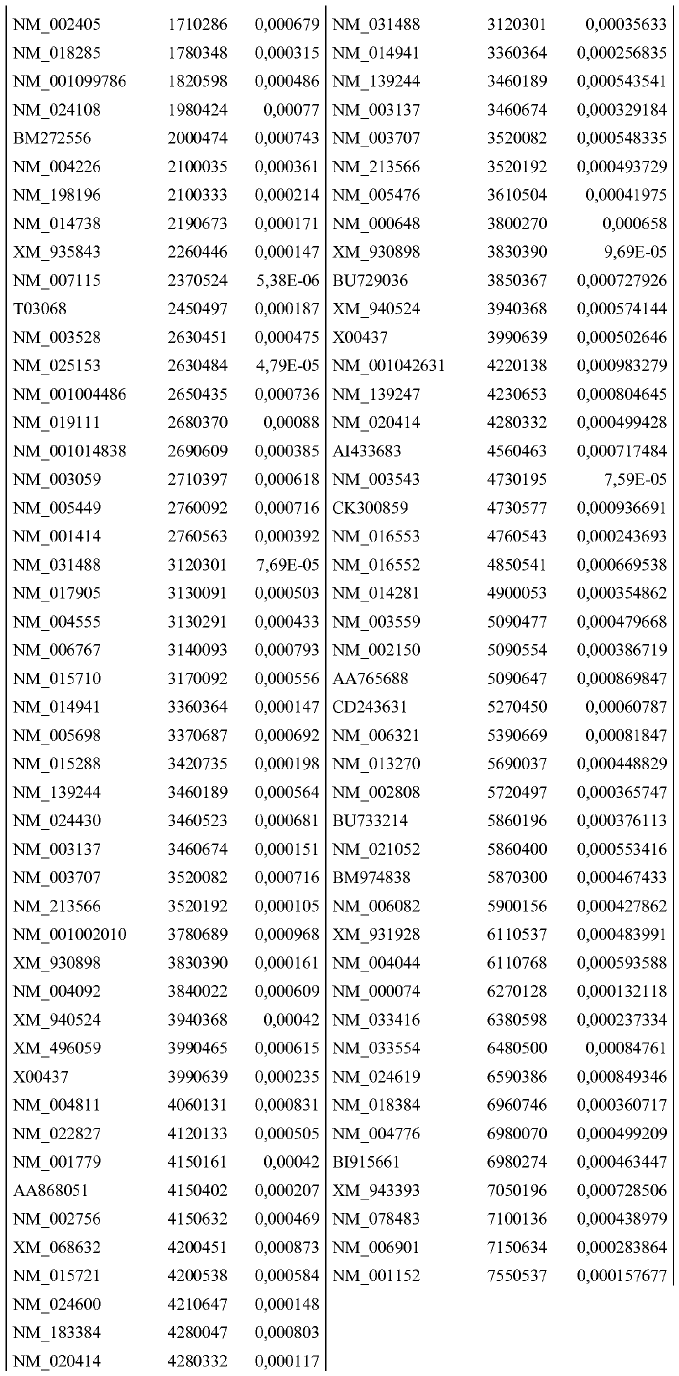

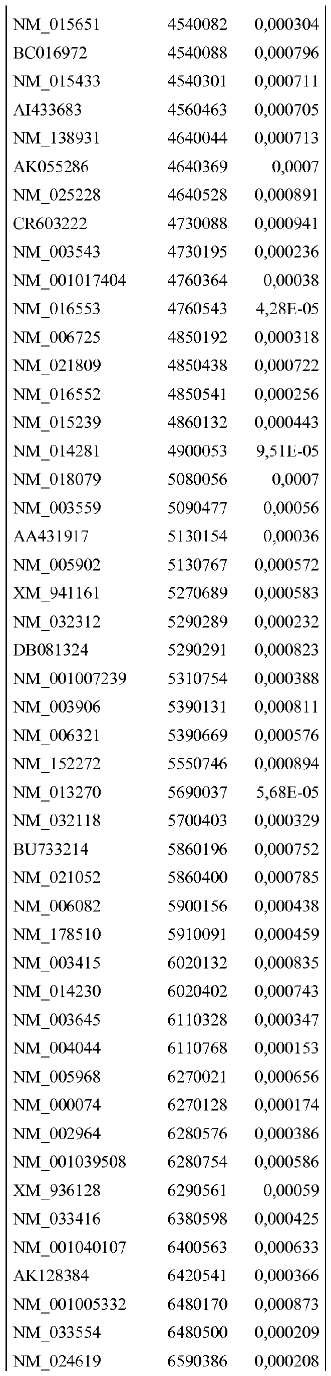

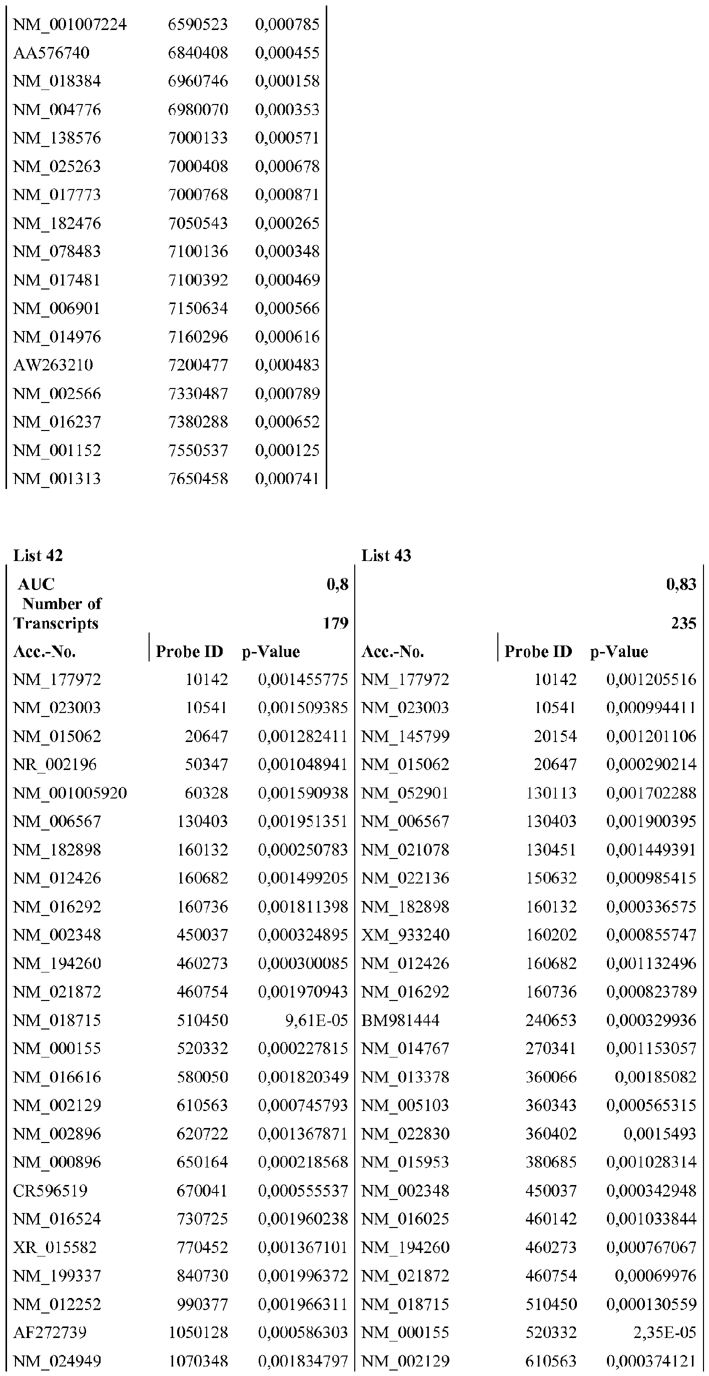

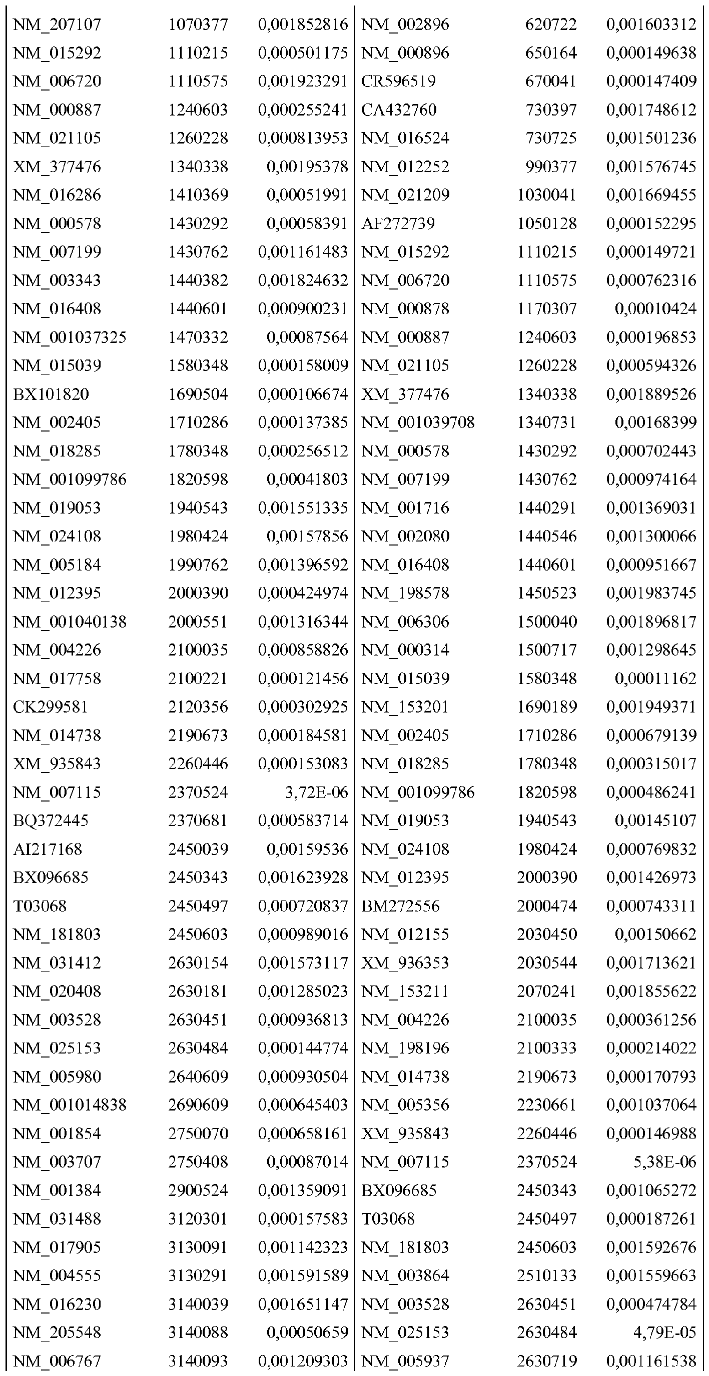

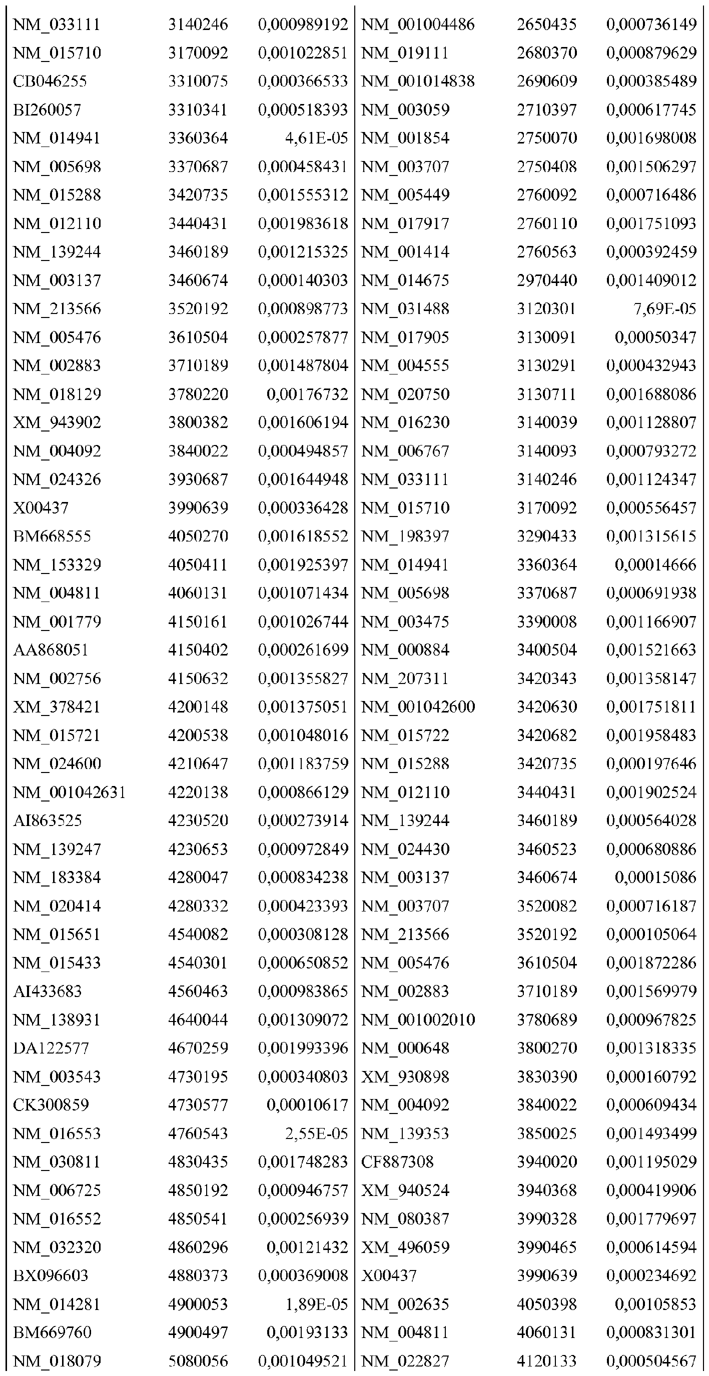

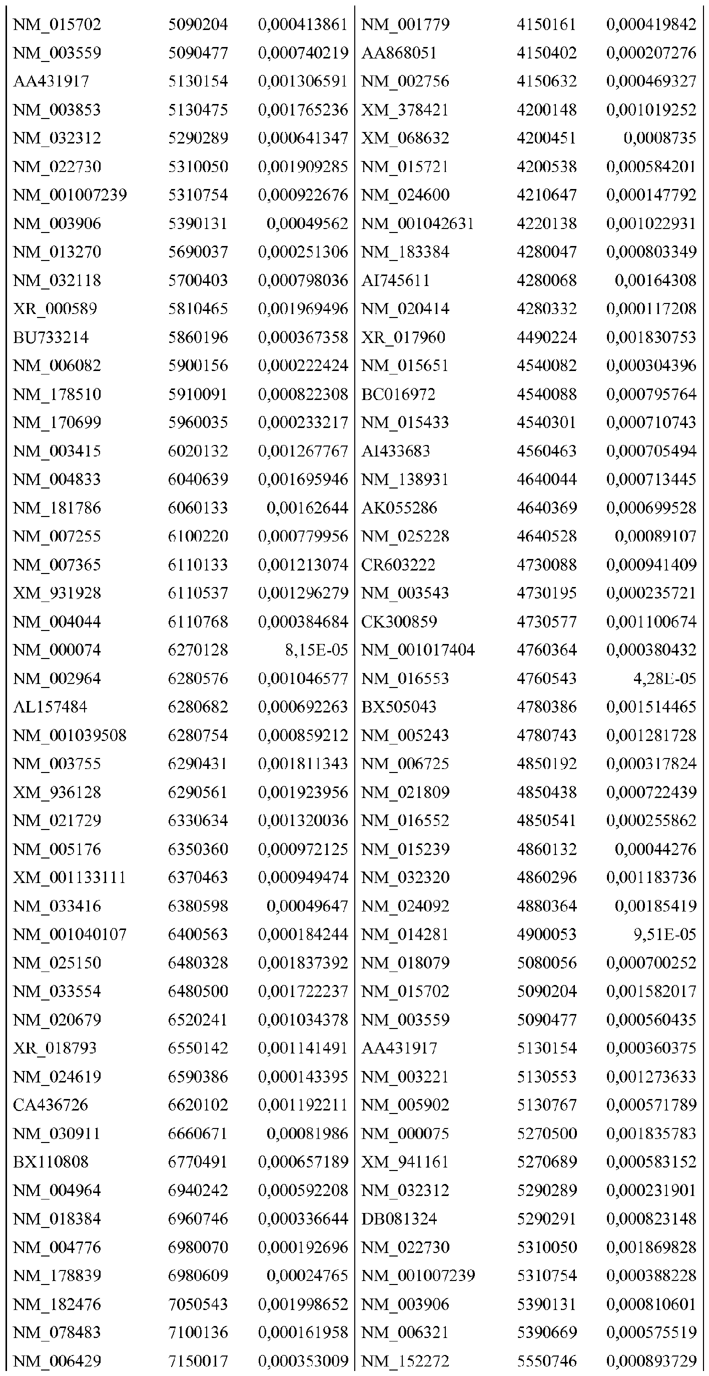

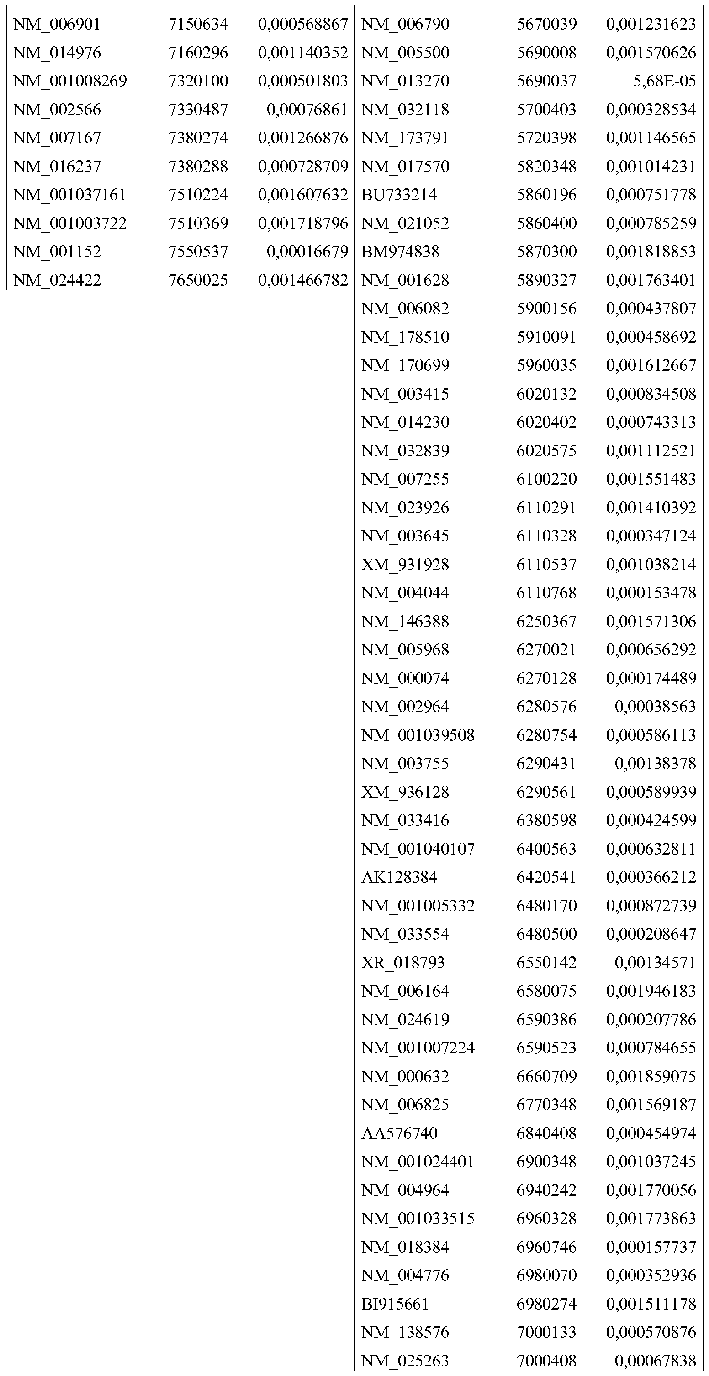

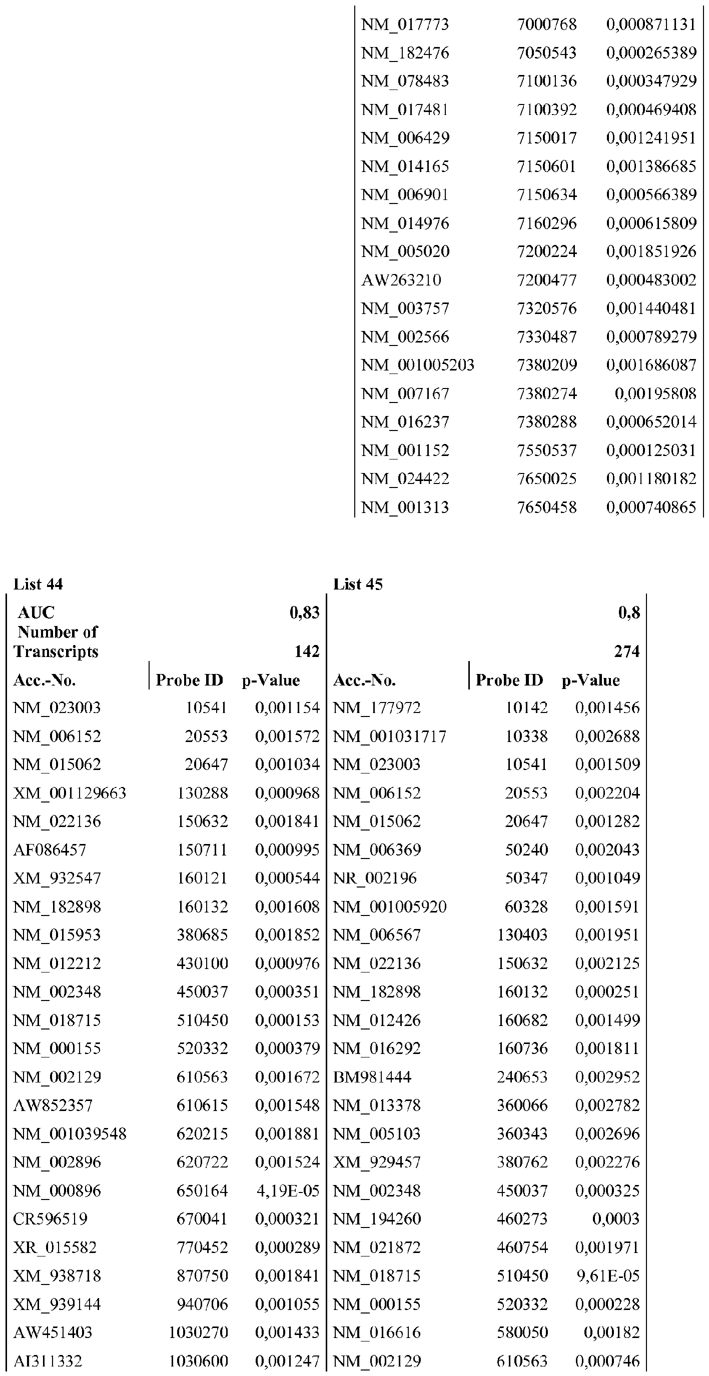

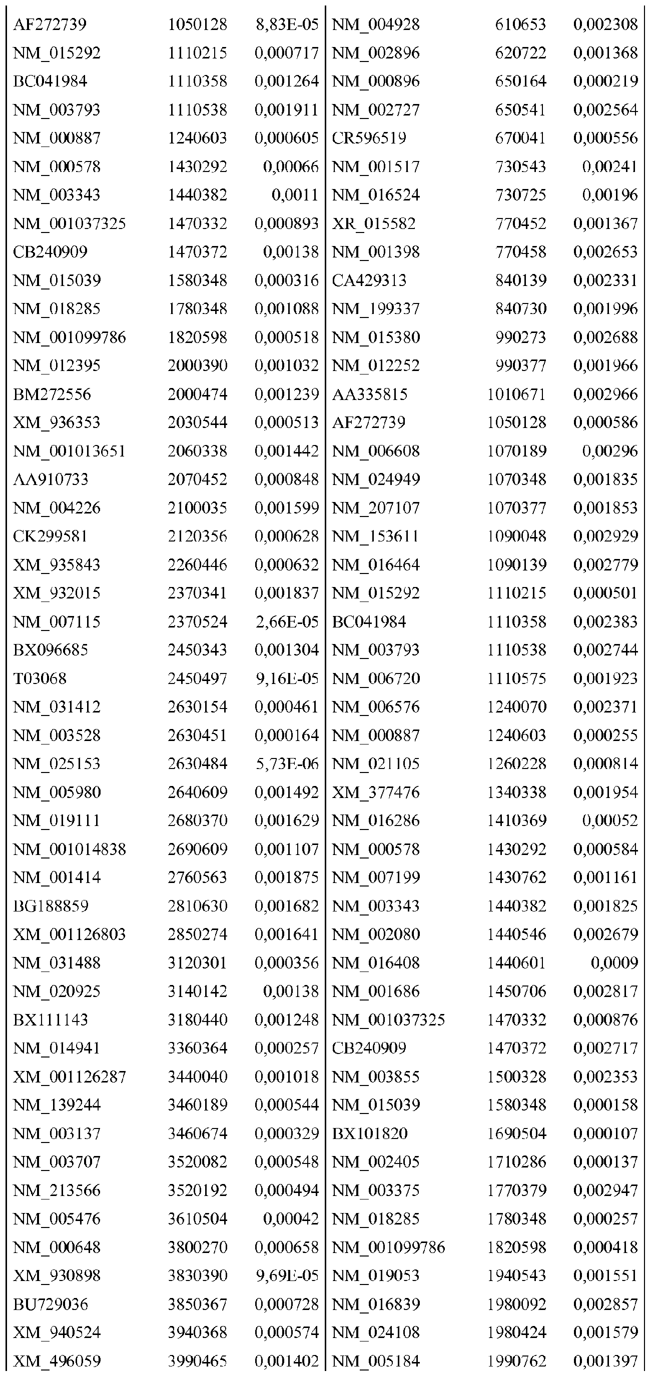

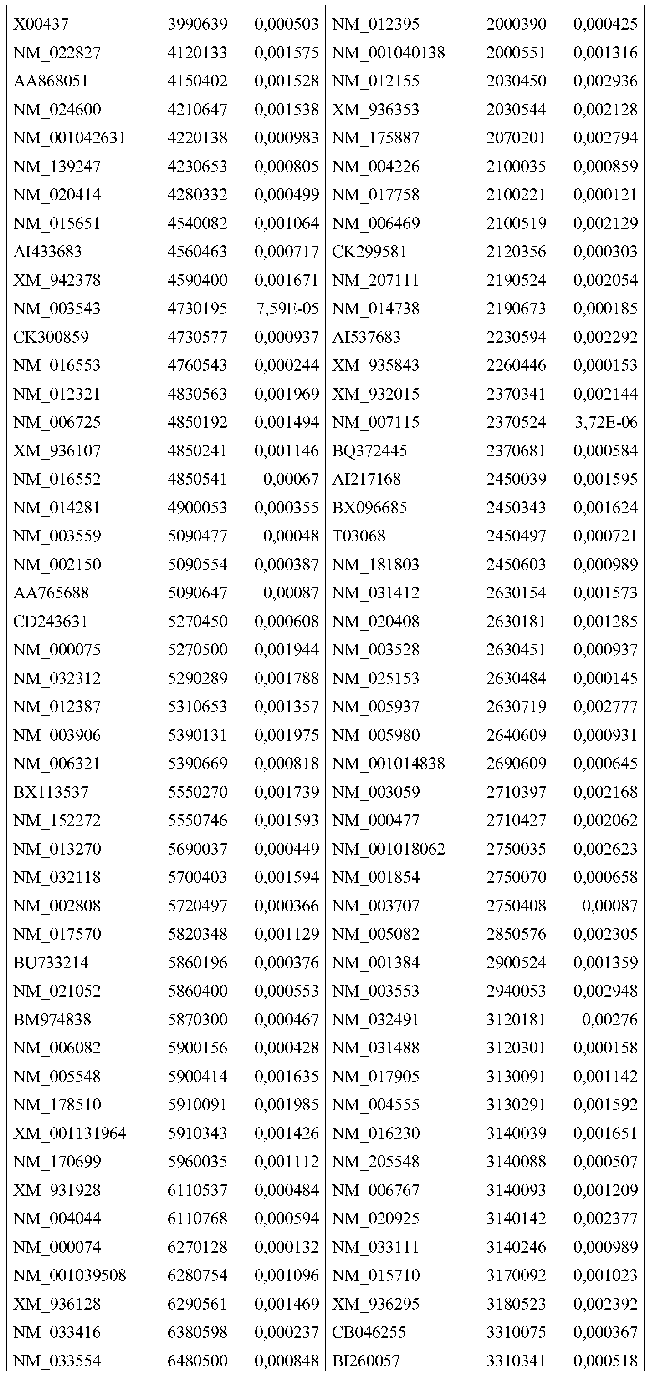

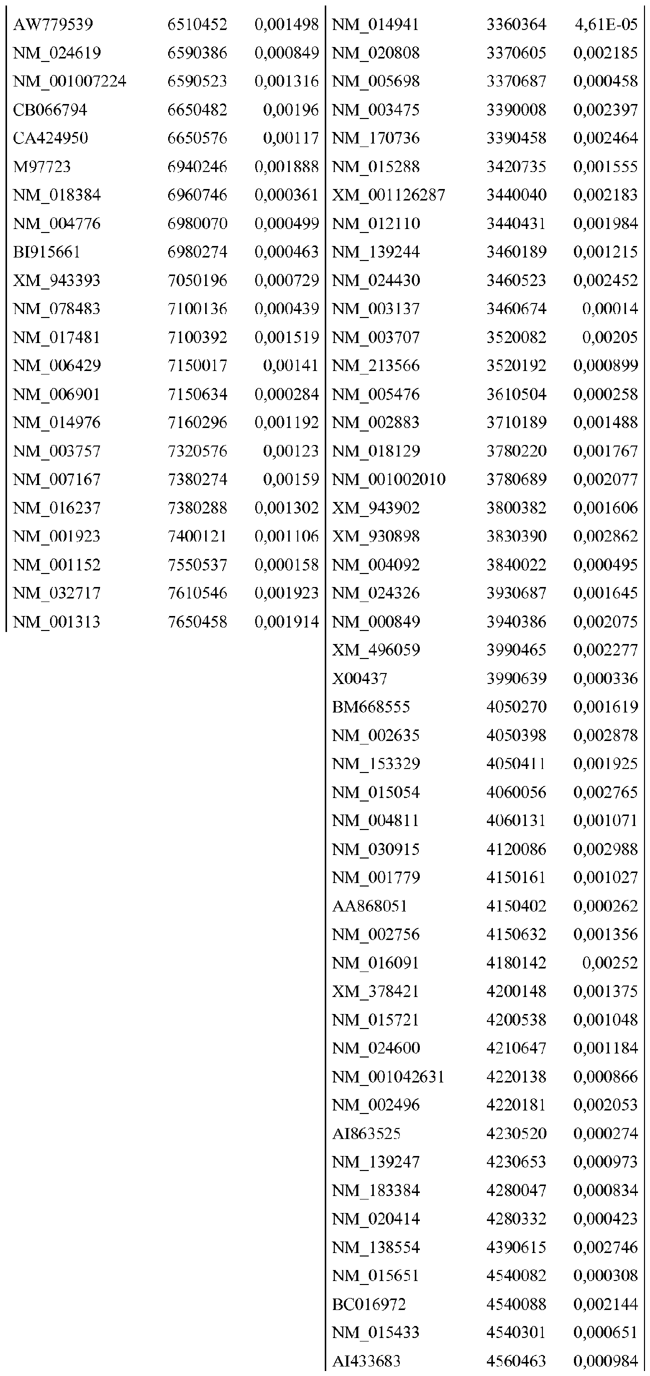

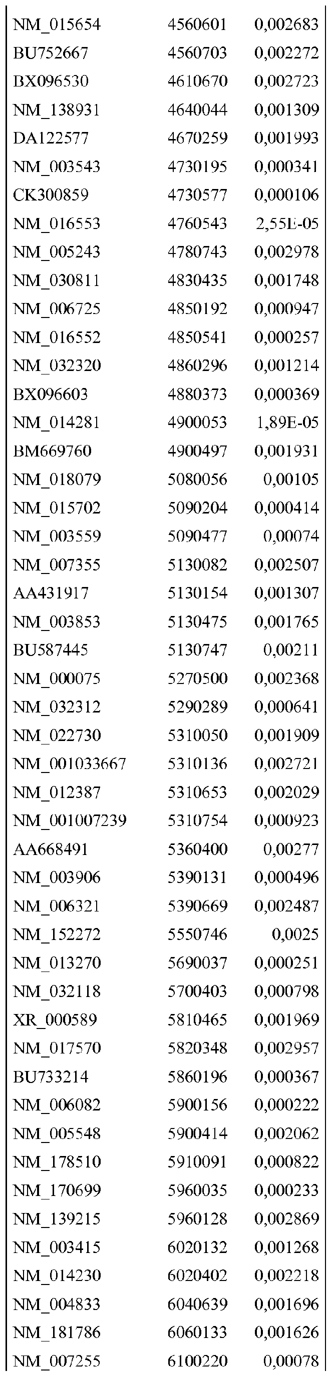

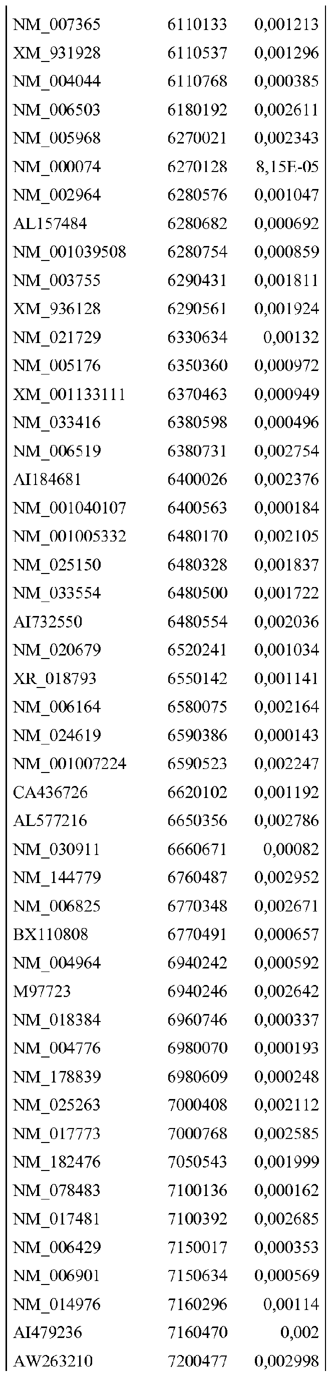

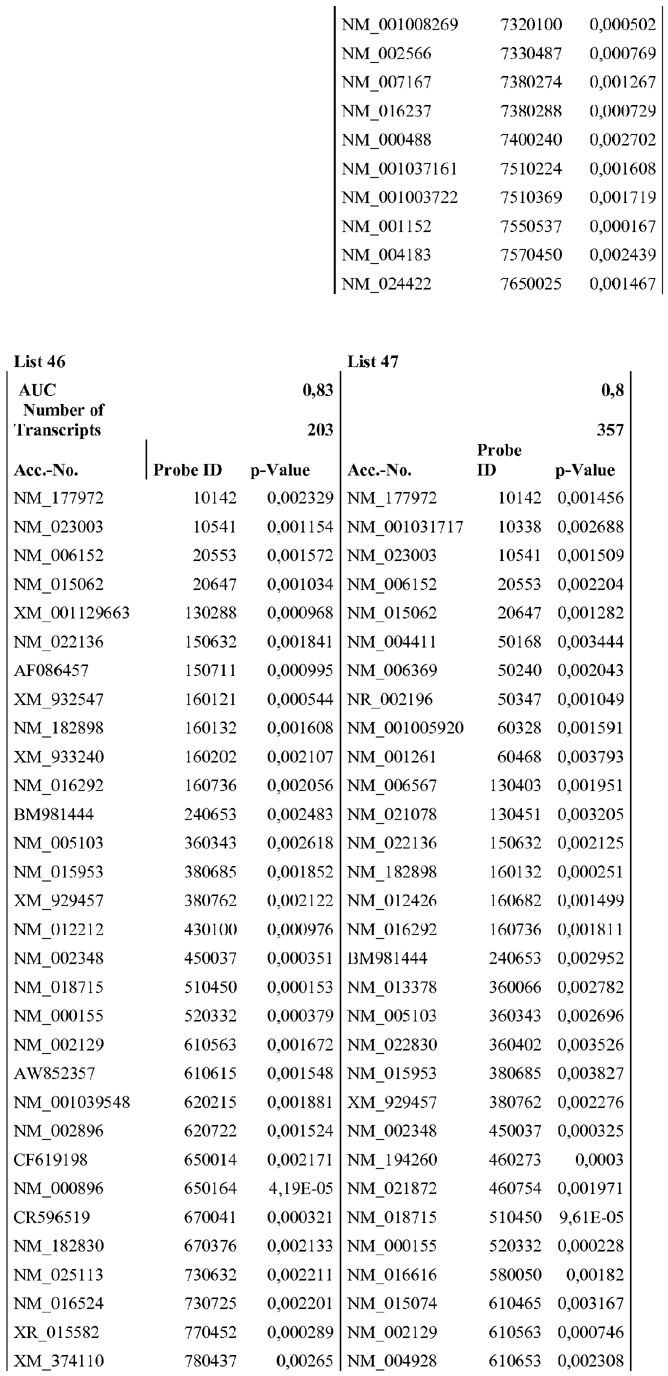

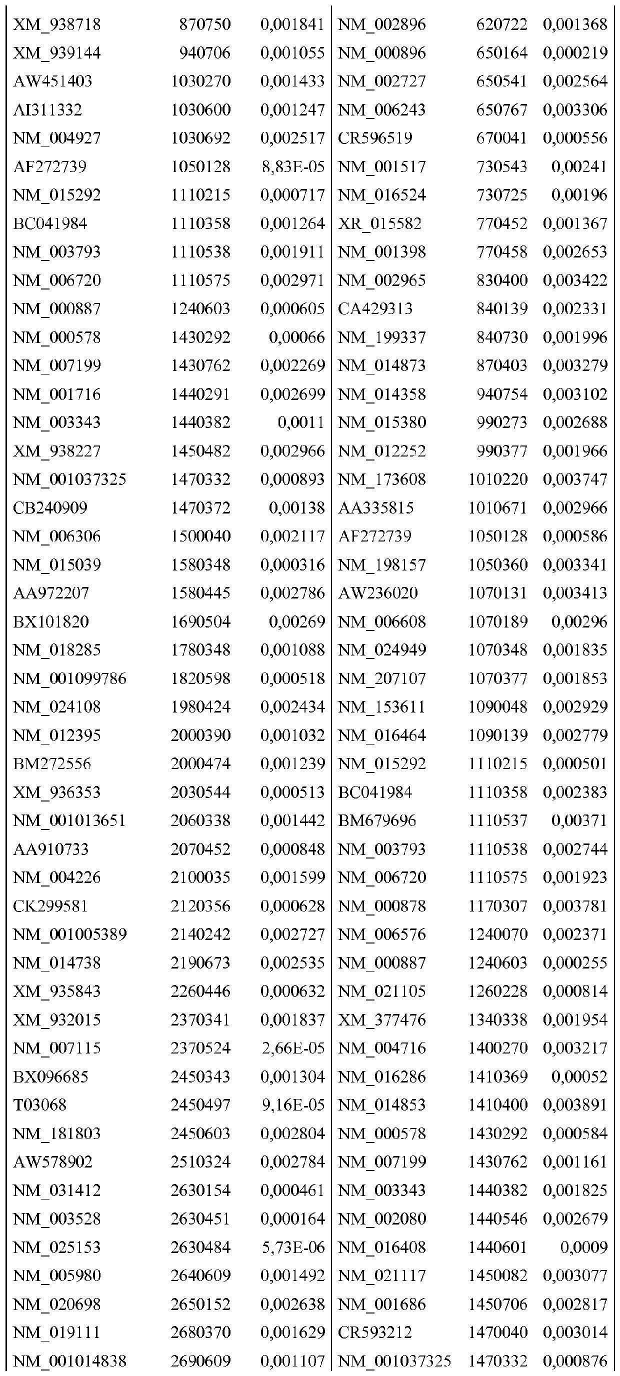

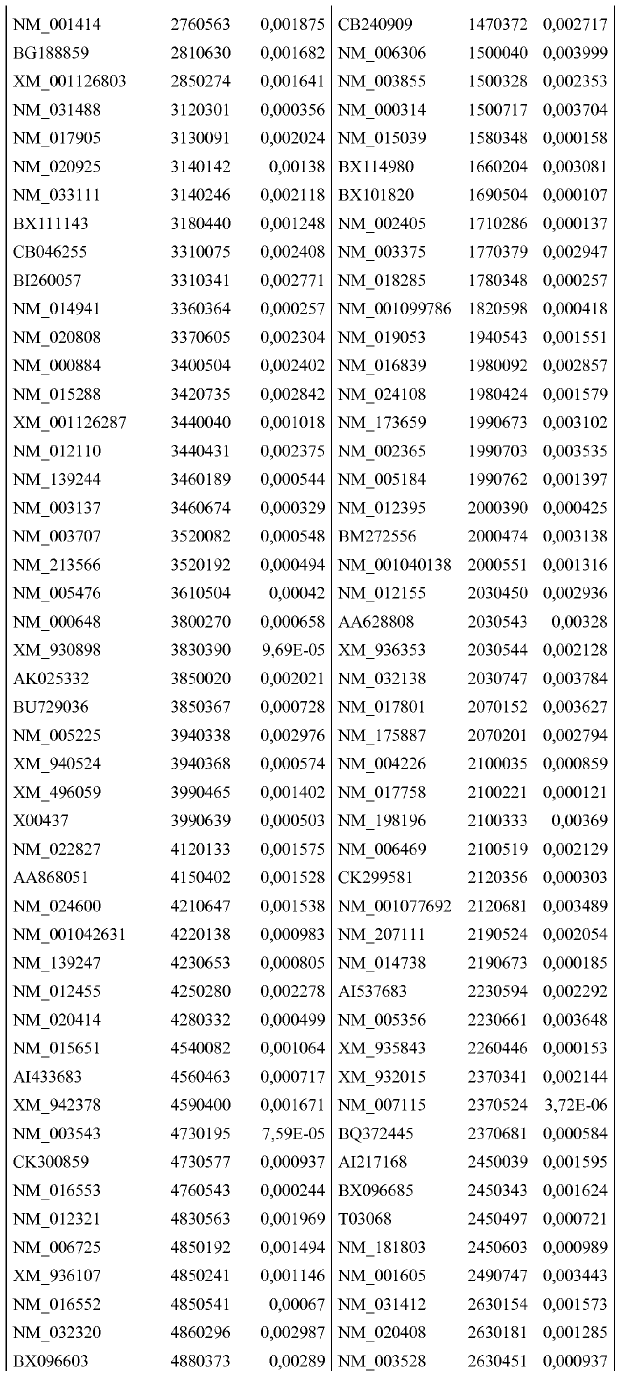

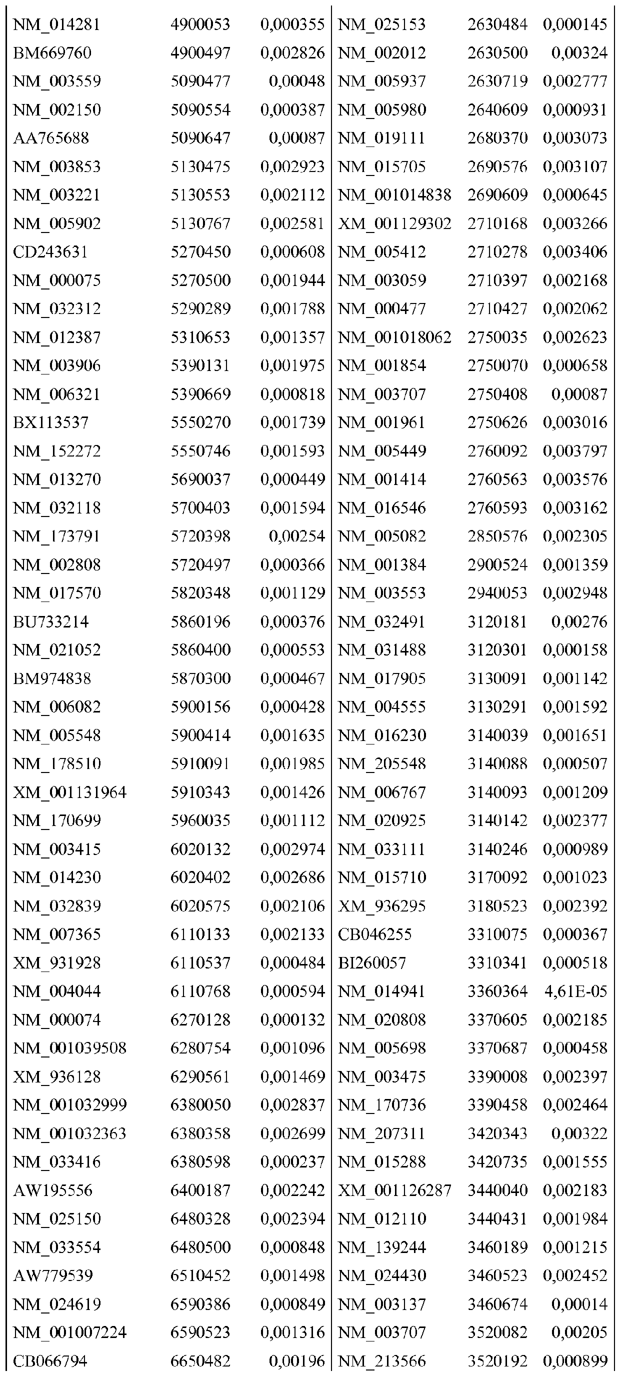

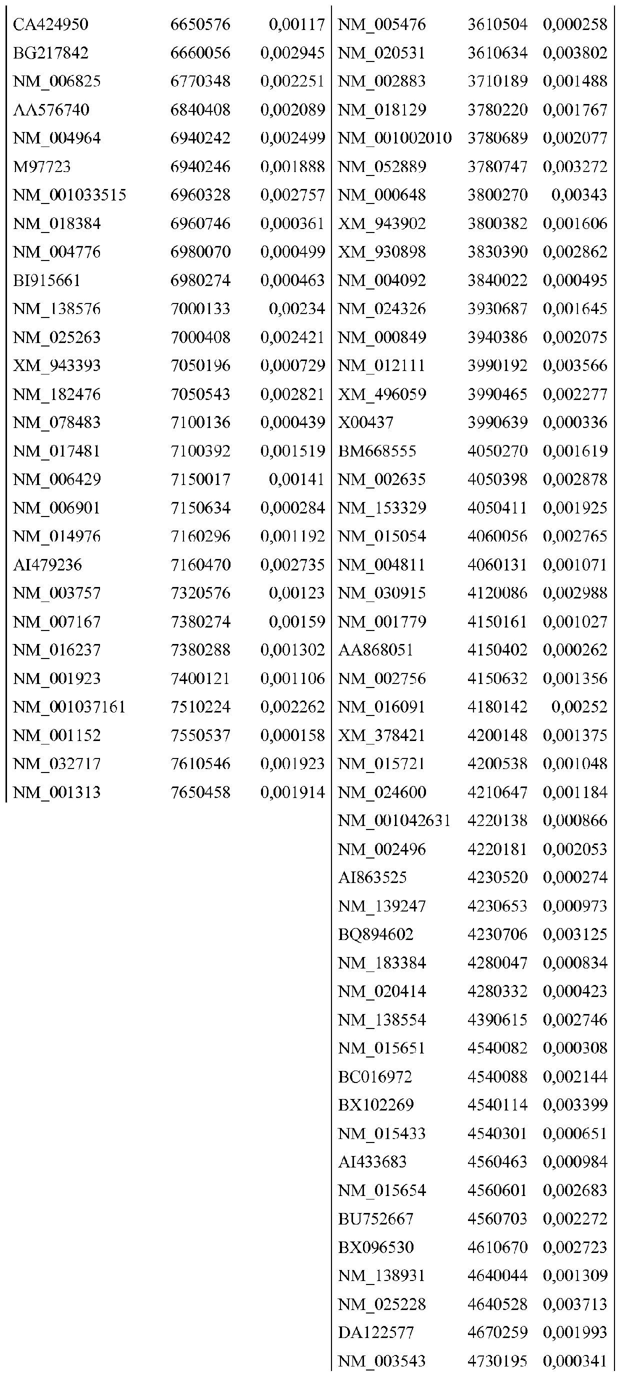

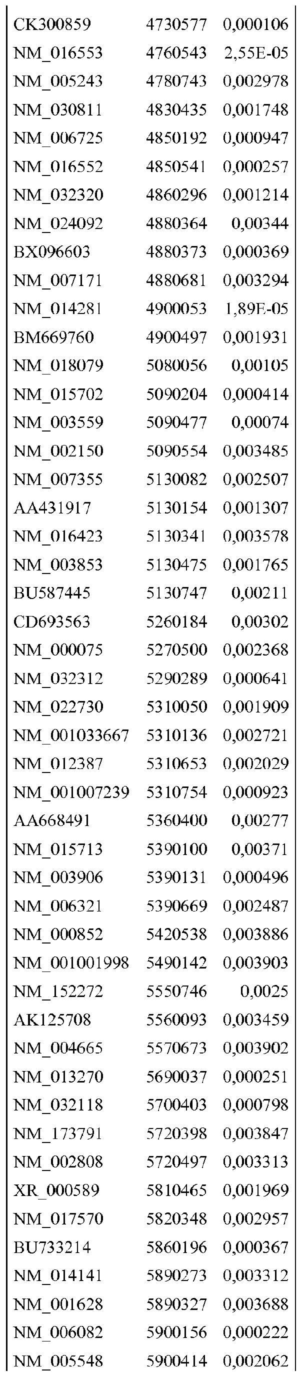

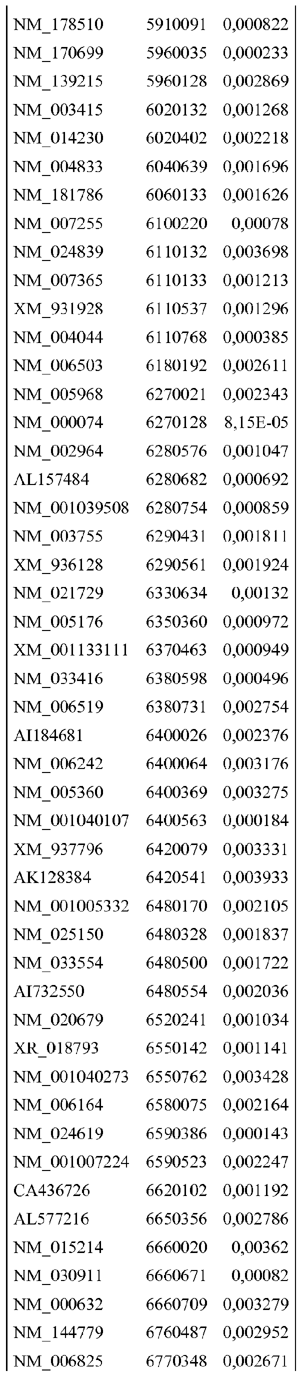

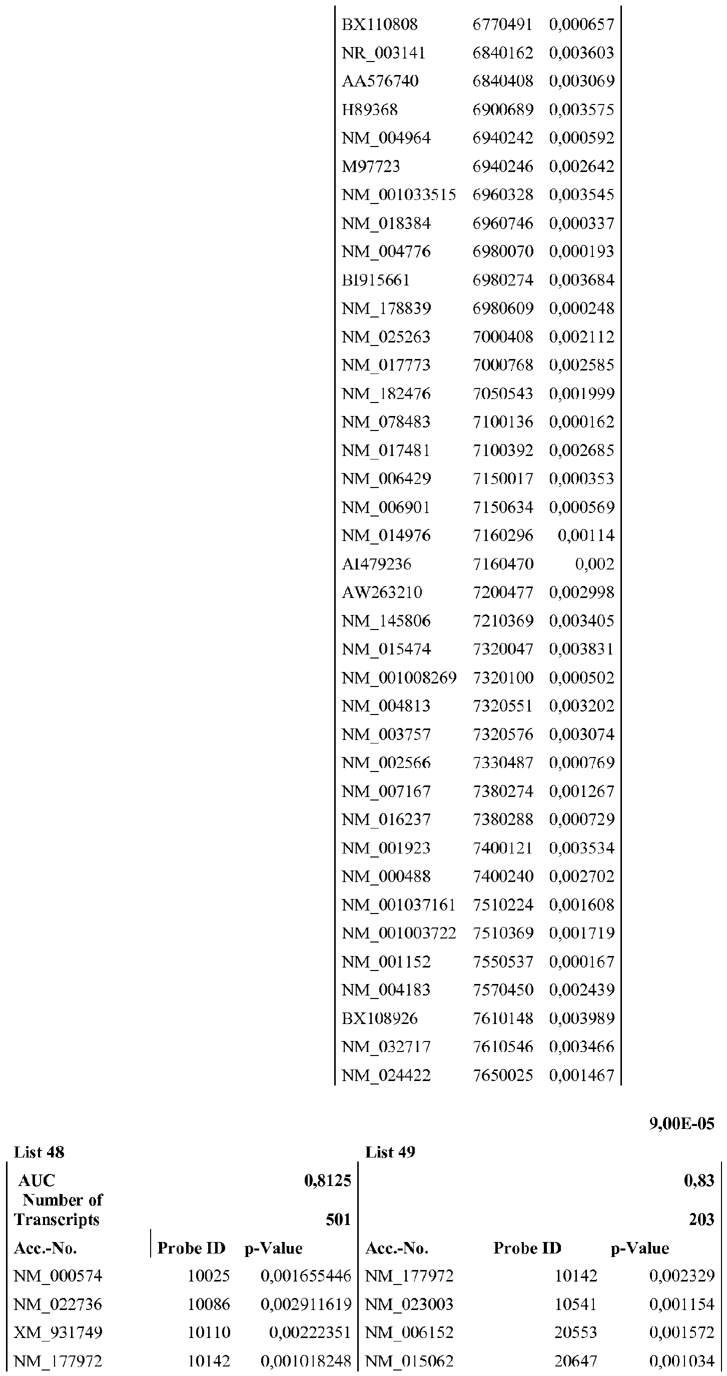

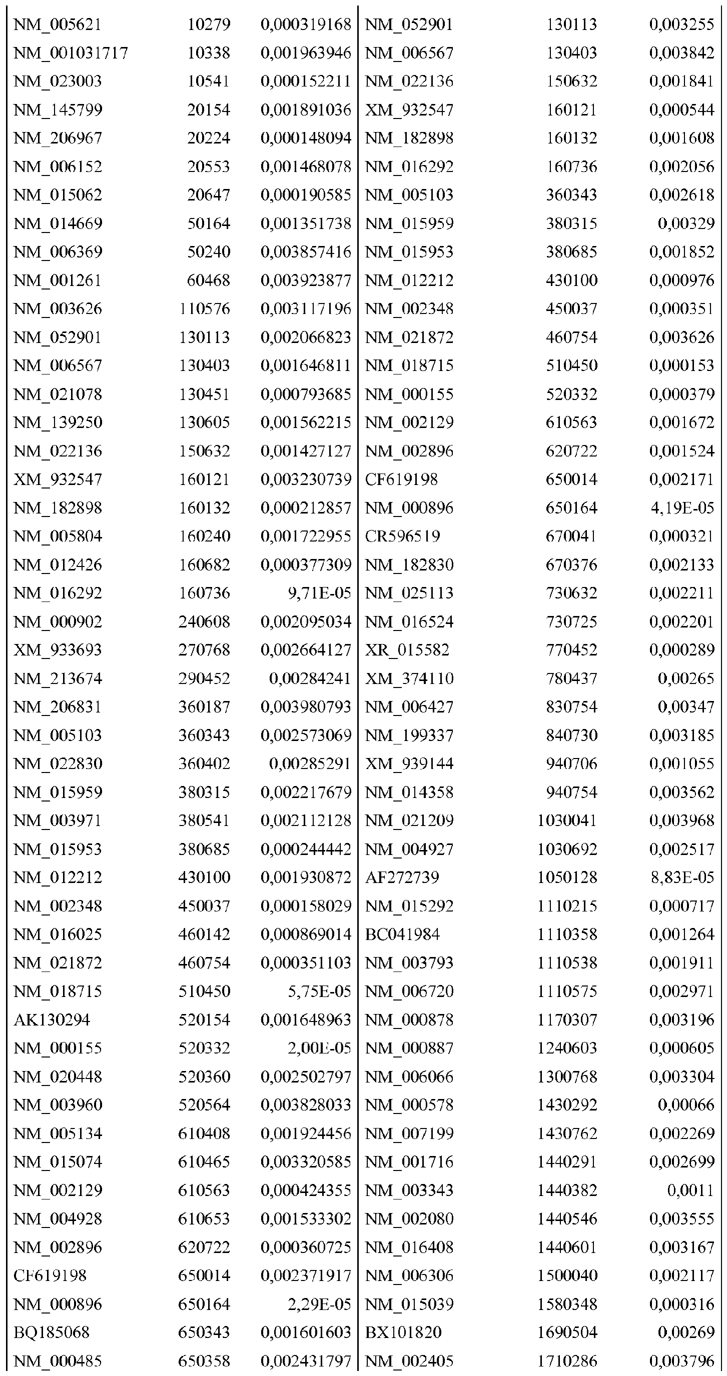

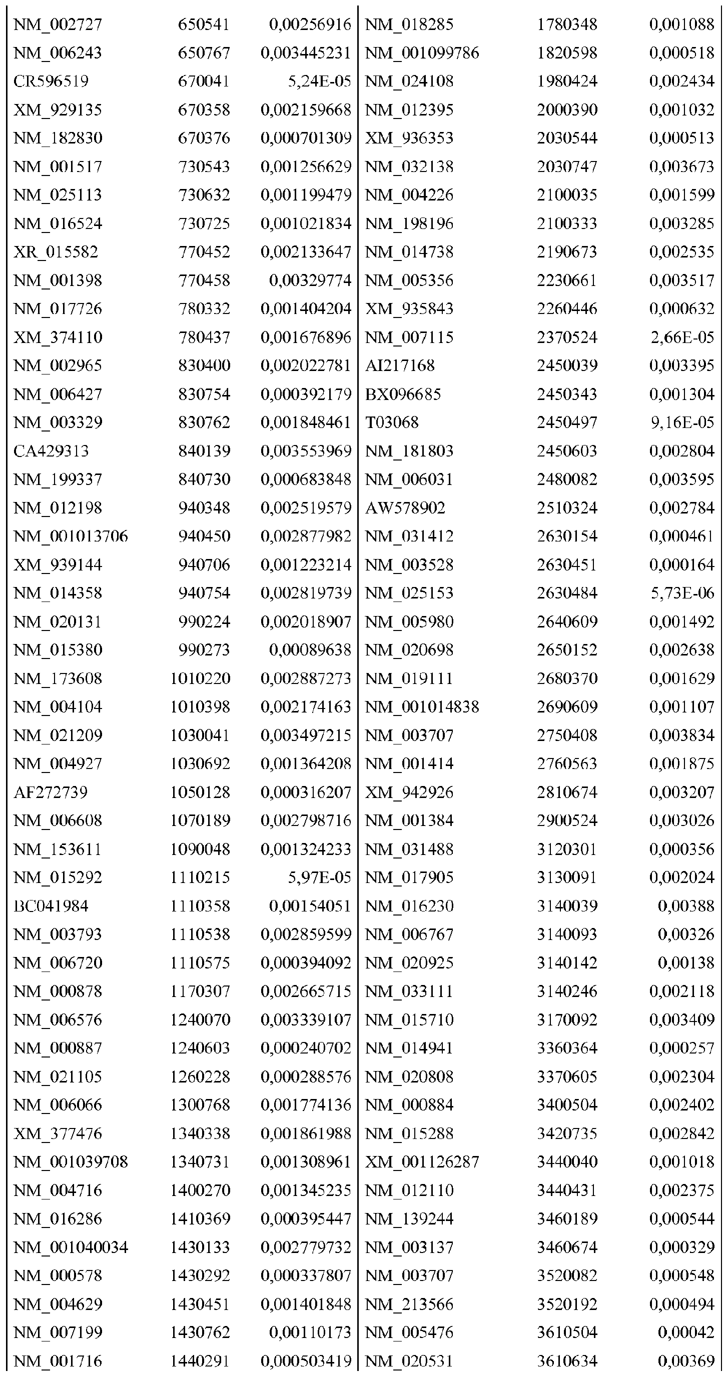

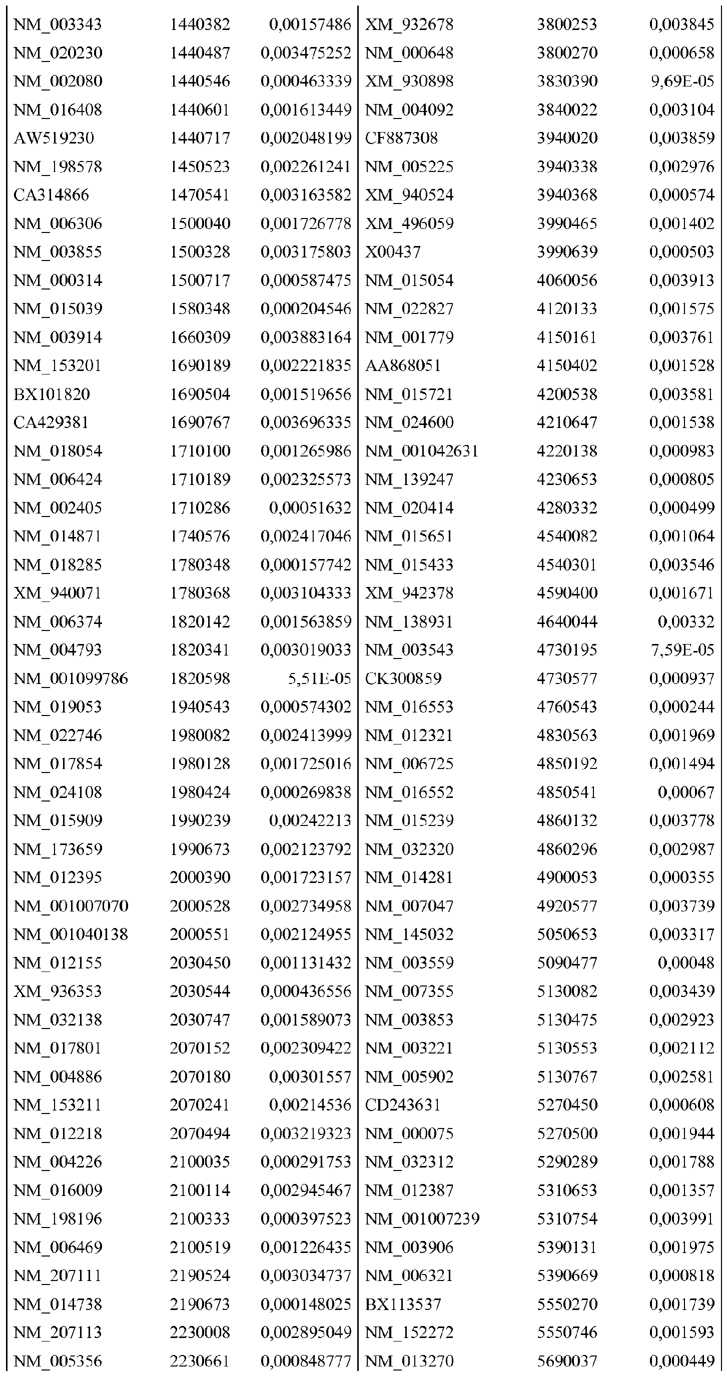

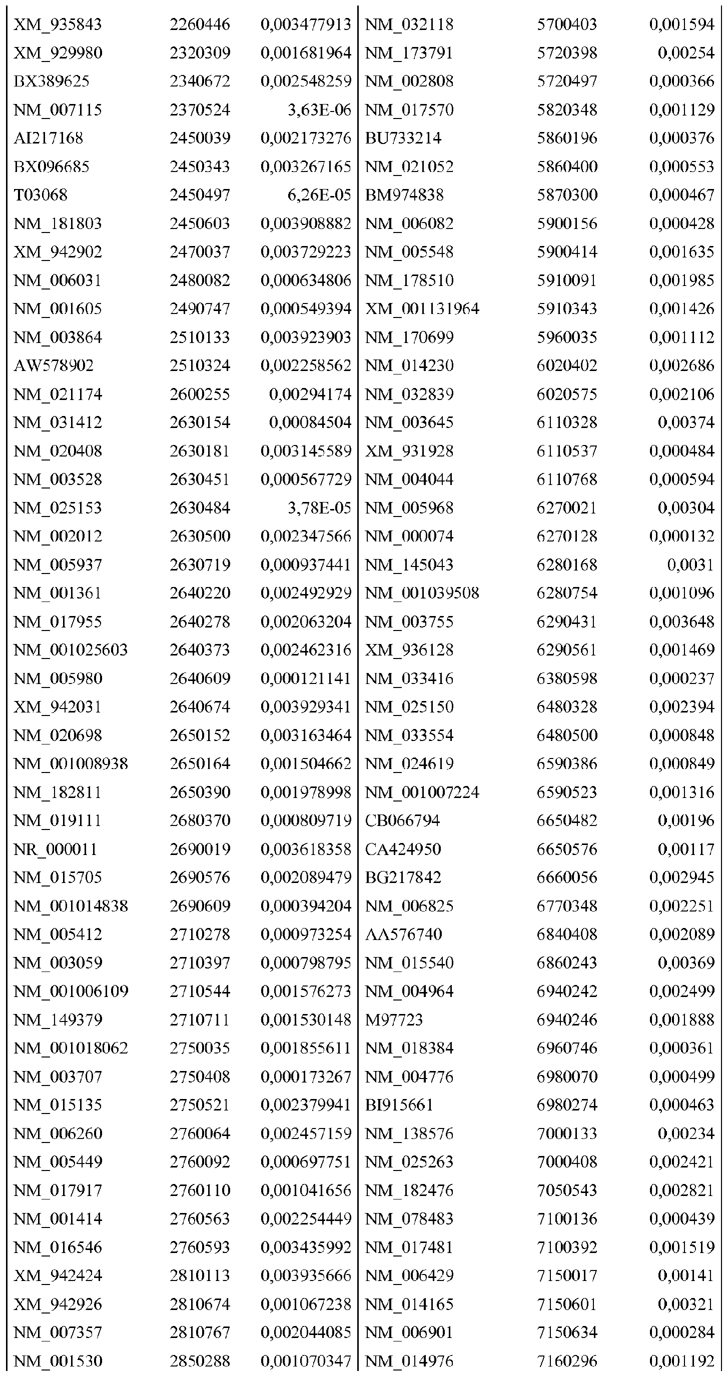

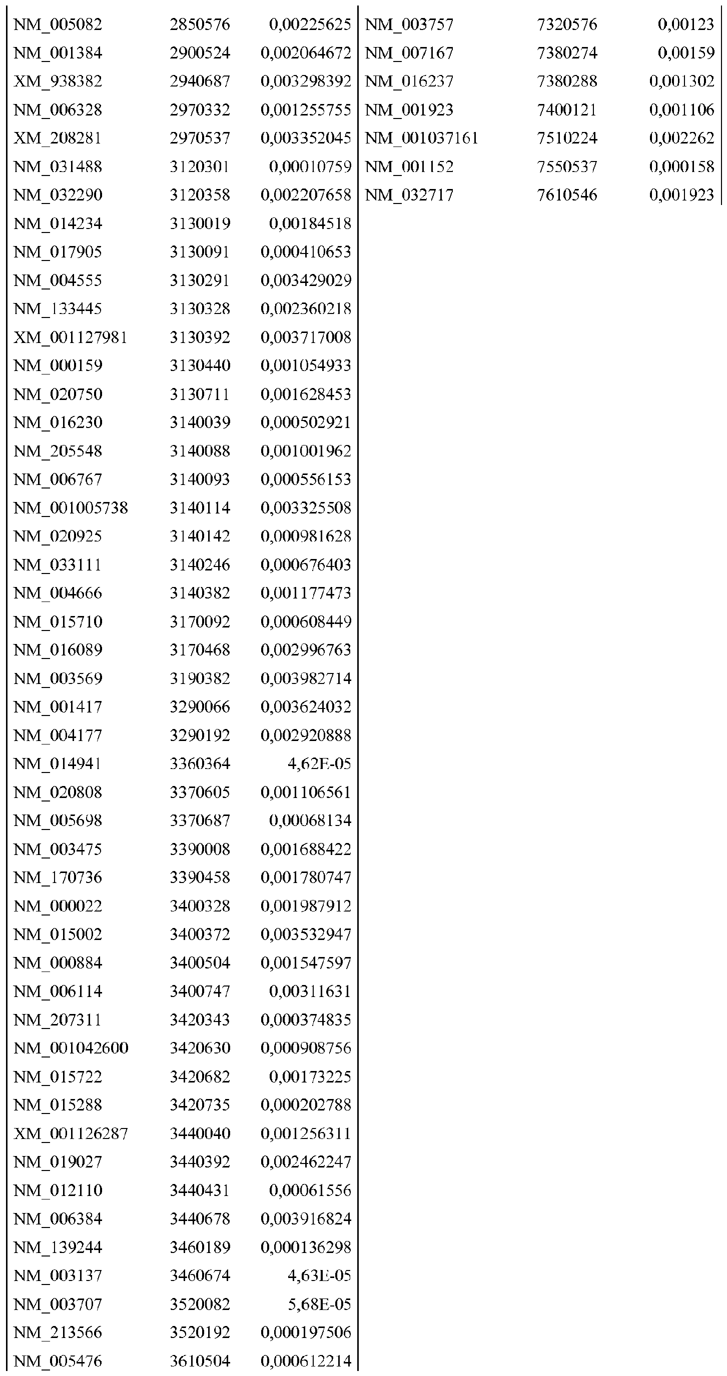

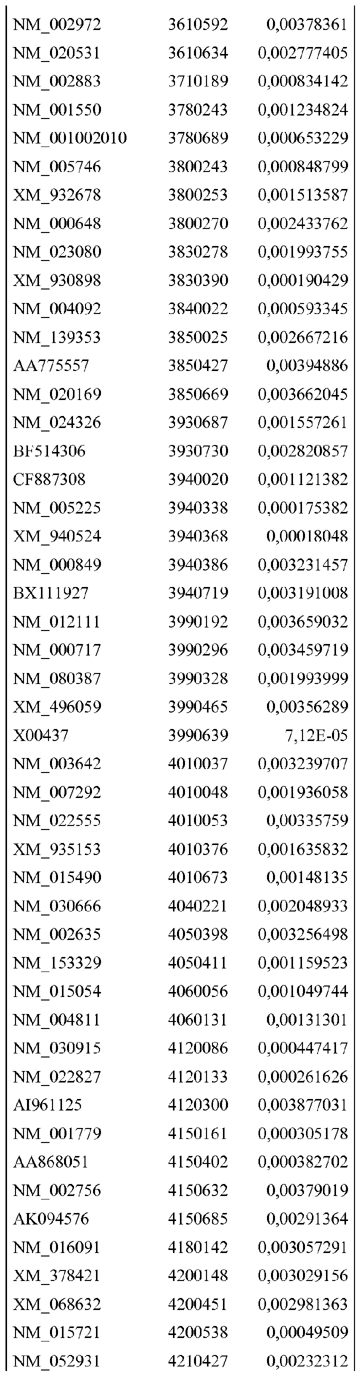

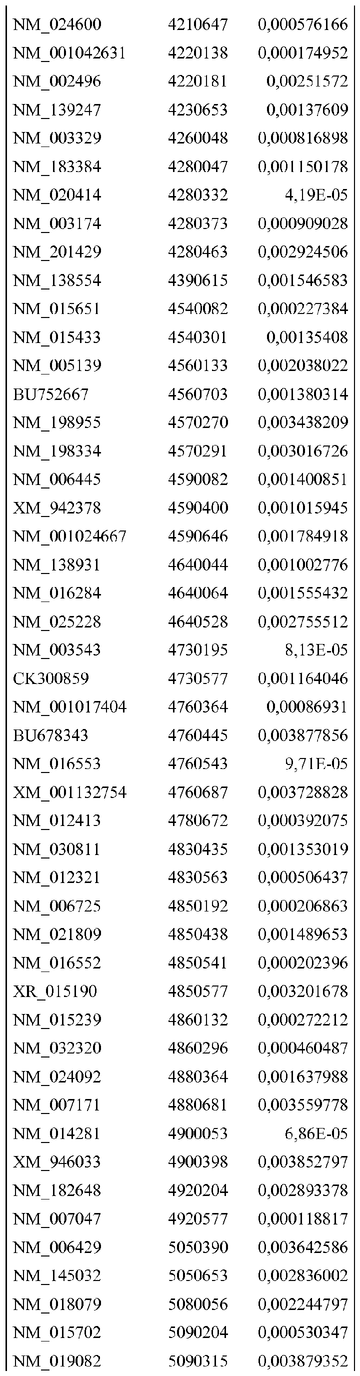

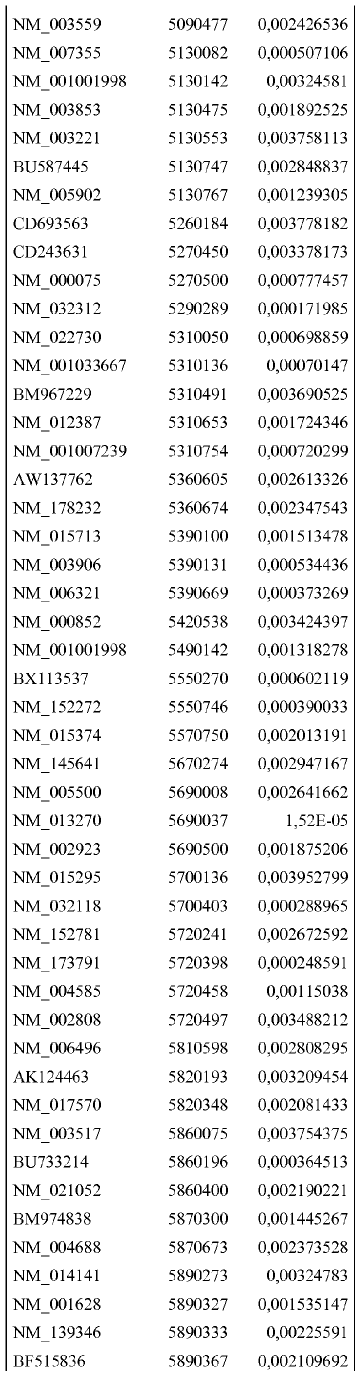

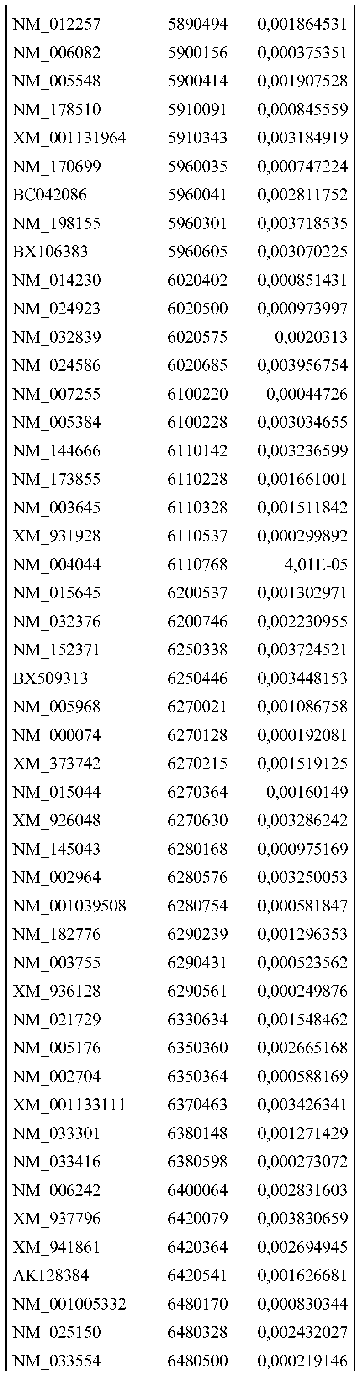

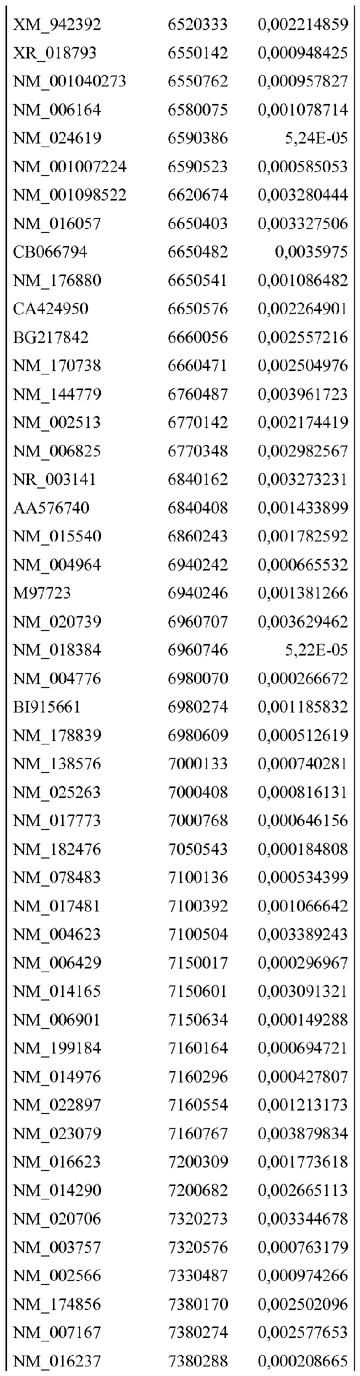

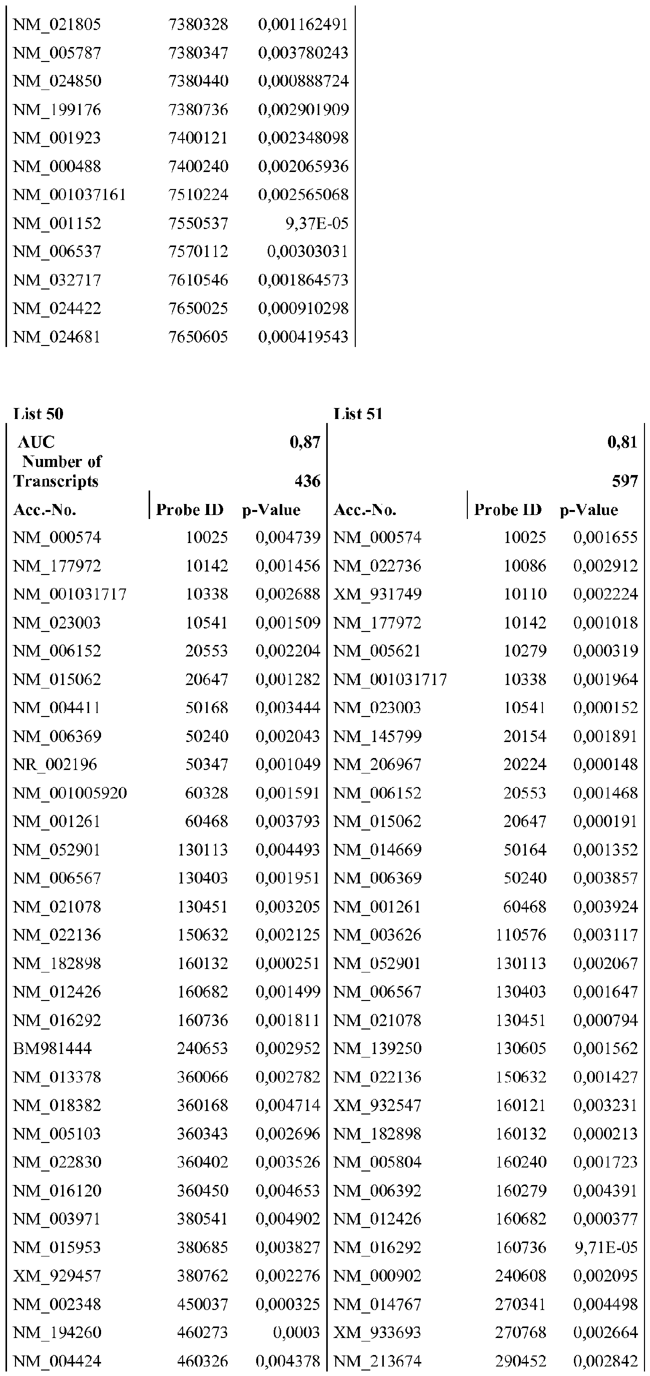

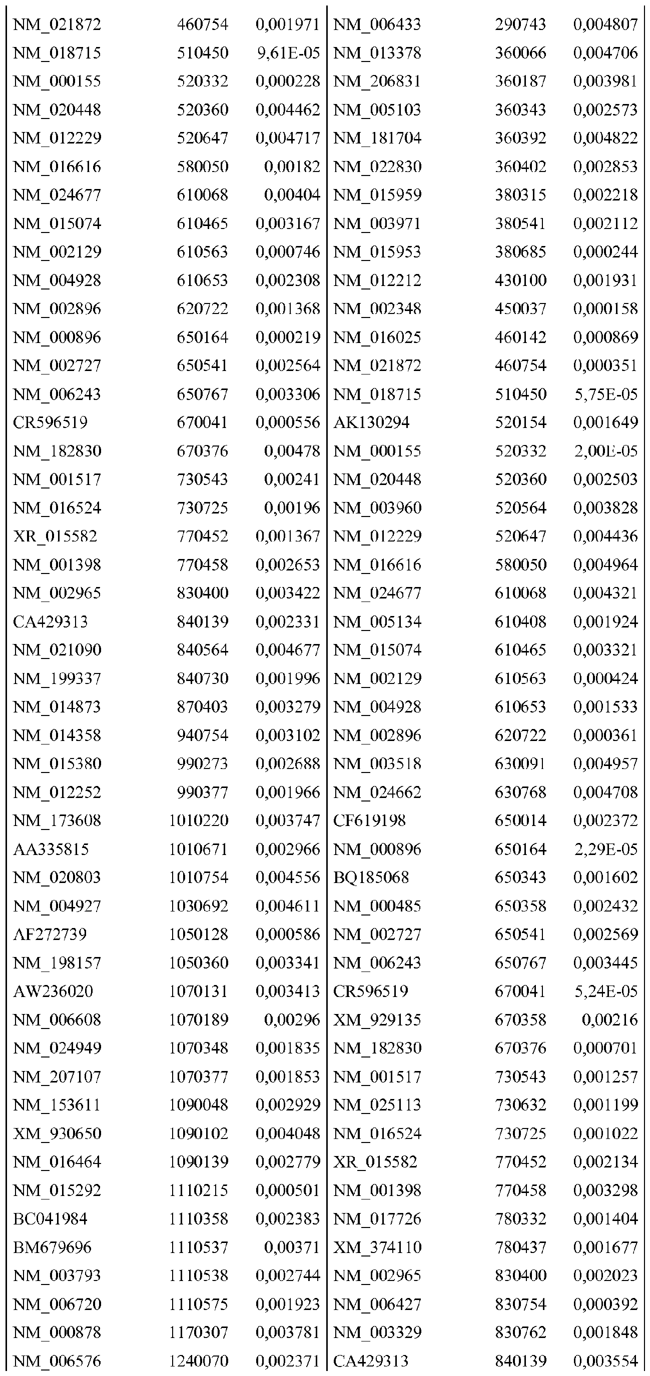

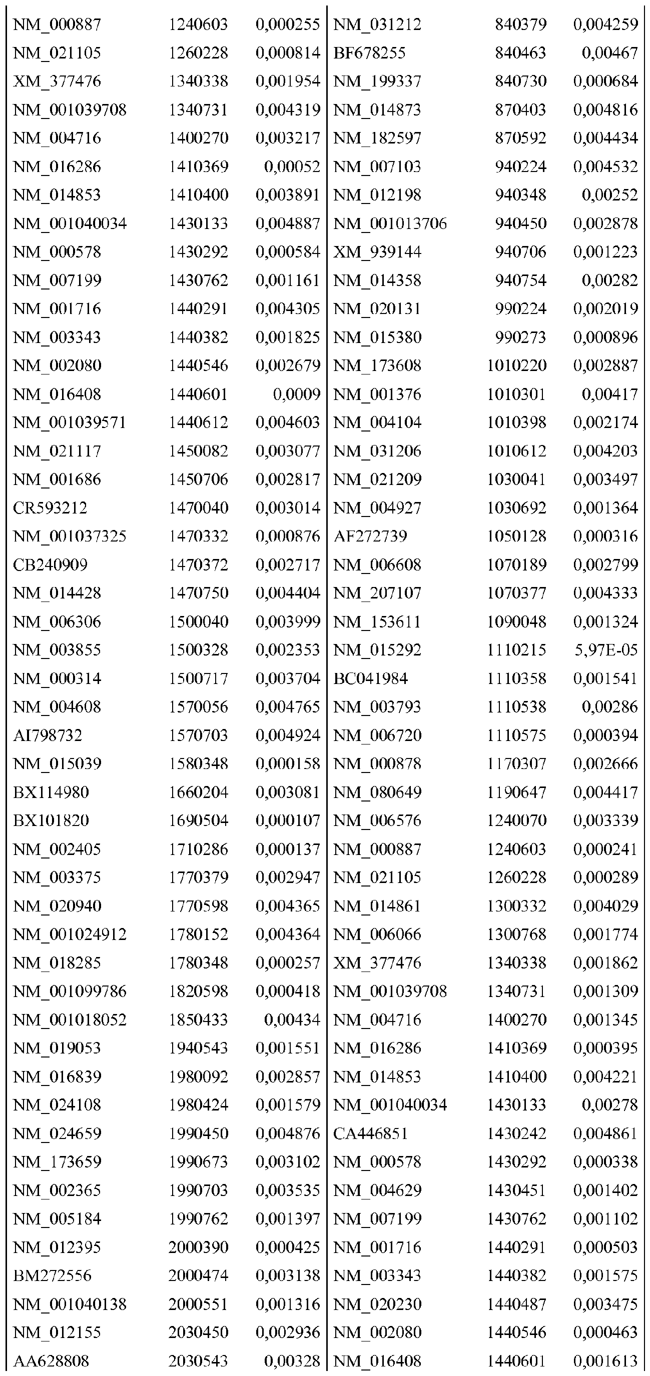

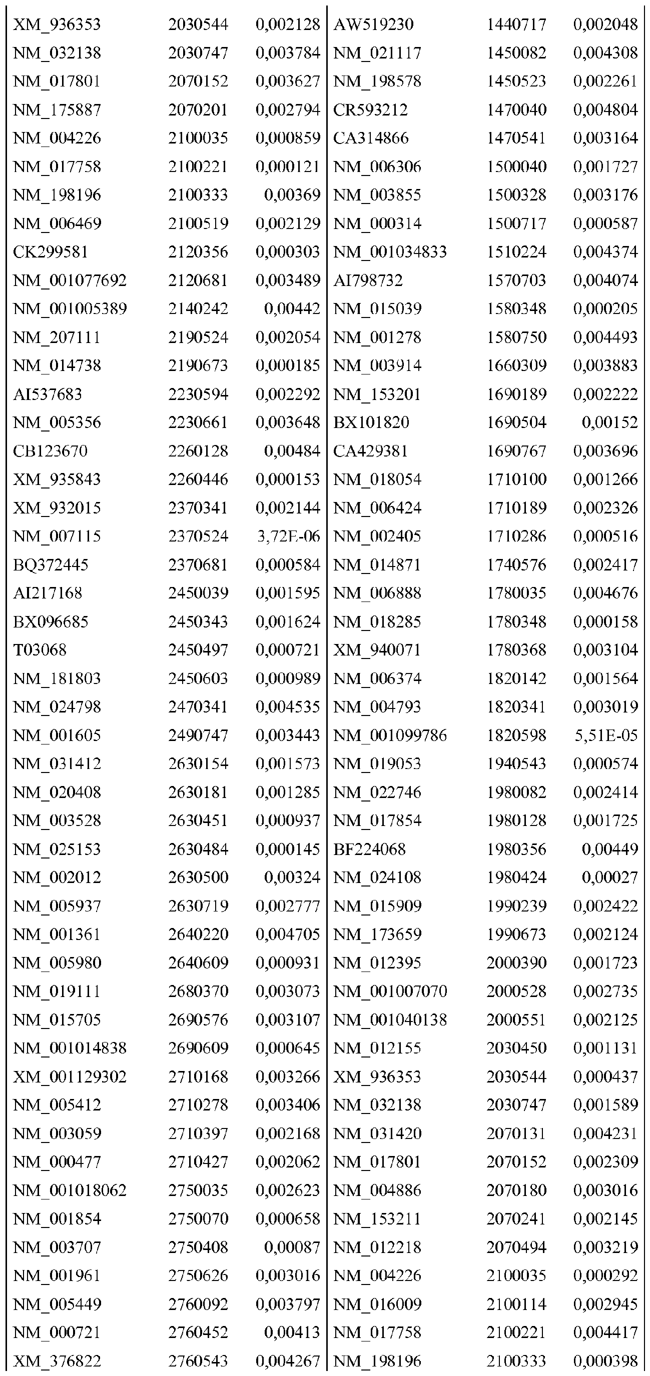

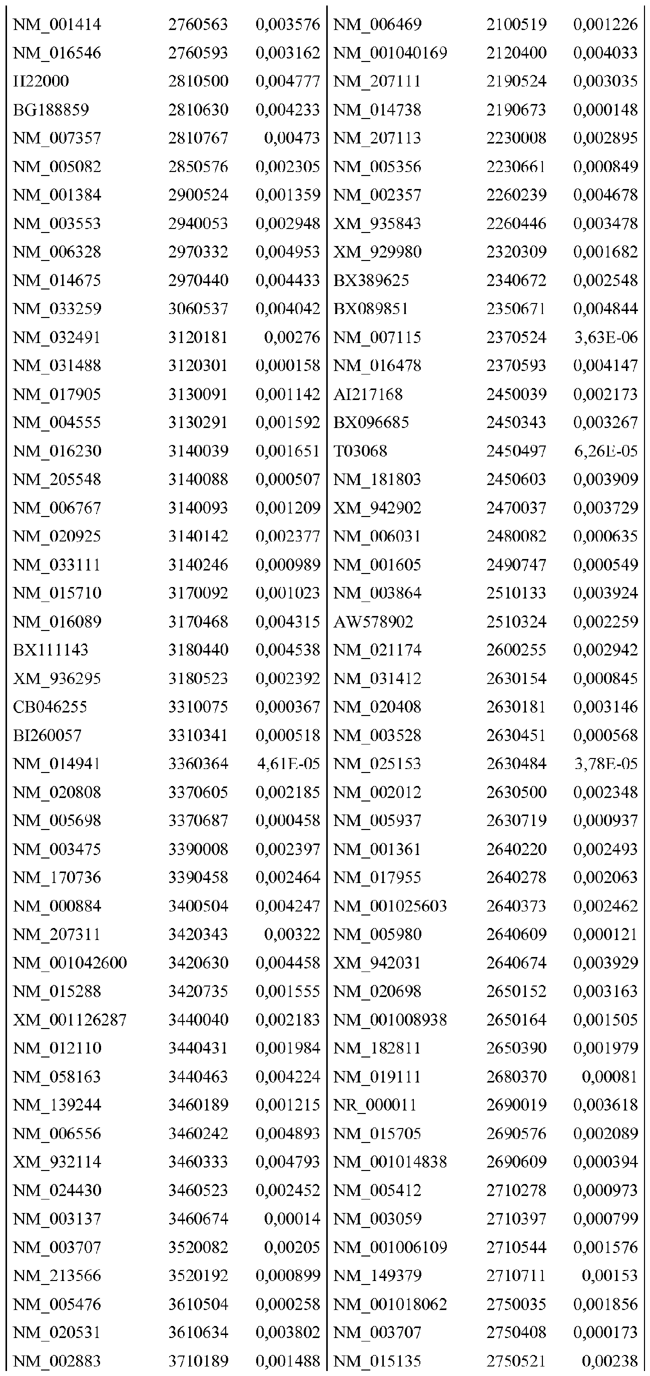

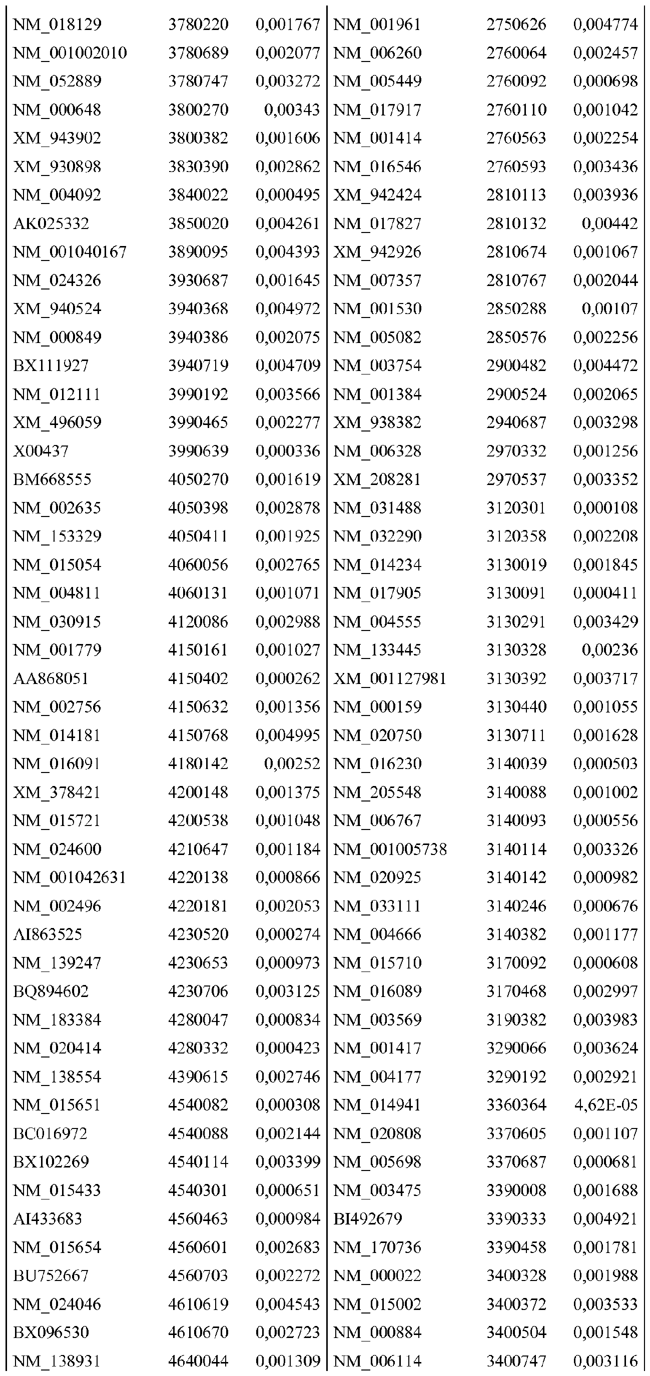

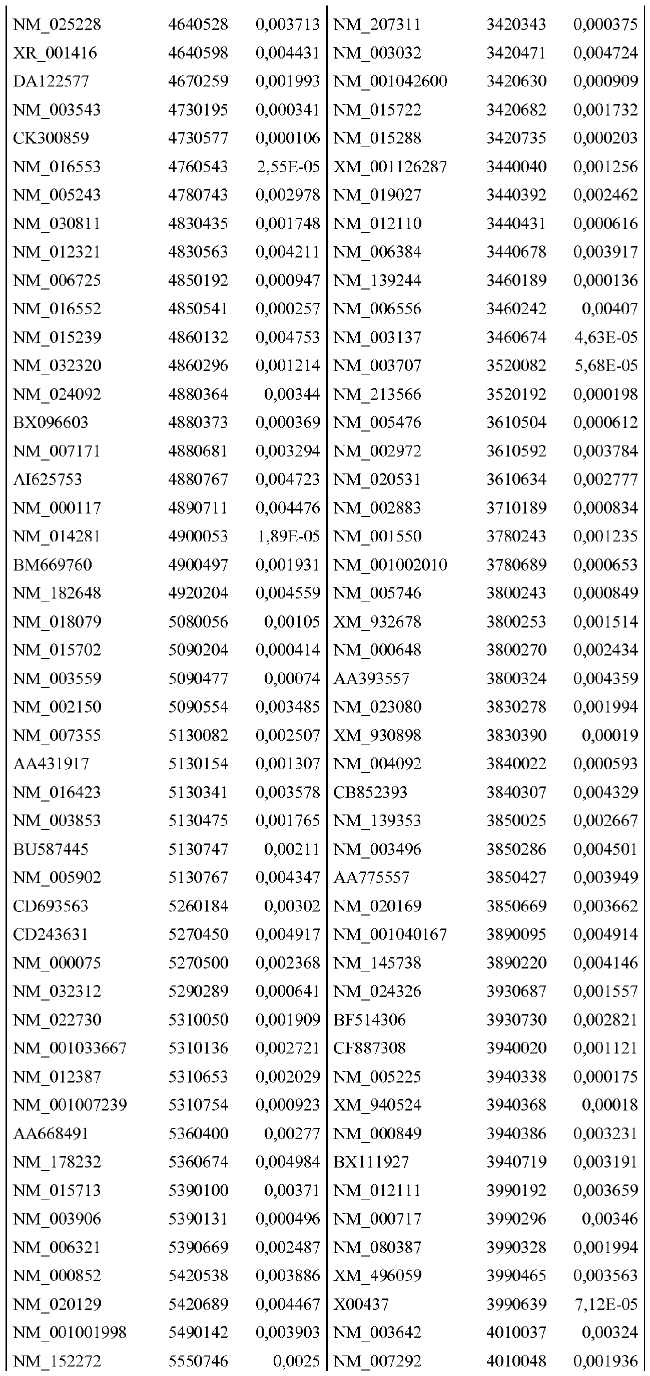

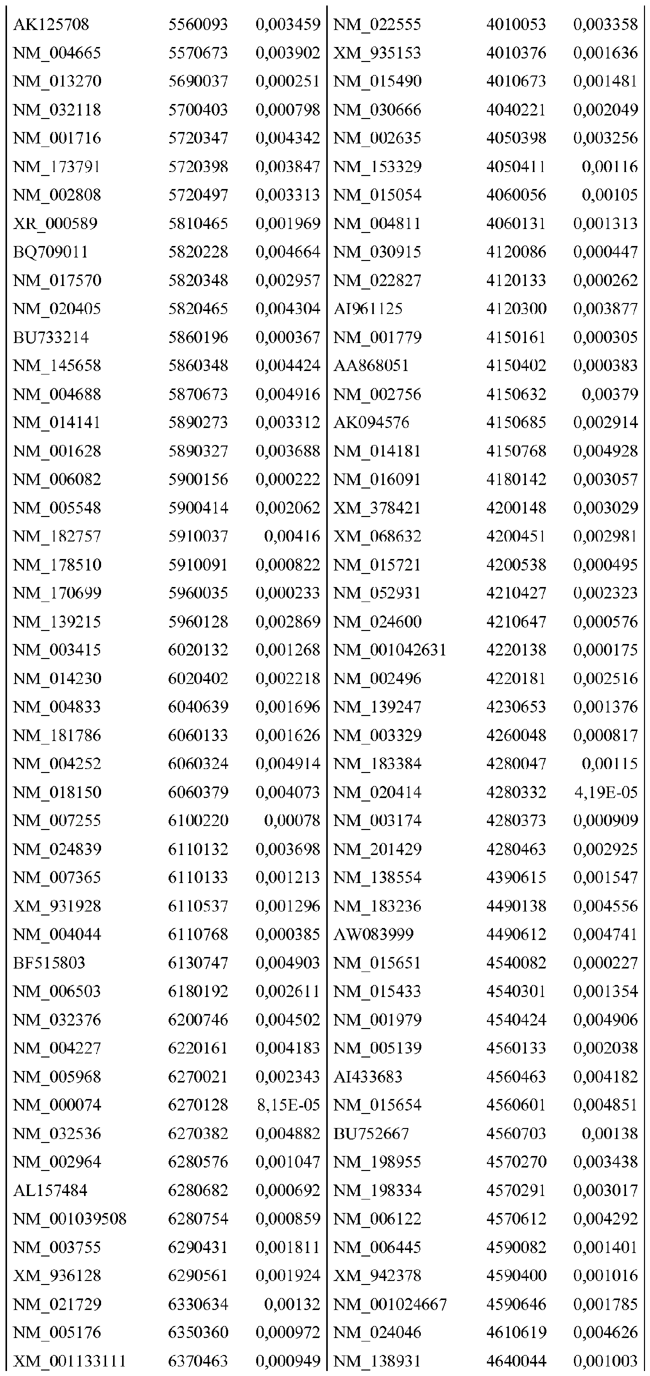

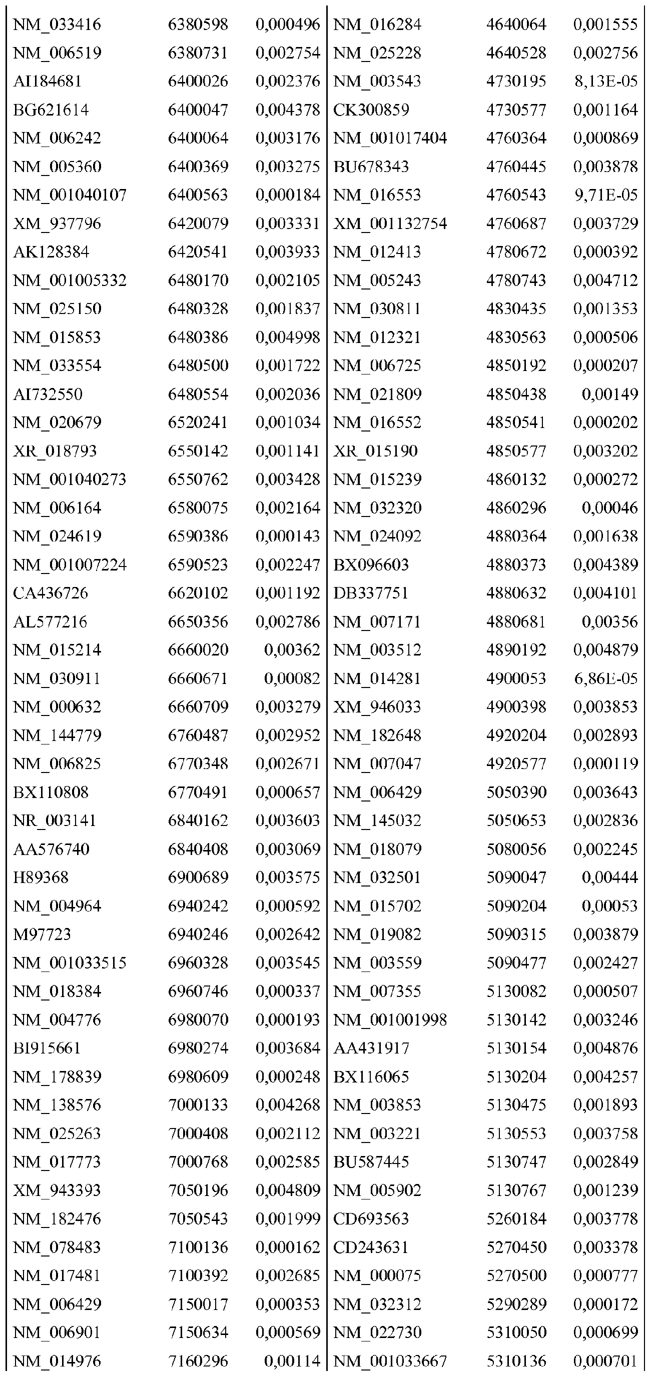

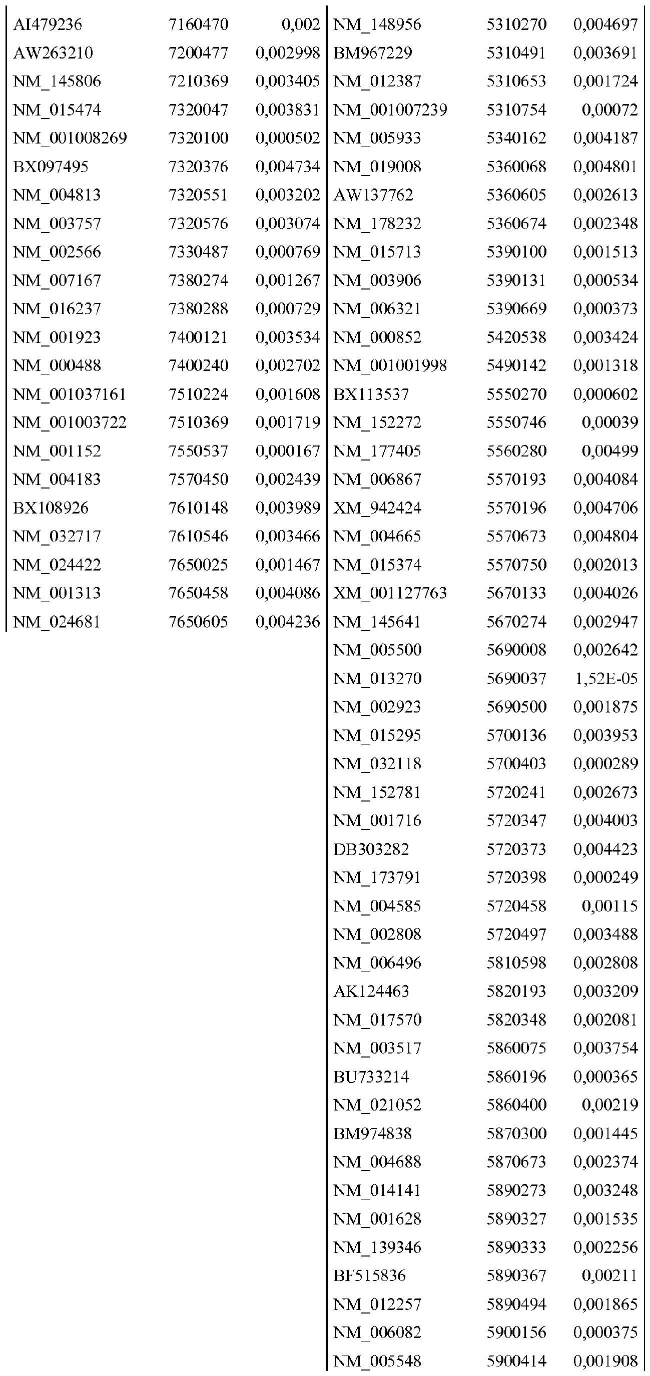

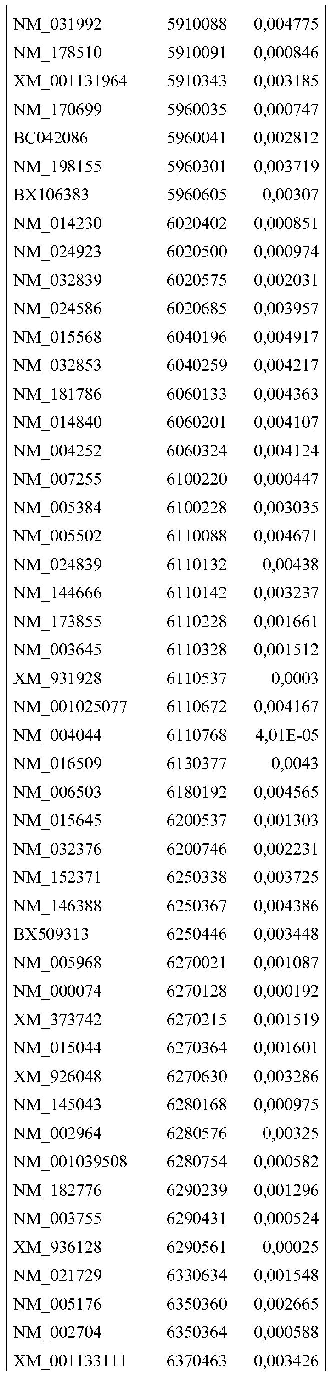

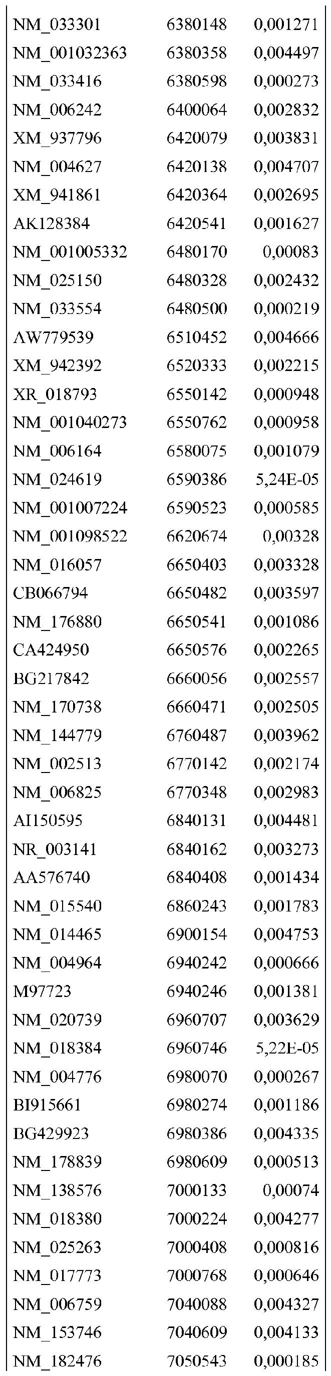

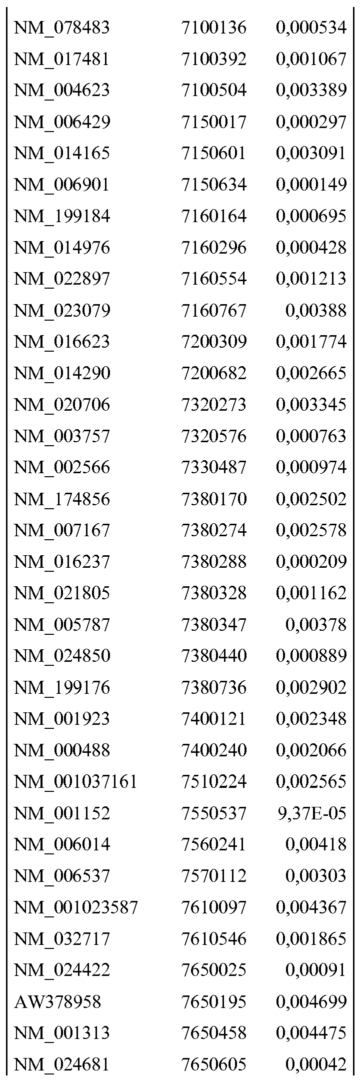

## Claims

1. A method for the detection of non-small cell lung cancer (NSCLC) in a human individual based on RNA obtained from a blood sample obtained from the individual, comprising:
- determining the abundance of at least 5 RNAs in the sample that are chosen from the RNAs listed in Table 2, and
- concluding based on the measured abundance whether the patient has NSCLC.

2. The method of claim 1, wherein the abundance of at least 7, or of at least 11 RNAs that are chosen from the RNAs listed in Table 2 is determined.

3. The method of claim 1 or 2, wherein
concluding comprises classifying the sample as being from a healthy individual or from an individual having NSCLC based on the specific difference of the abundance of the at least 5 RNAs in healthy individuals versus the abundance of the at least 5 RNAs in individuals with NSCLC.

4. The method of claim 3, wherein the classifying is achieved by applying a Support Vector Machines (SVM), a random forest method, or a K-nearest neighbor method.

5. The method of claim 1 to 4, which comprises determining the abundance (expression levels) of the RNAs specified in figure 2B.

6. The method of claims 1 to 5, wherein the abundance of the RNAs in the sample are increased or decreased as shown in Table 2.

7. The method of claim 6, wherein an increase of the abundance provides for a change of > 1.1, > 1.2, or > 1.3, and a decrease of the expression provides for a change < 0.9, < 0.8, or < 0.7 relative to the respective expression in a healthy individual.

8. The method of claims 1 to 7, wherein the abundance is determined with an RNA hybridization assay, preferably with a solid phase microarray, a real-time polymerase chain reaction, or sequencing.

9. The method of claims 1 to 8, wherein the abundance is determined through a hybridization with probes for determining the abundance of the at least 5 RNAs of Table 2.

10. The method of claim 9, wherein said probes
comprise 15 to 150, preferably 30 to 70 consecutive nucleotides with a reverse complementary sequence to the at least 5 RNAs whose abundance is to be determined.

11. A microarray for the detection of NSCLC comprising probes for detecting at least 5, or at least 7, or at least 11 RNAs that are chosen from the RNAs listed in Table 2.

12. Use of a microarray of claim 11 for the detection of non-small cell lung cancer (NSCLC) in a human individual based on RNA obtained from a blood sample obtained from the individual, preferably determining the abundance of at least 5 RNAs in the sample that are chosen from the RNAs listed in Table 2.

13. A kit for the detection of NSCLC, comprising means for determining the abundance of at least 5 RNAs in the sample that are chosen from the RNAs listed in Table 2.

14. The kit of claim 13, which comprises
- probes comprising 15 to 150, preferably 30 to 70 consecutive nucleotides with a reverse complementary sequence to the at least 5 RNAs whose abundance is to be determined, or
- a microarray comprising probes with a reverse complementary sequence to the at least 5 RNAs whose abundance is to be determined.

15. The kit of claim 13 or 14, which further comprises a mixture of at least 5 of the RNAs of table 2 in a given amount for use as a standard.
